# EUROPÄISCHE PATENTSCHRIFT

(11) **EP 1 940 769 B1**
(45) Veröffentlichungstag und Bekanntmachung des Hinweises auf die Patenterteilung: **10.08.2016**
(21) Anmeldenummer: 06806330.4
(22) Anmeldetag: 17.10.2006
(51) Int. Cl.: C07C 65/28, A61K 31/194, A61P 9/00

(54) **DICARBONSÄURE-DERIVATE UND IHRE VERWENDUNG**
DICARBOXYLIC ACID DERIVATIVES AND THEIR USE
DÉRIVÉS D'ACIDE DICARBOXYLIQUE ET LEUR UTILISATION

(30) Priorität: 21.10.2005 DE 102005050376
(43) Veröffentlichungstag der Anmeldung: 09.07.2008
(73) Patentinhaber: Bayer Intellectual Property GmbH, 40789 Monheim (DE)
(72) Erfinder: BARTEL, Stephan, 51515 Kürten (DE); HAHN, Michael, 40764 Langenfeld (DE); MORADI, Wahed Ahmed, 40789 Monheim (DE); BECKER, Eva-Maria, 42117 Wuppertal (DE); RÖLLE, Thomas, 51381 Leverkusen (DE); STASCH, Johannes-Peter, 42651 Solingen (DE); SCHLEMMER, Karl-Heinz, 42113 Wuppertal (DE); WUNDER, Frank, 42117 Wuppertal (DE); KNORR, Andreas, 40699 Erkrath (DE); LANG, Dieter, 42553 Velbert (DE)
(74) Vertreter: BIP Patents
(86) Internationale Anmeldenummer: PCT/EP2006/009996
(87) Internationale Veröffentlichungsnummer: WO 2007/045433

(56) Entgegenhaltungen:
- WO-A-01/19778
- WO-A2-01/19780

## Beschreibung

Die vorliegende Anmeldung betrifft neue Dicarbonsäure-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulärer, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch

Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-l-benzylindazol [YC-1, Wu et al., Blood 84 (1994), 4226; Mülsch et al., Br. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Br. J. Pharmacol. 114 (1995), 1587], sowie verschiedene substituierte Pyrazolderivate (WO 98/16223, WO 98/16507 und WO 98/23619).

Die vorstehend beschriebenen Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung [Gerzer et al., FEBS Lett. 132 (1981), 71] oder über einen Häm-abhängigen Mechanismus, der unabhängig von NO ist, aber zu einer Potenzierung der stimulierenden Wirkung von NO oder CO führt [z.B. YC-1, Hoenicka et al., J. Mol. Med. 77 (1999) 14; oder die in WO 98/16223, WO 98/16507 und WO 98/23619 beschriebenen Pyrazolderivate].

Die in der Literatur behauptete stimulierende Wirkung von Isoliquiritigenin und von Fettsäuren, wie z.B. von Arachidonsäure, Prostaglandin-Endoperoxiden und Fettsäure-Hydroperoxiden auf die lösliche Guanylatcyclase konnte nicht bestätigt werden [vgl. z.B. Hoenicka et al., J. Mol. Med. 77 (1999), 14].

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben [Ignarro et al., Adv. Pharmacol. 26 (1994), 35]. Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird [Mülsch et al., Naunyn Schmiedebergs Arch. Pharmacol. 355, R47].

Im Gegensatz zu den vorstehend beschriebenen Stimulatoren der löslichen Guanylatcyclase sind die Verbindungen der vorliegenden Erfindung in der Lage, sowohl die Häm-haltige als auch die Häm-freie Form der löslichen Guanylatcyclase zu aktivieren. Die Stimulierung des Enzyms verläuft bei diesen neuen Aktivatoren also über einen Häm-unabhängigen Weg, was auch dadurch belegt wird, dass die neuen Aktivatoren am Häm-haltigen Enzym einerseits keine synergistische Wirkung mit NO zeigen und andererseits sich die Wirkung dieser neuartigen Aktivatoren nicht durch den Häm-abhängigen Inhibitor der löslichen Guanylatcyclase, 1*H*-1,2,4-Oxadiazol-(4,3-a)-chinoxalin-1-on (ODQ), blockieren lässt.

In EP 0 341 551-A1 werden Alkensäure-Derivate als Leukotrien-Antagonisten für die Behandlung von Erkrankungen des Kreislauf- und Atmungssystems offenbart. In WO 01/19355, WO 01/19776, WO 01/19778, WO 01/19780, WO 02/070462 und WO 02/070510 werden Dicarbonsäure- bzw. Aminodicarbonsäure-Derivate als Stimulatoren der löslichen Guanylatcyclase zur Behandlung von Herz-Kreislauf-Erkrankungen beschrieben. Allerdings zeigte es sich, dass diese Verbindungen hinsichtlich ihrer pharmakokinetischen Eigenschaften Nachteile aufweisen, wie insbesondere eine geringe Bioverfügbarkeit und/oder eine nur kurze Wirkdauer nach oraler Gabe.

Aufgabe der vorliegenden Erfindung war daher die Bereitstellung neuer Verbindungen, welche als Aktivatoren der löslichen Guanylatcyclase wirken, jedoch nicht die vorstehend aufgeführten Nachteile der Verbindungen aus dem Stand der Technik aufweisen.

Diese Aufgabe wird durch die in der vorliegenden Erfindung beschriebenen Verbindungen gelöst. Diese Verbindungen zeichnen sich strukturell im Vergleich zu den Verbindungen aus dem Stand der Technik durch eine 3-Benzyl-1,5-diphenylpent-1-en-Kernstruktur aus.

Offenbarungsgegenstand sind Verbindungen der allgemeinen Formel (I) 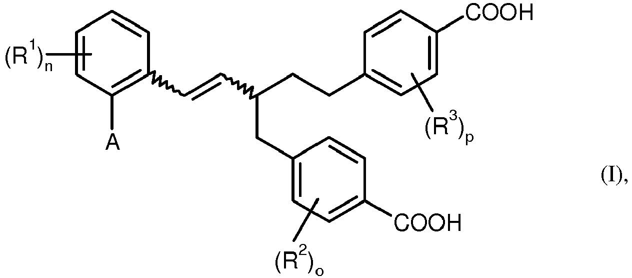 in welcher
A für Wasserstoff oder für eine Gruppe der Formel -O-A¹-A² steht, worin
   A¹ eine Bindung oder (C₁-C₇)-Alkandiyl, (C₂-C₆)-Alkandiyl-O-^{#}, (C₂-C₇)-Alkendiyl oder (C₂-C₇)-Alkindiyl, welche jeweils ein- oder mehrfach mit Fluor substituiert sein können und worin ^{#} die Verknüpfungsstelle mit der Gruppe A² darstellt,
      und
   A² Wasserstoff, Trifluormethyl oder eine Gruppe der Formel 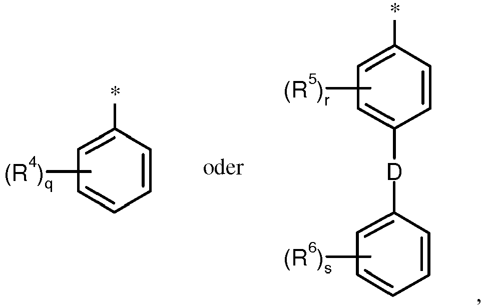 worin * die Verknüpfungsstelle mit der Gruppe A¹ und
   D eine Bindung, CH₂, -CH₂-CH₂- oder -CH=CH- darstellt, bedeuten,
      - R¹, R², R³, R⁵ und R⁶: unabhängig voneinander für einen Substituenten ausgewählt aus der Reihe Halogen, (C₁-C₆)-Alkyl, Trifluormethyl, (C₁-C₆)-Alkoxy, Trifluormethoxy, Cyano und Nitro stehen,
      - R⁴: für einen Substituenten ausgewählt aus der Reihe Halogen, (C₁-C₆)-Alkyl, (C₁-C₆)-Alkoxy, Cyano und Nitro steht, wobei Alkyl und Alkoxy jeweils ein- oder mehrfach mit Fluor substituiert sein können,
      und
      - n, o, p, q, r und s: unabhängig voneinander jeweils für die Zahl 0, 1, 2, 3 oder 4 stehen, wobei für den Fall, dass R¹, R², R³, R⁴, R⁵ oder R⁶ mehrfach auftreten, ihre Bedeutungen jeweils gleich oder verschieden sein können,
      sowie ihre Salze, Solvate und Solvate der Salze.

Erfindungsgemäße Verbindungen sind die Verbindungen der Formeln (I-A) und (I-B) und deren Salze, Solvate und Solvate der Salze, die von den Formeln (I-A) und (I-B) umfassten Verbindungen der nachfolgend genannten Formeln und deren Salze, Solvate und Solvate der Salze sowie die von den Formeln (I-A) und (I-B) umfassten, nachfolgend als Ausführungsbeispiele genannten Verbindungen und deren Salze, Solvate und Solvate der Salze, soweit es sich bei den von den Formeln (I-A) und (I-B) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Die Gruppierung 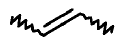 in Formel (I) bedeutet, dass diese CC-Doppelbindung in einer *cis-* oder in einer trans-Konfiguration vorliegen kann. Beide isomeren Formen werden von der vorliegenden Erfindung umfasst. Bevorzugt sind Verbindungen der Formel (I) mit einer *trans*-Anordnung dieser Doppelbindung.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Anmioniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, *iso*-Butyl, *sec*.-Butyl, *tert.*-Butyl*,* 1-Ethylpropyl, n-Pentyl und n-Hexyl.

(C₁-C₇)-Alkandiyl und (C₂-C₆)-Alkandiyl stehen im Rahmen der Erfindung für einen geradkettigen oder verzweigten divalenten Alkylrest mit 1 bis 7 bzw. 2 bis 6 Kohlenstoffatomen. Bevorzugt ist ein geradkettiger Alkandiylrest mit 1 bis 6 bzw. 2 bis 5 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl, Butan-2,3-diyl, Pentan-1,5-diyl, Pentan-2,4-diyl, 3-Methyl-pentan-2,4-diyl und Hexan-1,6-diyl.

(C₂-C₇)-Alkendiyl steht im Rahmen der Erfindung für einen geradkettigen oder verzweigten divalenten Alkenylrest mit 2 bis 7 Kohlenstoffatomen und bis zu 3 Doppelbindungen. Bevorzugt ist ein geradkettiger Alkendiylrest mit 2 bis 6 Kohlenstoffatomen und bis zu 2 Doppelbindungen. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl, Buta-1,3-dien-1,4-diyl, Pent-2-en-1,5-diyl, Hex-3-en-1,6-diyl und Hexa-2,4-dien-1,6-diyl.

(C₂-C₇)-Alkindiyl steht im Rahmen der Erfindung für einen geradkettigen oder verzweigten divalenten Alkinylrest mit 2 bis 7 Kohlenstoffatomen und bis 3 Dreifachbindungen. Bevorzugt ist ein geradkettiger Alkindiylrest mit 2 bis 6 Kohlenstoffatomen und bis zu 2 Dreifachbindungen. Beispielhaft und vorzugsweise seien genannt: Ethin-1,2-diyl, Propin-1,3-diyl, But-1-in-1,4-diyl, But-1-in-1,3-diyl, But-2-in-1,4-diyl, Pent-2-in-1,5-diyl, Pent-2-in-1,4-diyl und Hex-3-in-1,6-diyl.

(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert.*-Butoxy, n-Pentoxy und n-Hexoxy.

(C₁-C₄)-Alkoxycarbonyl steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, n-Propoxycarbonyl, Isopropoxycarbonyl und tert.-Butoxycarbonyl.

Halogen schließt im Rahmen der Erfindung Fluor, Chlor, Brom und Iod ein. Bevorzugt sind Chlor oder Fluor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.

Wenn ein Rest in den erfindungsgemäßen Verbindungen mehrfach mit Fluor substituiert sein kann, so schließt dies im Rahmen der vorliegenden Erfindung eine Perfluor-Substitution mit ein.

Ebenfalls Offenbarungsgegenstand sind Verbindungen der Formel (I), in welcher
- A: für Wasserstoff oder für eine Gruppe der Formel -O-A¹-A² steht, worin A¹ eine Bindung, (C₁-C₇)-Alkandiyl, (C₂-C₇)-Alkendiyl oder (C₂-C₇)-Alkindiyl und A² Wasserstoff, Trifluormethyl oder eine Gruppe der Formel

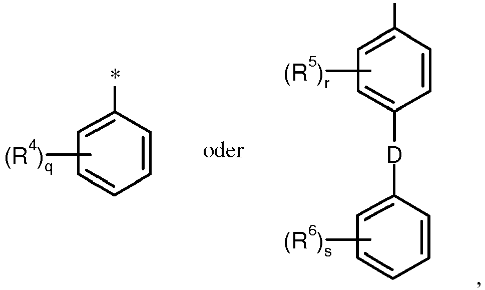

worin * die Verknüpfungsstelle mit der Gruppe A¹ und
D eine Bindung, CH₂, -CH₂-CH₂- oder -CH=CH- darstellt,
   bedeuten,
R¹, R², R³, R⁴, R⁵ und R⁶ unabhängig voneinander für einen Substituenten ausgewählt aus der Reihe Halogen, (C₁-C₆)-Alkyl, Trifluormethyl, (C₁-C₆)-Alkoxy, Trifluormethoxy, Cyano und Nitro stehen,
und
n, o, p, q, r und s unabhängig voneinander jeweils für die Zahl 0, 1, 2, 3 oder 4 stehen,
   wobei für den Fall, dass R¹, R², R³, R⁴, R⁵ oder R⁶ mehrfach auftreten, ihre Bedeutungen jeweils gleich oder verschieden sein können,
sowie ihre Salze, Solvate und Solvate der Salze.

Ebenfalls Offenbarungsgegenstand sind Verbindungen der Formel (I), in welcher
- A: für eine Gruppe der Formel -O-A¹-A²- steht, worin A¹ eine Bindung oder (C₁-C₇)-Alkandiyl und A² Wasserstoff, Trifluormethyl oder eine Gruppe der Formel

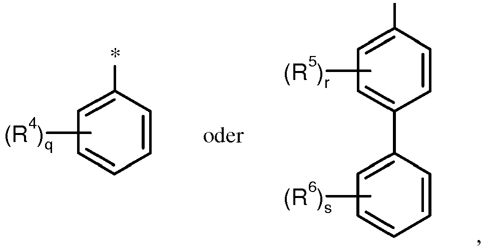

worin * die Verknüpfungsstelle mit der Gruppe A¹ darstellt,
   bedeuten,
R¹, R⁴, R⁵ und R⁶ unabhängig voneinander für einen Substituenten ausgewählt aus der Reihe Fluor, Chlor, Brom, (C₁-C₄)-Alkyl, Trifluormethyl, (C₁-C₄)-Alkoxy und Trifluormethoxy stehen,
n, q, r und s unabhängig voneinander jeweils für die Zahl 0, 1 oder 2 stehen,
   wobei für den Fall, dass R¹, R⁴, R⁵ oder R⁶ mehrfach auftreten, ihre Bedeutungen jeweils gleich oder verschieden sein können,
R² und R³ jeweils für Fluor stehen
und
o und p unabhängig voneinander jeweils für die Zahl 0 oder 1 stehen,
sowie ihre Salze, Solvate und Solvate der Salze.

Gegenstand der vorliegenden Erfindung sind Verbindungen der Formel (I-A) 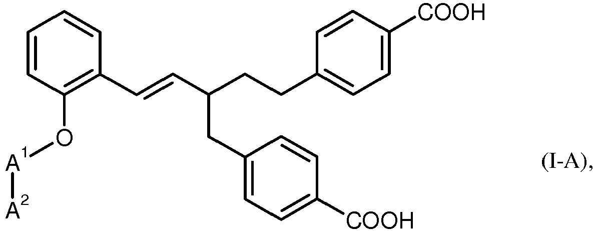 in welcher
- A¹: für (C₁-C₇)-Alkandiyl
und
- A²: für Wasserstoff oder eine Gruppe der Formel

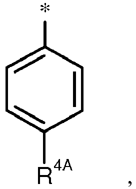

worin * die Verknüpfungsstelle mit der Gruppe A¹ und
R^{4A} Wasserstoff, Fluor, Chlor, Methyl, *tert.*-Butyl*,* Trifluormethyl, Methoxy oder Trifluormethoxy bedeuten,
stehen,
sowie ihre Salze, Solvate und Solvate der Salze.

Gegenstand der vorliegenden Erfindung sind auch Verbindungen der Formel (I-B) 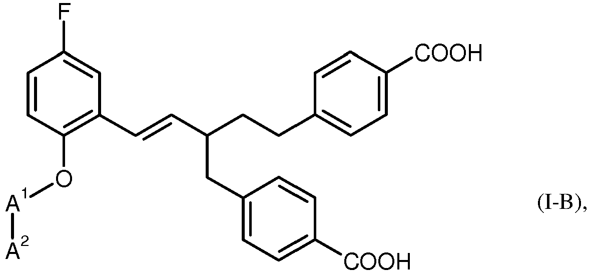 in welcher
- A¹: für (C₁-C₇)-Alkandiyl
und
- A²: für Wasserstoff oder eine Gruppe der Formel 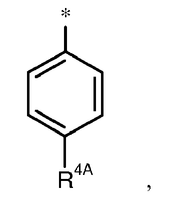
worin * die Verknüpfungsstelle mit der Gruppe A¹ und
- R^{4A}: Wasserstoff, Fluor, Chlor, Methyl, *tert.*-Butyl*,* Trifluormethyl, Methoxy oder Trifluormethoxy bedeuten,
stehen,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man Verbindungen der Formel (II) 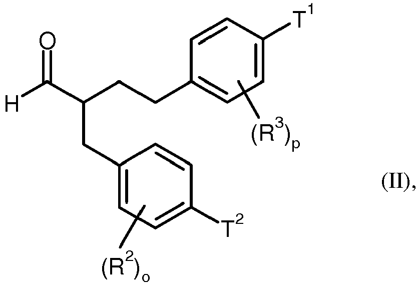
in welcher R², R³ für Wasserstoff stehen, o und p jeweils gleich 4 sind und
T¹ und T² gleich oder verschieden sind und für Cyano oder (C₁-C₄)-Alkoxycarbonyl stehen,
   entweder
   [A] in einem inerten Lösungsmittel in Gegenwart einer Base mit einer Verbindung der Formel (III-A) 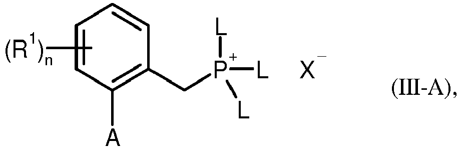 in welcher A für -O-A¹-A²-, R¹ für Wasserstoff steht und n gleich 4 ist und
      - L: für Phenyl oder o-, m- oder p-Tolyl
      und
      - X: für Halogenid oder Tosylat steht,
      zu Verbindungen der Formel (IV-A) 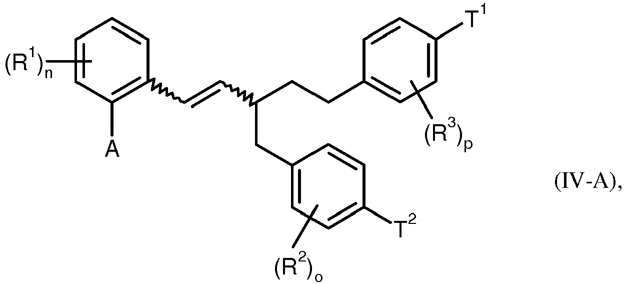
      in welcher A, R¹, R², R³, n, o, p, T¹ und T² jeweils die oben angegebenen Bedeutungen haben,
      umsetzt
      oder
   [B] in einem inerten Lösungsmittel in Gegenwart einer Base mit einer Verbindung der Formel (III-B) 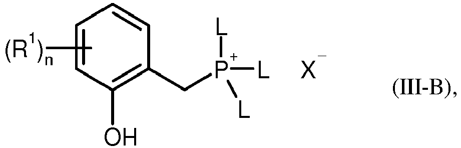 in welcher R1, n, L und X jeweils die oben angegebenen Bedeutungen haben,
      zunächst zu Verbindungen der Formel (IV-B) 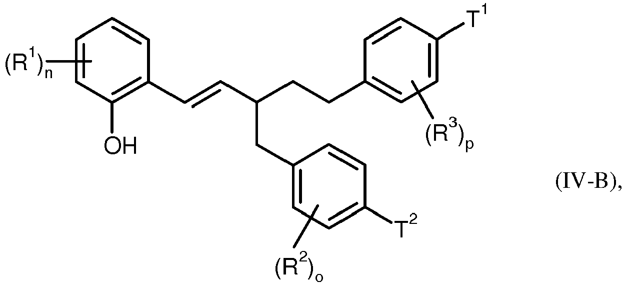 in welcher R1, R2, R3, n, o, p, T1 und T2 jeweils die oben angegebenen Bedeutungen haben,
      umsetzt und diese anschließend in einem inerten Lösungsmittel in Gegenwart einer Base mit einer Verbindung der Formel (V)

      A²-A^{1A}-Q (V),

      in welcher A² die oben angegebene Bedeutung hat,
      A^{1A} die oben angegebene Bedeutung von A¹ hat, jedoch nicht für eine Bindung steht,
      und
      Q für eine Abgangsgruppe, wie beispielsweise Halogen, Tosylat oder Mesylat, steht,
      zu Verbindungen der Formel (IV-C) 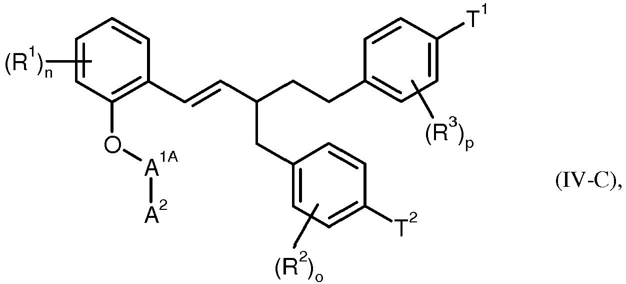 in welcher A^{1A}, A², R¹, R², R³, n, o, p, T¹ und T² jeweils die oben angegebenen Bedeutungen haben,
         alkyliert
   und die resultierenden Verbindungen der Formel (IV-A) bzw. (IV-C) dann durch Hydrolyse der Ester- bzw. Nitril-Gruppen T¹ und T² in die Dicarbonsäuren der Formeln (I-A) bzw. (I-B) überführt und die Verbindungen der Formeln (I-A) bzw. (I-B) gegebenenfalls mit den entsprechenden (i) Lösungsmitteln und/oder (ii) Basen oder Säuren zu ihren Solvaten, Salzen und/oder Solvaten der Salze umsetzt.

Inerte Lösungsmittel für die Verfahrensschritte (II) + (III-A) → (IV-A) und (II) + (III-B) → (IV-B) sind beispielsweise Ether wie Diethylether, Tetrahydrofuran, Glykoldimethylether oder Diethylenglykoldimethylether, oder Kohlenwasserstoffe wie Benzol, Toluol, Xylol, Pentan, Hexan, Heptan, Cyclohexan oder Erdölfraktionen, oder Gemischte dieser Lösungsmittel. Bevorzugt wird Tetrahydrofuran im Gemisch mit Hexan verwendet.

Als Basen für diese Verfahrensschritte sind die für eine Wittig-Reaktion üblichen Basen geeignet. Hierzu zählen insbesondere starke Basen wie *n*-, *sek.-* oder *tert.*-Butyllithium, Lithiumdiisopropylamid (LDA) oder Lithium-, Natrium- oder Kalium-bis(trimethylsilyl)amid. Bevorzugt ist *n*-Butyllithium.

Die Umsetzungen (II) + (III-A) → (IV-A) und (II) + (III-B) → (IV-B) werden im Allgemeinen in einem Temperaturbereich von -78°C bis +20°C, bevorzugt bei -20°C bis +10°C durchgeführt.

Inerte Lösungsmittel für den Verfahrensschritt (IV-B) + (V) → (IV-C) sind beispielsweise Ether wie Diethylether, Dioxan, Tetrahydrofuran, Glykoldimethylether oder Diethylenglykoldimethylether, oder andere Lösungsmittel wie Acetonitril, Dimethylformamid, Dimethylsulfoxid, *N,N'-*Dimethylpropylenharnstoff (DMPU) oder *N*-Methylpyrrolidon (NMP). Ebenso ist es möglich, Gemischte der genannten Lösungsmittel einzusetzen. Bevorzugt wird Acetonitril verwendet.

Für diesen Verfahrensschritt geeignete Basen sind insbesondere Kaliumcarbonat, Natrium- oder Kaliumhydrid, Lithiumdiisopropylamid oder *n*-Butyllithium. Bevorzugt wird Kaliumcarbonat verwendet.

Die Reaktion (IV-B) + (V) → (IV-C) wird im Allgemeinen in einem Temperaturbereich von +20°C bis +120°C, bevorzugt bei +50°C bis +100°C durchgeführt.

Die Hydrolyse der Ester- bzw. Nitril-Gruppen T¹ und T² in den Verfahrensschritten (IV-A) → (I) und (IV-C) → (I) erfolgt nach üblichen Methoden, indem man die Ester bzw. Nitrile in inerten Lösungsmitteln mit Säuren oder Basen behandelt, wobei bei letzterem die zunächst entstehenden Salze durch Behandeln mit Säure in die freien Carbonsäuren überführt werden. Im Falle der *text.-*Butylester erfolgt die Esterspaltung bevorzugt mit Säuren.

Bei unterschiedlichen Gruppen T¹ und T² kann die Hydrolyse gegebenenfalls simultan in einer Eintopf-Reaktion oder in zwei separaten Reaktionsschritten durchgeführt werden.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemischte der genannten Lösungsmittel einzusetzen. Im Falle einer basischen Ester-Hydrolyse werden bevorzugt Gemischte von Wasser mit Dioxan, Tetrahydrofuran, Methanol und/oder Ethanol, bei der Nitril-Hydrolyse bevorzugt Wasser oder n-Propanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium-, Kalium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemischte gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von 0°C bis +100°C, bevorzugt bei +20°C bis +60°C. Die Nitril-Hydrolyse wird im Allgemeinen in einem Temperaturbereich von +50°C bis +150°C, bevorzugt bei +90°C bis +110°C durchgeführt.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die Aldehyde der Formel (II) können in Analogie zu literaturbekannten Verfahren beispielsweise über eine sequentielle Dialkylierung von Malonsäurediallylester mit Verbindungen der Formeln (VI) und (VII) 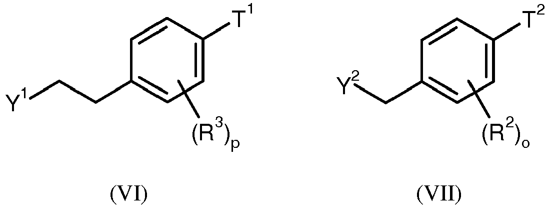
in welchen R², R³, o, p, T¹ und T² jeweils die oben angegebenen Bedeutungen haben und
Y¹ und Y² gleich oder verschieden sind und für eine Abgangsgruppe, wie beispielsweise Halogen, Mesylat oder Tosylat, stehen,
zu Verbindungen der Formel (VIII) 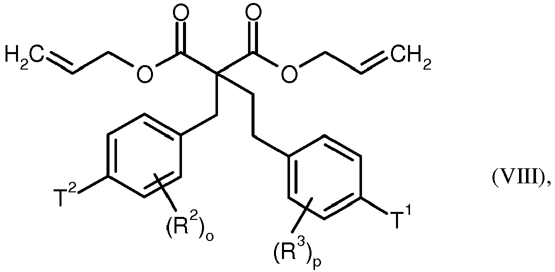
   in welcher R², R³, o, p, T¹ und T² jeweils die oben angegebenen Bedeutungen haben, anschließende Esterspaltung zu Verbindungen der Formel (IX) 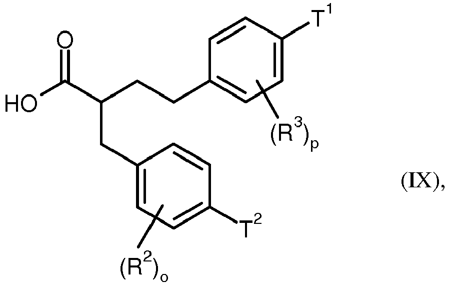
      in welcher R², R³, o, p, T¹ und T² jeweils die oben angegebenen Bedeutungen haben,
      und nachfolgende Reduktion der Carbonsäure-Gruppierung hergestellt werden (siehe auch nachfolgende Reaktionsschemata 1 und 2).

Die Verbindungen der Formeln (III-A) und (III-B) können nach literaturüblichen Verfahren durch Umsetzung von Verbindungen der Formel (X-A) bzw. (X-B) 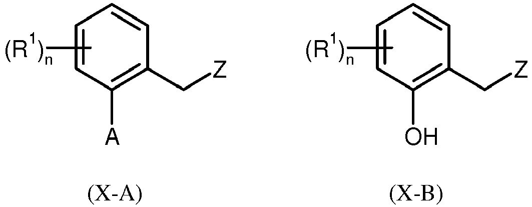
in welchen A, R¹ und n jeweils die oben angegebenen Bedeutungen haben und
Z für eine Abgangsgruppe, wie beispielsweise Halogen oder Tosylat, oder für Hydroxy steht,
mit beispielsweise Triphenylphosphin bzw. (im Falle von Z = OH) Triphenylphosphin-Hydrobromid erhalten werden (siehe auch nachfolgendes Reaktionsschema 3).

Die Verbindungen der Formeln (V), (VI), (VII), (X-A) und (X-B) sind kommerziell erhältlich, literaturbekannt oder können in Analogie zu literaturbekannten Verfahren hergestellt werden (zur Herstellung der erfindungsgemäßen Verbindungen insgesamt vergleiche auch die in EP 0 341 551-A1, WO 01/19355, WO 01/19776 sowie WO 01/19778 beschriebenen Herstellverfahren).

Eine Trennung der erfindungsgemäßen Verbindungen in die entsprechenden Enantiomere und/oder Diastereomere kann gegebenenfalls, je nach Zweckmäßigkeit, auch bereits auf der Stufe der Verbindungen (IV-A), (IV-B), (IV-C) oder (IX) erfolgen, welche dann in separierter Form entsprechend der zuvor beschriebenen Verfahrenssequenz weiter umgesetzt werden. Eine solche Auftrennung der Stereoisomeren läßt sich nach üblichen, dem Fachmann bekannten Methoden durchführen; vorzugsweise werden chromatographische Verfahren oder eine Trennung über diastereomere Salze verwendet.

Die Herstellung der erfindungsgemäßen Verbindungen kann durch die folgenden Syntheseschemata veranschaulicht werden:

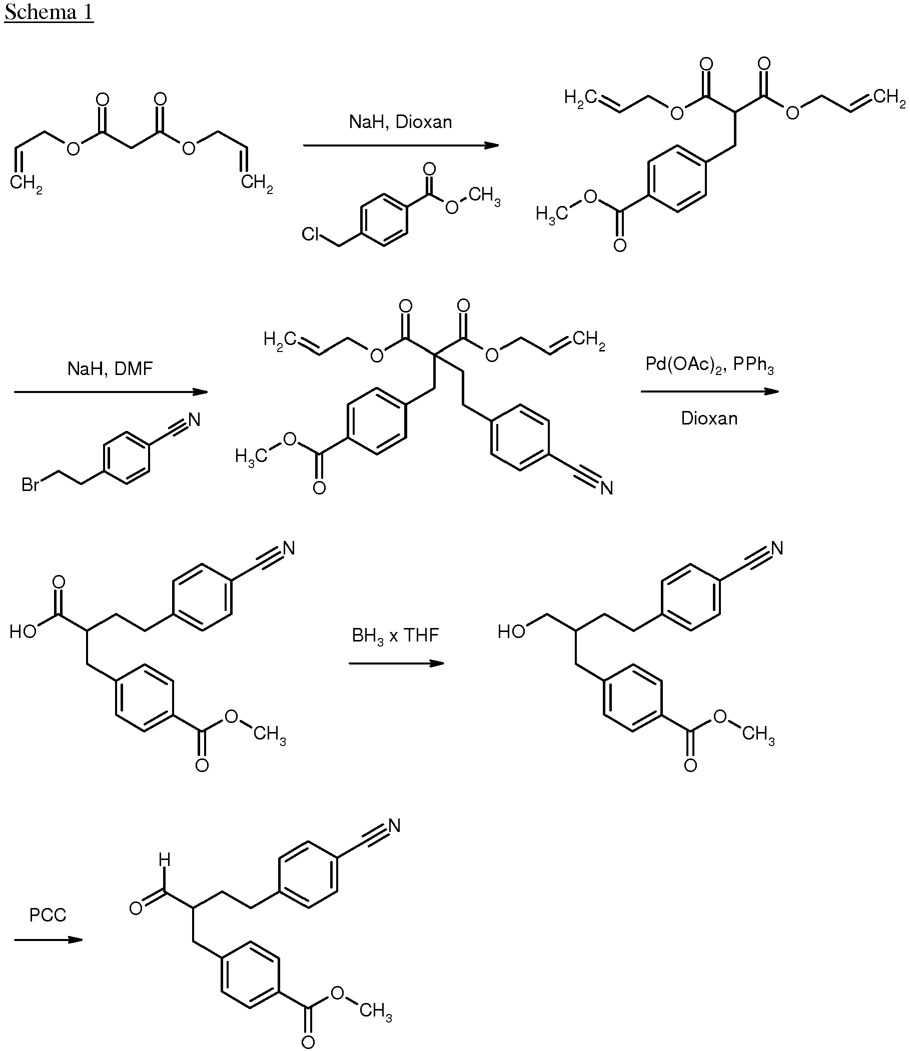

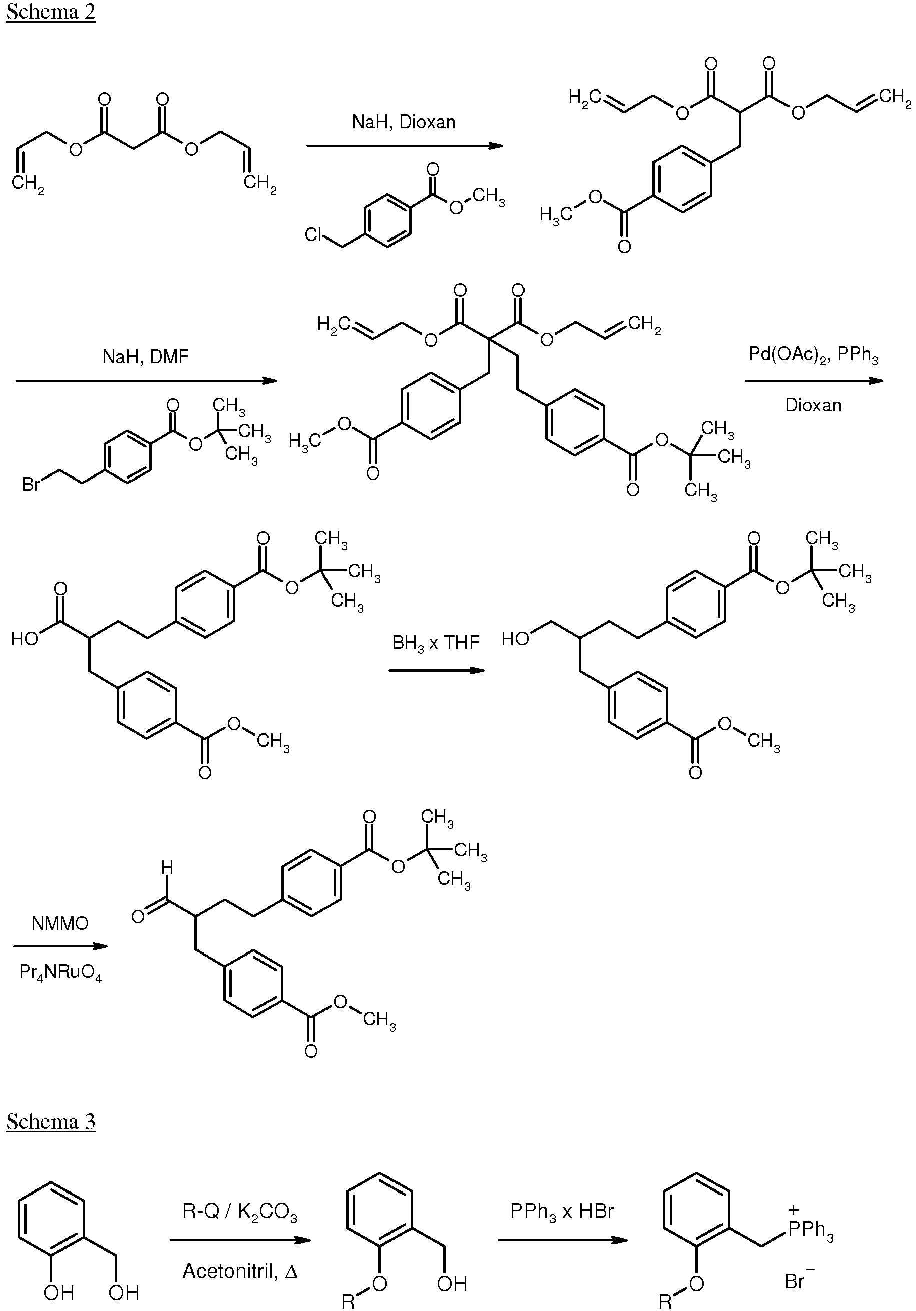

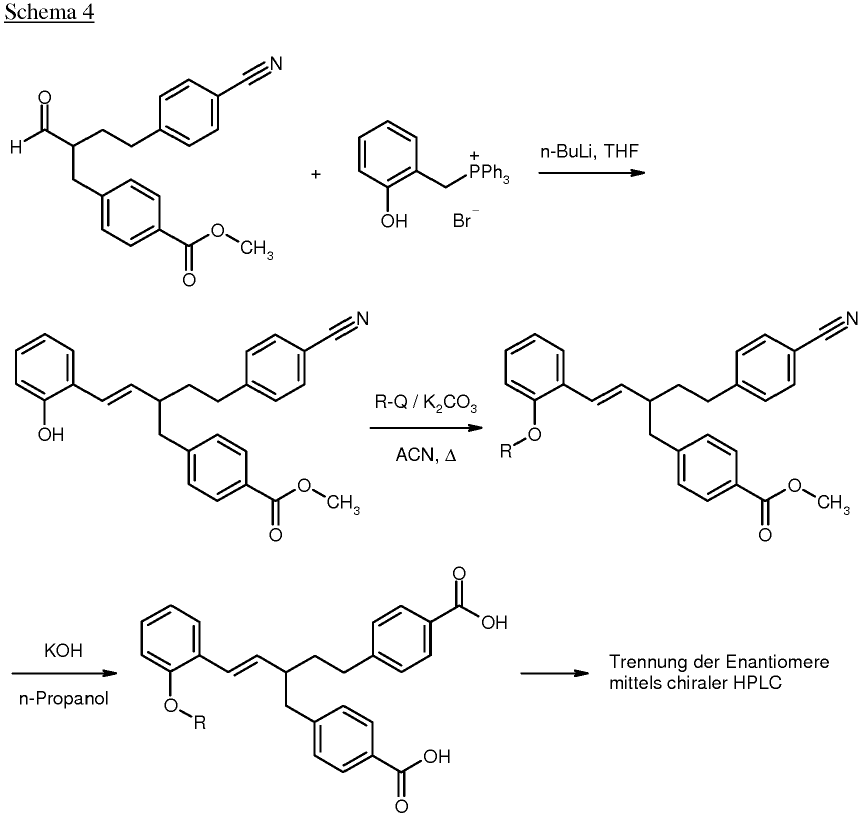

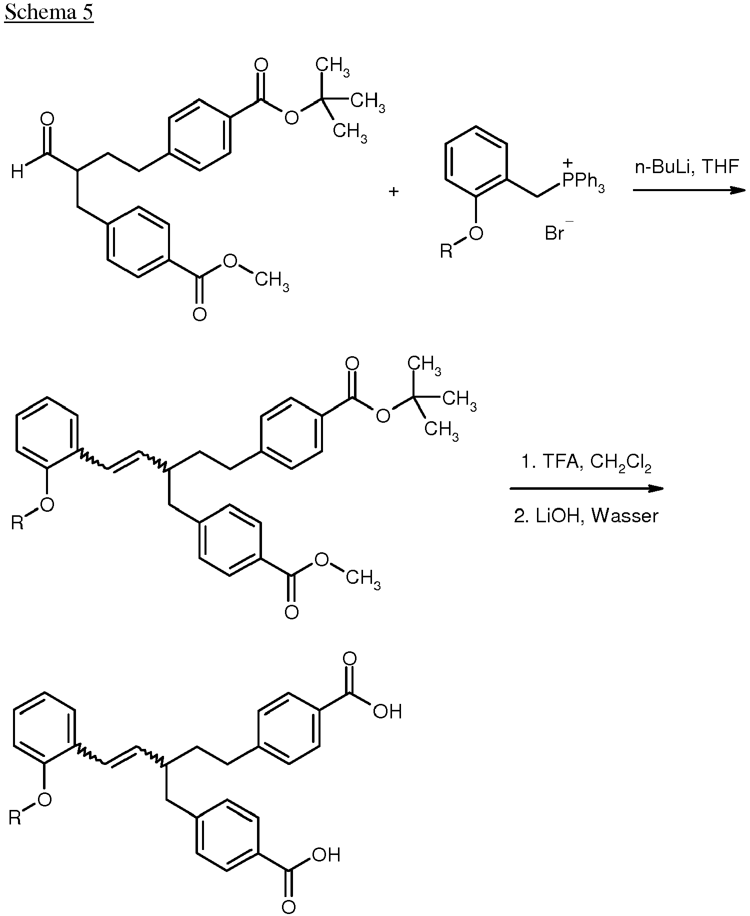

[Abkürzungen: Ac = Acetyl; ACN = Acetonitril; Bu = Butyl; DMF = Dimethylformamid; NMMO = *N*-Methylmorpholin-*N*-oxid; PCC = Pyridiniumchlorochromat; Ph = Phenyl; Pr = Propyl; Q = Abgangsgruppe, z.B. Halogen; TFA = Trifluoressigsäure; THF = Tetrahydrofuran].

Die erfindungsgemäßen Verbindungen besitzen wertvolle pharmakologische Eigenschaften und können zur Vorbeugung und Behandlung von Erkrankungen bei Menschen und Tieren verwendet werden.

Als besonderes und überraschendes Merkmal weisen die Verbindungen der vorliegenden Erfindung vorteilhafte pharmakokinetische Eigenschaften wie beispielsweise eine erhöhte Bioverfügbarkeit und/oder eine verlängerte Wirkdauer nach oraler Gabe auf.

Die erfindungsgemäßen Verbindungen führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte Aktivierung der löslichen Guanylatcyclase und einen intrazellulären cGMP-Anstieg vermittelt.

Die erfindungsgemäßen Verbindungen können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, pulmonaler Hypertonie, peripheren und kardialen Gefäßerhrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, zur Verhinderung von Restenosen wie nach Thrombolysetherapien, percutan-transluminalen Angioplastien (PTA), percutan-transluminalen Koronarangioplastien (PTCA) und Bypass sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz, von Osteoporose, Glaukom und Gastroparese eingesetzt werden.

Außerdem können die erfindungsgemäßen Verbindungen zur Behandlung von primärem und sekundäre Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, CREST-Syndrom, Erythematose, Onychomykose sowie von rheumatischen Erkrankungen verwendet werden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", alters assoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Weiterer Gegenstand der vorliegenden Erfindung ist die Verwendung der erfindungsgemäßen Verbindungen zur Behandlung und/oder Prävention von Erkrankungen., insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittels, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriunmitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise BAY 59-7939, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-ganmla-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele 1-6, 11-22, 29-43, 45-46 und 53-58 erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- abs.: absolut
- aq.: wässrig
- Bsp.: Beispiel
- CI: chemische Ionisation (bei MS)
- DC: Dünnschichtchromatographie
- DCI: direkte chemische Ionisation (bei MS)
- DMF: Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- eq.: Äquivalent(e)
- ESI: Elektrospray-Ionisation (bei MS)
- GC: Gaschromatographie
- h: Stunde(n)
- HPLC: Hochdruck-, Hochleistungsflüssigchromatographie
- LC-MS: Flüssigchromatographie-gekoppelte Massenspektroskopie
- min: Minute(n)
- MS: Massenspektroskopie
- NMR: Kernresonanzspektroskopie
- R_{f}: Retentionsindex (bei DC)
- RT: Raumtemperatur
- Rₜ: Retentionszeit (bei HPLC)
- THF: Tetrahydrofuran
- UV: Ultraviolett-Spektroskopie
- v/v: Volumen zu Volumen-Verhältnis (einer Lösung)

### LC/MS-Methoden:

### Methode 1 (LC-MS)

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasse + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS)

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS)

Instrument: Micromass Platform LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS)

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 5 (LC-MS)

Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 6 (LC-MS)

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1, Eluent B: Acetonitril + 500 µl 50%-ige Ameisensäure / 1; Gradient: 0.0 min 10% B → 7.0 min 95% B → 9.0 min 95% B; Fluss: 0.0 min 1.0 ml/min → 7.0 min 2.0 ml/min → 9.0 min 2.0 ml/min; Ofen: 35°C; UV-Detektion: 210 nm.

### Methode 7 (LC-MS)

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 8 (LC-MS)

Instrument: Micromass Quattro LCZ mit HPLC Agilent Serie 1100; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### GC/MS-Methoden:

### Methode 1 (GC-MS)

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 2 (GC-MS)

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (8.7 min halten).

### HPLC-Methoden:

### Methode 1 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 2 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

### Ausgangsverbindungen und Intermediate:

### Beispiel 1A

### (5-Brompentyl)benzol

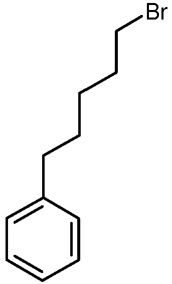

Eine Lösung von 416.7 ml (1.83 mol) 48%-iger Bromwasserstoffsäure wird bei 0°C mit 50 g (0.304 mol) 5-Phenylpentan-1-ol versetzt und 30 min bei 0°C nachgerührt. Anschließend wird die Reaktionslösung 12 Stunden bei 100°C gerührt. Nach vollständiger Umsetzung wird der Ansatz auf Raumtemperatur abgekühlt und mit 200 ml Essigsäureethylester versetzt. Nach Extraktion wird die organische Phase abgetrennt, mit gesättigter Natriumhydrogencarbonat-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (laufmittel: Cyclohexan) gereinigt. Es werden 59.4 g (0.26 mol, 86% Ausbeute) einer farblosen Flüssigkeit erhalten.

¹H-NMR (300 MHz, CDCl₃, δ/ppm): 7.32-7.22 (2H, m), 7.21-7.11 (3H, m), 3.40 (2H, t), 2.61 (2H, t), 1.97-1.81 (2H, m), 1.72-1.58 (2H, m), 1.56-1.39 (2H, m).

MS (CI): m/z = 226 (M⁺).

### Beispiel 2A

### [4-(2-Bromethyl)phenyl]methanol

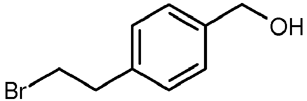

Eine Lösung von 2 g (8.73 mmol) 4-(2-Bromethyl)benzoesäure in 50 ml trockenem THF wird bei -10°C tropfenweise mit 13.10 ml (13.10 mmol) 1 M Boran-THF-Komplex versetzt. Nach Erwärmung auf Raumtemperatur wird der Ansatz eine Stunde nachgerührt. Nach vollständiger Umsetzung wird der Ansatz mit gesättigter Ammoniumchlorid-Lösung versetzt, in Essigsäureethylester aufgenommen, die organische Phase abgetrennt und über Natriumsulfat getrocknet. Nach Filtration wird das Lösungsmittel im Vakuum entfernt. Es werden 1.67 g (7.76 mmol, 79% Ausbeute) eines farblosen Öls erhalten, das ohne weitere Reinigung in der nächsten Stufe eingesetzt wird.

¹H-NMR (300 MHz, DMSO-d₆, δ/ppm): 7.33-7.28 (4H, m), 5.14 (1H, t), 4.48 (2H, d), 3.77 (2H, t), 3.11 (2H, t).

MS (DCI, NH₃): m/z = 232 (M+NH₄⁺).

### Beispiel 3A

### 4-(2-Bromethyl)benzaldehyd

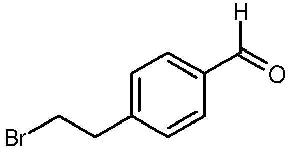

### Verfahren 1:

Eine Lösung von 200 mg (0.93 mmol) [4-(2-Bromethyl)phenyl]methanol in 20 ml Dichlormethan wird mit 240.5 mg (1.12 mmol) Pyridiniumchlorochromat (PCC) versetzt und 3 Stunden bei Raumtemperatur gerührt. Anschließend wird die Reaktionslösung mit ca. 2 g Kieselgel versetzt und bis zur Trockene eingeengt. Der erhaltene Rückstand wird durch Flash-Chromatographie an Kieselgel (Laufmittel: Cyclohexan/Essigsäureethylester 4:1) gereinigt. Es werden 183 mg (0.85 mmol, 82% Ausbeute) eines farblosen Feststoffs erhalten.

### Verfahren 2:

Eine Lösung von 44.4 g (0.38 mol) Dichlormethyl-methylether in 230 ml Dichlormethan wird unter Kühlung (4-5°C) innerhalb von 10 min mit 42.26 ml (0.385 mmol) Titantetrachlorid versetzt und 1 Stunde nachgerührt. Danach werden 64.89 g (0.34 mol) (2-Bromethyl)benzol, gelöst in 24 ml Dichlormethan, bei 5-7°C innerhalb von 50 min zu der Reaktionslösung dosiert. Anschließend erwärmt man die Reaktionslösung langsam auf Raumtemperatur und lässt den Ansatz über Nacht rühren. Nach vollständiger Umsetzung werden sehr vorsichtig innerhalb von 1 Stunde 140 ml Wasser zugetropft (Vorsicht: zunächst endotherme Reaktion infolge Gasentwicklung, dann exotherme Reaktion bis 30°C, Kühlung erforderlich). Die Reaktionslösung wird dann dreimal mit Dichlormethan extrahiert, die vereinigten organischen Phasen mit 170 ml Wasser gewaschen sowie mit 115 ml Natriumhydrogencarbonat-Lösung neutralisiert und über Natriumsulfat getrocknet. Nach Filtration wird das Lösungsmittel im Vakuum entfernt. Der erhaltene Rückstand wird durch Flash-Chromatographie an Kieselgel (Laufmittel: Dichlormethan/Petrolether 1:2 → 1:1) gereinigt. Es werden 29.3 g (0.14 mol, 37% Ausbeute) eines farblosen Feststoffes erhalten.

¹H-NMR (300 MHz, DMSO-d₆, δ/ppm): 9.99 (1H, s), 7.88 (2H, d), 7.52 (2H, d), 3.80 (2H, t), 3.24 (2H, t).

MS (EI): m/z = 212 (M⁺).

### Beispiel 4A

### 4-(2-Bromethyl)benzonitril

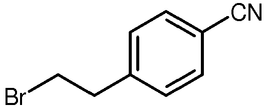

Eine Lösung von 29.3 g (0.14 mol) 4-(2-Bromethyl)benzaldehyd in 112.4 ml Ameisensäure wird mit 12.42 g (0.18 mol) Hydroxylamin-Hydrochlorid versetzt und 2 Stunden unter Rückfluss erhitzt. Nach langsamem Abkühlen auf Raumtemperatur werden 670 ml Wasser zugesetzt und das Reaktionsgemisch langsam unter Kühlung mit 6 N Natronlauge neutralisiert. Anschließend wird der Ansatz dreimal mit Methyl-*tert*.-butylether extrahiert. Die vereinigten organischen Phasen werden über Magnesiumsulfat getrocknet und zur Trockene eingeengt. Der erhaltene Rückstand wird durch Flash-Chromatographie an Kieselgel (laufmittel: Dichlormethan) gereinigt. Es werden 21.3 g (0.10 mol, 74% Ausbeute) eines gelblichen Feststoffs erhalten.

¹H-NMR (400 MHz, DMSO-d₆, δ/ppm): 7.80 (2H, d), 7.51 (2H, d), 3.77 (2H, t), 3.22 (2H, t).

MS (DCI, NH₃): m/z = 227 (M+NH₄⁺).

### Beispiel 5A

### 2-(4-Methoxycarbonylbenzyl)malonsäurediallylester

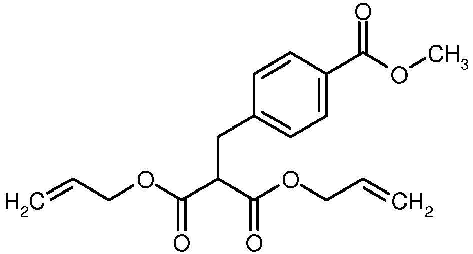

Eine Lösung von 56.7 g (0.3 mol) Malonsäurediallylester in 375 ml Dioxan und 75 ml THF wird bei 0°C portionsweise mit 14.42 g (0.36 mol) Natriumhydrid versetzt (Vorsicht: Wasserstoffentwicklung). Nach Erwärmen auf Raumtemperatur wird der Ansatz 1 Stunde bei 40°C gerührt. Anschließend werden 111.88 g (0.6 mol) 4-Chlormethyl-benzoesäuremethylester, gelöst in 375 ml Dioxan, langsam bei 40°C zugetropft und die Reaktionslösung dann über Nacht bei 110°C (Badtemperatur) gerührt. Nach Abkühlen auf Raumtemperatur wird das Reaktionsgemisch auf 1200 ml Wasser gegeben. Dabei ist darauf zu achten, dass der pH-Wert <7 ist (gegebenenfalls werden wenige ml 1 M Salzsäure bis ca. pH 2 zudosiert). Der Ansatz wird dann dreimal mit Essigsäureethylester extrahiert, die vereinigten organischen Phasen mit gesättigter Natriumchlorid-Lösung gewaschen und über Natriumsulfat getrocknet. Nach Filtration wird das Lösungsmittel im Vakuum bis zur Trockene eingeengt. Das erhaltene Rohprodukt wird durch Flash-Chromatographie an 3 kg Kieselgel (Laufmittel: Petrolether/Essigsäureethylester 10:1) gereinigt. Es werden 85.4 g (0.26 mol, 85% Ausbeute) eines farblosen Feststoffes erhalten.

¹H-NMR (300 MHz, CDCl₃, δ/ppm): 7.96 (2H, d), 7.29 (2H, d), 5.91-5.74 (2H, m), 5.32-5.17 (4H, m), 4.59 (4H, d), 3.93 (3H, s), 3.74 (1H, t), 3.31 (2H, d).

MS (DCI, NH₃): m/z = 349 (M+NH₄⁺).

### Beispiel 6A

### 2-[2-(4-Cyanophenyl)ethyl]-2-(4-methoxycarbonylbenzyl)malonsäurediallylester

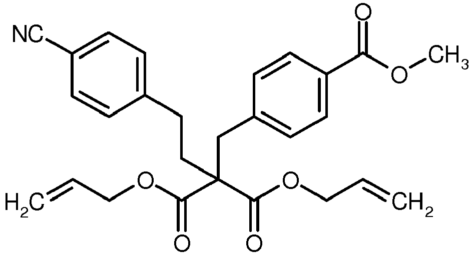

Eine Lösung von 55.71 g (0.17 mol) 2-(4-Methoxycarbonylbenzyl)malonsäurediallylester in 34 ml DMF wird bei 0°C portionsweise mit 6.70 g (0.17 mol) Natriumhydrid versetzt. Anschließend lässt man die Reaktionslösung auf Raumtemperatur kommen und rührt 1 Stunde nach. Dann wird die Reaktionslösung wieder auf 0°C abgekühlt, mit 42.98 g (0.20 mol) 4-(2-Bromethyl)benzonitril in 21 ml DMF versetzt und 30 min bei 0°C nachgerührt. Der Ansatz wird danach bei Raumtemperatur über Nacht gerührt. Das Reaktionsgemisch wird tropfenweise mit Wasser versetzt, dreimal mit Essigsäureethylester extrahiert, die vereinigten organischen Phasen mit gesättigter Kochsalz-Lösung gewaschen und über Natriumsulfat getrocknet. Nach Filtration wird das Lösungsmittel im Vakuum bis zur Trockene eingeengt. Das erhaltene Rohprodukt wird durch Flash-Chromatographie an 3 kg Kieselgel (Laufmittel: Petrolether/Essigsäureethylester 3:1) gereinigt. Es werden 36 g (78 mmol, 46% Ausbeute) eines farblosen Feststoffes erhalten.

¹H-NMR (300 MHz, CDCl₃, δ/ppm): 7.95 (2H, d), 7.55 (2H, d), 7.21 (4H, t), 5.97-5.69 (2H, m), 5.40-5.23 (4H, m), 4.62 (4H, d), 3.92 (3H, s), 3.40 (2H, s), 2.72-2.61 (2H, m), 2.13-2.01 (2H, m).

MS (DCI, NH₃): m/z = 479 (M+NH₄⁺).

### Beispiel 7A

### 4-[2-Carboxy-4-(4-cyanophenyl)butyl]benzoesäuremethylester

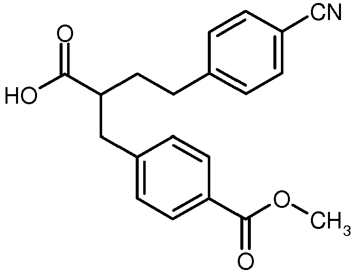

Eine Lösung von 43.5 g (0.09 mol) 2-[2-(4-Cyanophenyl)ethyl]-2-(4-methoxycarbonylbenzyl)-malonsäurediallylester, 1.67 g (0.01 mol) Triphenylphosphin und 410 mg Palladiumacetat in 505 ml Dioxan wird bei Raumtemperatur mit einer Lösung von 41.8 ml (0.3 mol) Trimethylamin und 8.6 ml (0.23 mol) Ameisensäure in 500 ml Dioxan versetzt. Das Reaktionsgemisch wird anschließend 2 Stunden bei 100°C gerührt. Nach vollständigem Umsatz wird die Reaktionslösung abgekühlt und das Lösungsmittel im Vakuum entfernt. Der erhaltene Rückstand wird durch Flash-Chromatographie an Kieselgel (Laufmittel: Dichlormethan/Methanol 50:1) gereinigt. Es werden 25 g (74 mmol, 82% Ausbeute) eines farblosen Feststoffes erhalten.

¹H-NMR (400 MHz, DMSO-d₆, δ/ppm): 12.55-12.24 (1H, breit), 7.86 (2H, d), 7.72 (2H, d), 7.38 (2H, d), 7.32 (2H, d), 3.84 (3H, s), 2.99-2.81 (2H, m), 2.78-2.55 (3H, m), 1.90-1.67 (2H, m).

MS (ESI): m/z = 338 (M+H⁺).

### Beispiel 8A

### 4-[4-(4-Cyanophenyl)-2-hydroxymethyl-butyl]benzoesäuremethylester

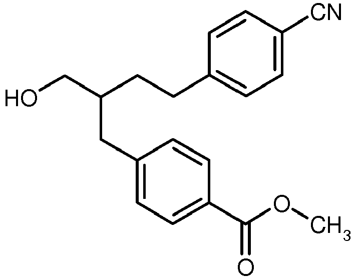

Zu einer Lösung von 4.2 g (12.98 mmol) 4-[2-Carboxy-4-(4-cyanophenyl)butyl]benzoesäuremethylester in 40 ml THF werden 20.6 ml (20.6 mmol) einer 1 M Boran-THF-Komplex-Lösung bei -15°C zugetropft und das Reaktionsgemisch 3 Stunden bei dieser Temperatur nachgerührt. Danach werden nochmals 10 ml (10 mmol) 1 M Boran-THF-Komplex-Lösung zugetropft und weitere 30 min bei -15°C gerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und wiederum vom Lösungsmittel befreit. Das erhaltene Rohprodukt wird durch Flash-Chromatographie an 150 g Kieselgel (Laufmittel: Essigsäureethylester/Petrolether 1:1) gereinigt. Es werden 3.1 g (90% Reinheit, 83% Ausbeute) eines farblosen Feststoffes erhalten.

¹H-NMR (300 MHz, DMSO-d₆, δ/ppm): 7.88 (2H, d), 7.71 (2H, d), 7.46 (4H, t), 4.54 (1H, t), 3.83 (3H, s), 3.41 (2H, t), 2.80-2.55 (4H, m), 1.79-1.39 (3H, m).

MS (ESI): m/z = 324 (M+H⁺).

### Beispiel 9A

### 4-[4-(4-Cyanophenyl)-2-formyl-butyl]benzoesäuremethylester

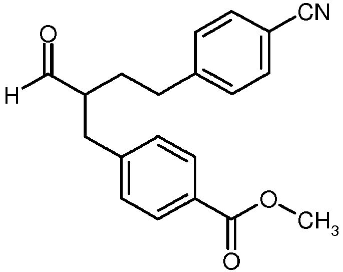

Eine Lösung von 5.7 g (17.63 mmol) 4-[4-(4-Cyanophenyl)-2-hydroxymethyl-butyl]benzoesäuremethylester in 250 ml Dichlormethan wird mit 4.56 g (21.15 mmol) Pyridiniumchlorochromat (PCC) versetzt und 5 Stunden bei Raumtemperatur gerührt. Nach vollständigem Umsatz werden ca. 10 g Kieselgel zugegeben und das Lösungsmittel im Vakuum bis zur Trockene entfernt. Der Rückstand wird durch Flash-Chromatographie an Kieselgel (Laufmittel: Cyclohexan/Essigsäureethylester 10:1 → 4:1) gereinigt. Es werden 4.16 g (12.94 mmol, 73% Ausbeute) eines farblosen Feststoffs erhalten.

¹H-NMR (300 MHz, DMSO-d₆, δ/ppm): 9.68 (1H, s), 7.88 (2H, d), 7.73 (2H, d), 7.47 (4H, dd), 3.86 (3H, s), 3.14-3.02 (1H, m), 2.92-2.80 (1H, m), 2.78-2.54 (3H, m), 1.98-1.81 (1H, m), 1.76-1.60 (1H, m).

MS (DCI, NH₃): m/z = 339 (M+NH₄⁺).

### Beispiel 10A

*E*-4-[2-[2-(4-Cyanophenyl)ethyl]-4-(2-hydroxyphenyl)but-3-enyl]benzoesäuremethylester

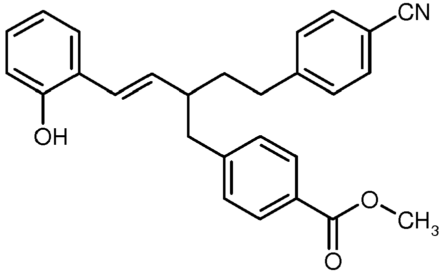

Zu einer Lösung von 1820 mg (4.05 mmol) (2-Hydroxybenzyl)-triphenylphosphoniumbromid in 10 ml THF werden bei 0°C 5.9 ml (9.45 mmol) einer 1.6 M Lösung von n-Butyllithium in Hexan langsam zugetropft. Anschließend werden bei dieser Temperatur 1085 mg (3.38 mmol) 4-[4-(4-Cyanophenyl)-2-formyl-butyl]benzoesäuremethylester in 10 ml THF langsam zudosiert. Nach Erwärmung auf Raumtemperatur wird die Reaktionslösung 12 Stunden nachgerührt, anschließend mit etwas Wasser versetzt und bis zur Trockene eingeengt. Der Rückstand wird in Essigsäureethylester aufgenommen, mit Wasser und gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/ Essigsäureethylester 4:1 → 2:1) gereinigt. Es werden 1150 mg (2.79 mmol, 83% Ausbeute) eines farblosen Feststoffs erhalten.

¹H-NMR (300 MHz, DMSO-d₆, δ/ppm): 9.39 (1H, s), 7.82 (2H, d), 7.60 (2H, d), 7.41-7.27 (5H, m), 7.01 (1H, t), 6.81-6.68 (2H, m), 6.45 (1H, d), 6.13-5.99 (1H, m), 3.81 (3H, s), 2.92-2.58 (5H, m), 1.86-1.56 (2H, m).

MS (DCI, NH₃): m/z = 429 (M+NH₄⁺).

### Beispiel 11A

### 4-{ (3E)-4-{2-[(4-tert.-butylbenzyl)oxy]phenyl}-2-[2-(4-cyanophenyl)ethyl]but-3-en-1-yl}benzoesäuremethylester

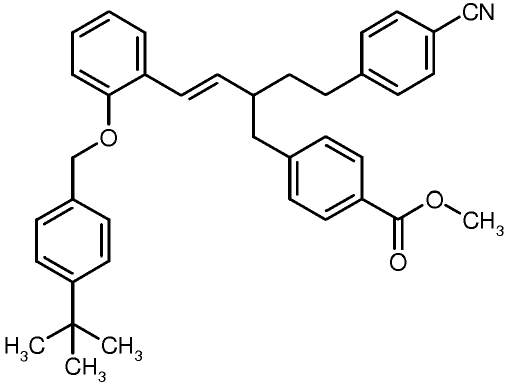

Eine Lösung von 2 g (3.6 mmol) *E*-4-[2-[2-(4-Cyanophenyl)ethyl]-4-(2-hydroxyphenyl)-but-3-enyl]benzoesäuremethylester in 8 ml trockenem Acetonitril wird mit 2.20 g (9.71 mmol) 4-(*text-*Butyl)benzylbromid und 2.02 g (14.59 mmol) wasserfreiem Kaliumcarbonat versetzt und dann 12 Stunden unter Rückfluss erhitzt. Anschließend wird der Ansatz filtriert und das Filtrat zur Trockene eingeengt. Der Rückstand wird chromatographisch an Kieselgel (Laufmittel: Cyclohexan/Essigsäureethylester 10:1) aufgereinigt. Es werden 1.9 g (3.30 mmol, 97% Reinheit, 92% Ausbeute) eines Öls isoliert.

¹H-NMR (400 MHz, CDCl₃, δ/ppm): 7.94-7.89 (2H, m), 7.49-7.30 (7H, m), 7.21-7.12 (5H, m), 6.97-6.90 (2H, m), 6.68-6.62 (1H, m), 5.06-5.02 (2H, m), 3.89 (3H, s), 2.83-2.69 (3H, m), 2.65-2.39 (2H, m), 1.86-1.59 (2H, m), 1.33 (9H, s).

LC-MS (Methode 2): Rₜ = 3.37 min; m/z = 557 (M⁺).

### Beispiel 12A

### 4-((3E)-2-[2-(4-Cyanophenyl)ethyl]-4-{2-[(5-phenylpentyl)oxy]phenyl}but-3-en-1-yl)benzoesäuremethylester

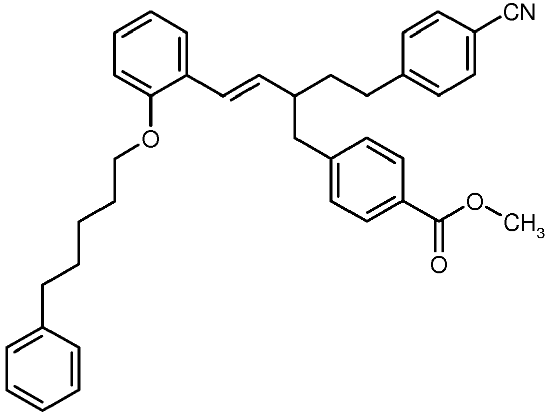

Eine Lösung von 700.8 mg (1.7 mmol) 4-[(3*E*)-2-[2-(4-Cyanophenyl)ethyl]-4-(2-hydroxyphenyl)-but-3-en-1-yl]benzoesäuremethylester in 65 ml Acetonitril wird mit 464.2 mg (2.04 mmol) (5-Brompentyl)benzol und 353.05 mg (2.55 mmol) Kaliumcarbonat versetzt und dann 12 Stunden unter Rückfluss gerührt. Nach dem Abkühlen wird das Kaliumcarbonat abfiltriert und das Filtrat eingeengt. Der erhaltene Rückstand wird über Flash-Chromatographie an Kieselgel gereinigt (Laufmittel: Cyclohexan/Essigsäureethylester 7:3). Es werden 945 mg (1.7 mmol, 99.5% Ausbeute) erhalten.

LC-MS (Methode 2): Rₜ = 3.37 min; MS (ESIpos): m/z = 575 (M+NH₄⁺).

### Beispiel 13A

### 4-[(3E)-2-[2-(4-Cyanophenyl)ethyl]-4-(2-{[4-(2-phenylethyl)benzyl]oxy}phenyl)but-3-en-1-yl]-benzoesäuremethylester

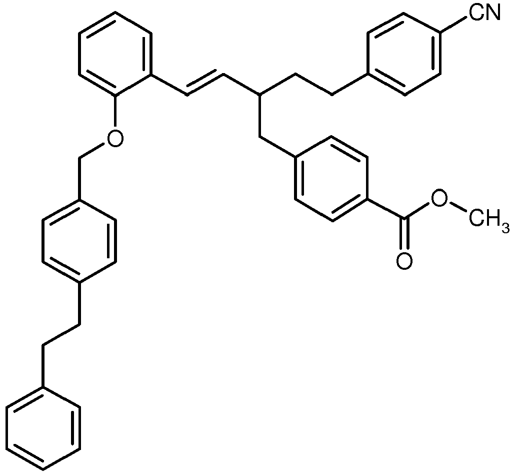

Eine Lösung von 411 mg (1 mmol) 4-[(3*E*)-2-[2-(4-Cyanophenyl)ethyl]-4-(2-hydroxyphenyl)but-3-en-1-yl]benzoesäuremethylester in 10 ml trockenem Acetonitril wird mit 460 mg (2 mmol) 1-(Chlormethyl)-4-(2-phenylethyl)benzol [Herstellung gemäß M. Carrara et al., Arzneim. Forsch. 47 (7), 803-809 (1997)] und 414 mg (3 mmol) wasserfreiem Kaliumcarbonat versetzt und dann 12 Stunden unter Rückfluss erhitzt. Anschließend wird der Ansatz zur Trockene eingeengt. Der Rückstand wird in Essigsäureethylester aufgenommen, mit Wasser und mit gesättigter Natriumchlorid-Lösung gewaschen und über Natriumsulfat getrocknet. Nach Filtration wird die organische Phase eingeengt und das erhaltene Rohprodukt über präparative HPLC gereinigt. Es werden 431 mg (0.7 mmol, 65% Ausbeute) eines Feststoffs erhalten.

¹H-NMR (400 MHz, CDCl₃, δ/ppm): 7.91 (2H, d), 7.43 (2H, d), 7.38 (1H, d), 7.34-7.27 (4H, m), 7.22-7.1 (10H, m), 7.95 (2H, t), 6.63 (1H, d), 6.0 (1H, dd), 5.04 (2H, s), 3.89 (3H, s), 3.0-2.88 (4H, m), 2.8-2.7 (3H, m), 2.63-2.5 (1H, m), 2.5-2.4 (1H, m), 1.86-1.73 (1H, m), 1.7-1.59 (1H, m).

### Beispiel 14A

### 4'-Trifluormethyl-biphenyl-4-carbonsäureethylester

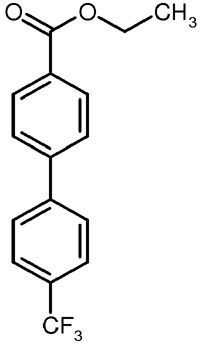

7 g (30.56 mmol) 4-Brombenzoesäureethylester werden in 60 ml 1,2-Dimethoxyethan gelöst und unter Argon mit 6.96 g (36.67 mmol) 4-Trifluormethylphenylboronsäure, 271 mg Bis(triphenylphosphin)palladium(II)chlorid und mit 40.7 ml einer 2 M wässrigen Natriumcarbonat-Lösung versetzt. Das Reaktionsgemisch wird anschließend 12 Stunden unter Rückfluss gerührt. Dann wird der Ansatz abgekühlt, über 1 g Extrelute filtriert, mit Dichlormethan nachgewaschen und eingeengt. Das erhaltene Rohprodukt wird durch Flash-Chromatographie an Kieselgel (Laufmittel: Cyclohexan/Dichlormethan 2:1) gereinigt. Es werden 6.31 g (21.4 mmol, 70% Ausbeute) eines farblosen Feststoffs erhalten.

¹H-NMR (300 MHz, CDCl₃, δ/ppm): 8.17 (2H, d), 7.72 (4H, s), 7.67 (2H, d), 4.41 (2H, q), 1.43 (3H, t).

MS (EI): m/z = 294 (M⁺).

### Beispiel 15A

### (4'-Trifluormethyl-biphenyl-4-yl)-methanol

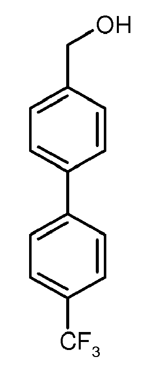

Eine Lösung von 6.24 g (21.21 mmol) 4'-Trifluormethyl-biphenyl-4-carbonsäureethylester in 60 ml trockenem THF wird bei 0°C tropfenweise mit 12.73 ml (12.73 mmol) einer 1 M Lösung von Lithiumaluminiumhydrid in THF versetzt. Nach vollständiger Umsetzung wird der Ansatz mit gesättigter Ammoniumchlorid-Lösung versetzt, in Essigsäureethylester aufgenommen, die organische Phase abgetrennt und über Natriumsulfat getrocknet. Nach Filtration wird das Lösungsmittel im Vakuum entfernt. Das erhaltene Rohprodukt wird durch Flash-Chromatographie an Kieselgel (Laufmittel: Cyclohexan/Essigsäureethylester 5:1) gereinigt. Es werden 5.1 g (20.21 mmol, 95% Ausbeute) eines farblosen Feststoffes erhalten.

¹H-NMR (300 MHz, DMSO-d₆, δ/ppm): 7.88 (2H, d), 7.82 (2H, d), 7.71 (2H, d), 7.46 (2H, d), 5.23 (1H, t), 4.58 (2H, d).

MS (EI): m/z = 252 (M⁺).

### Beispiel 16A

### 4-Chlormethyl-4'-trifluormethyl-biphenyl

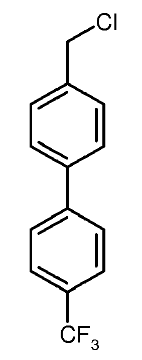

Eine Lösung von 5.0 g (19.82 mmol) (4'-Trifluormethyl-biphenyl-4-yl)-methanol in 40 ml Chloroform wird mit 2.89 ml (39.65 mmol) Thionylchlorid, gelöst in 10 ml Chloroform, versetzt und dann 12 Stunden bei Raumtemperatur gerührt. Nach vollständiger Umsetzung wird das Reaktionsgemisch bis zur Trockene eingeengt, der Rückstand in Essigsäureethylester aufgenommen und mit gesättigter Natriumcarbonat-Lösung gewaschen. Die organische Phase wird abgetrennt, über Natriumsulfat getrocknet und nach Filtration eingeengt. Das erhaltene Rohprodukt wird durch Flash-Chromatographie an Kieselgel (Laufmittel: Cyclohexan/Essigsäureethylester 9:1) gereinigt. Es werden 5.26 g (19.43 mmol, 98% Ausbeute) eines farblosen Feststoffes erhalten.

¹H-NMR (300 MHz, DMSO-d₆, δ/ppm): 7.91 (2H, d), 7.82 (2H, d), 7.78 (2H, d), 7.58 (2H, d), 4.83 (2H, s).

MS (EI): m/z = 270 (M⁺).

### Beispiel 17A

### [2-(4'-Trifluormethyl-biphenyl-4-yl-methoxy)phenyl]methanol

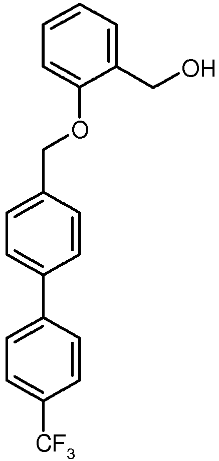

Eine Lösung von 4.59 g (36.94 mmol) 2-Hydroxybenzylalkohol in 200 ml trockenem Acetonitril wird mit 10 g (36.94 mmol) 4-Chlormethyl-4'-trifluormethyl-biphenyl und 6.13 g (44.33 mmol) wasserfreiem Kaliumcarbonat versetzt und dann 12 Stunden unter Rückfluss erhitzt. Anschließend wird der Ansatz zur Trockene eingeengt. Der Rückstand wird in Essigsäureethylester aufgenommen, mit Wasser und mit gesättigter Kochsalz-Lösung gewaschen und über Natriumsulfat getrocknet. Die organische Phase wird eingeengt und das Rohprodukt durch Flash-Chromatographie an Kieselgel (Laufmittel: Cyclohexan/Essigsäureethylester 5:1) gereinigt. Es werden 11.8 g (32.92 mmol, 89% Ausbeute) eines Feststoffs erhalten.

¹H-NMR (300 MHz, DMSO-d₆, δ/ppm): 7.92 (2H, d), 7.87-7.72 (4H, m), 7.60 (2H, d), 7.41 (1H, d), 7.21 (1H, t), 7.03 (1H, d), 6.96 (1H, t), 5.20 (2H, s), 5.03 (1H, t), 4.58 (2H, d).

MS (DCI, NH₃): m/z = 376 (M+NH₄⁺).

### Beispiel 18A

### Triphenyl-[2-(4'-trifluormethyl-biphenyl-4-yl-methoxy)benzyl]phosphoniumbromid

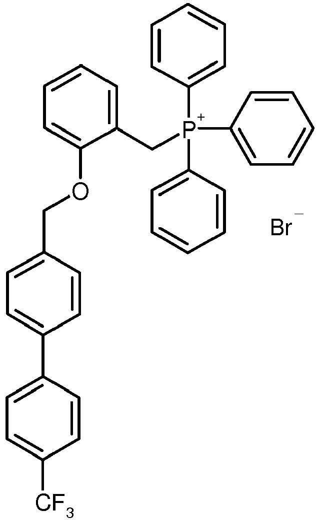

Eine Lösung von 11.7 g (32.65 mmol) [2-(4'-Trifluormethyl-biphenyl-4-yl-methoxy)phenyl]-methanol in 100 ml Acetonitril wird mit 10.64 g (31.02 mmol) Triphenylphosphoniumhydrobromid versetzt und 3 Stunden unter Rückfluss erhitzt. Anschließend wird die Reaktionslösung bis zur Trockene eingeengt und das erhaltene Öl in Diethylether aufgenommen und verrieben. Dabei kristallisiert das Produkt als weißer Feststoff aus. Nach Filtration wird der Feststoff bei 50°C im Trockenofen über Nacht getrocknet. Es werden 20.5 g (30 mmol, 92% Ausbeute) kristallines Produkt erhalten.

¹H-NMR (300 MHz, DMSO-d₆, δ/ppm): 7.99-7.79 (8H, m), 7.78-7.50 (12H, m), 7.32 (4H, d), 7.08 (1H, d), 6.97 (1H, d), 6.86 (1H, t), 5.03 (2H, d), 4.70 (2H, s).

### Beispiel 19A

### 4-Methylbenzoesäure-tert.-butylester

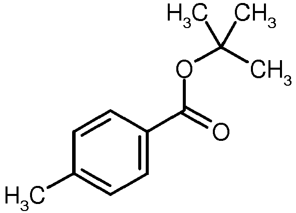

10.52 ml (109.96 mmol) *tert*.-Butanol und 10.46 ml (129.37 mmol) Pyridin werden unter Eiskühlung tropfenweise mit 8.55 ml (64.69 mmol) 4-Toluoylchlorid versetzt. Es wird 12 Stunden bei Raumtemperatur gerührt. Nach beendeter Umsetzung wird Wasser zugegeben und mit Essigsäureethylester extrahiert. Die organische Phase wird nacheinander mit Natriumhydrogencarbonat-Lösung, Wasser und Natriumchlorid-Lösung gewaschen, über Natriumsulfat getrocknet und eingeengt. Der erhaltene Rückstand wird über Flash-Chromatographie an Kieselgel gereinigt (Laufmittels: Cyclohexan/Essigsäureethylester 50:1). Es werden 7.76 g (40.36 mmol, 62% Ausbeute) der Titelverbindung als farbloses Öl erhalten.

¹H-NMR (300 MHz, CDCl₃, δ/ppm): 7.88 (2H, d), 7.2 (2H, d), 2.4 (3H, s), 1.59 (9H, s).

### Beispiel 20A

### 4-(2-Methoxy-2-oxoethyl)benzoesäure-tert.-butylester

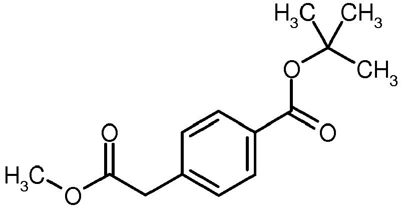

Unter Sauerstoffausschluss werden 11.79 ml (84.11 mmol) Diisopropylamin in 40 ml THF vorgelegt, auf -78°C abgekühlt und 52.57 ml (84.11 mmol) n-Butyllithium (1.6 M Lösung in Hexan) langsam zugetropft. Es wird 10 Minuten bei -78°C gerührt und dann 10.17 ml (84.11 mmol) 1,3-Dimethyltetrahydro-2-(1*H*)-pyrimidinon zugegeben. Nach 15 Minuten werden 7.7 g (40.05 mmol) 4-Methylbenzoesäure-*tert*.-butylester, gelöst in 40 ml THF, zugetropft und weitere 15 Minuten nachgerührt. 3.25 ml (42.05 mmol) Chlorameisensäuremethylester werden separat in 20 ml THF gelöst, auf -70°C gekühlt und dann schnell zum Reaktionsansatz hinzugegeben. Nach weiteren 15 Minuten wird das Reaktionsgemisch mit Ammoniumchlorid-Lösung versetzt und auf Raumtemperatur erwärmt. Es wird mit Wasser verdünnt und die Phasen getrennt. Die wässrige Phase wird zweimal mit Diethylether extrahiert. Die vereinigten organischen Phasen werden mit gesättigter Natriumchlorid-Lösung gewaschen, über Natriumsulfat getrocknet und eingeengt. Der erhaltene Rückstand wird über Flash-Chromatographie an Kieselgel gereinigt (Laufmittel: Cyclohexan/ Essigsäureethylester 50:1). Es werden 4.2 g (16.78 mmol, 40% Ausbeute) der Titelverbindung als farbloses Öl erhalten.

¹H-NMR (300 MHz, CDCl₃, δ/ppm): 7.95 (2H, d), 7.32 (2H, d), 3.7 (3H, s), 3.68 (2H, s), 1.59 (9H, s).

### Beispiel 21A

### [4-(tert.-Butoxycarbonyl)phenyl]essigsäure

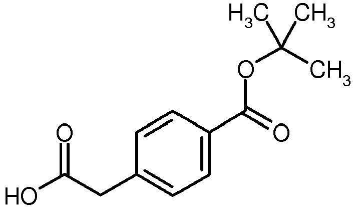

Eine Lösung von 4.2 g (16.78 mmol) 4-(2-Methoxy-2-oxoethyl)benzoesäure-*tert*.-butylester in 50 ml Wasser und 50 ml THF wird mit 0.8 g (33.6 mmol) Lithiumhydroxid versetzt und 1 Stunde bei Raumtemperatur gerührt. Die Phasen werden getrennt und die wässrige Phase mit 2 M Salzsäure auf pH 3 gestellt. Es wird mit Essigsäureethylester extrahiert. Die organische Phase wird anschließend mit Natriumchlorid-Lösung gewaschen, über Natriumsulfat getrocknet und eingeengt. Es werden 3.6 g (15.2 mmol, 87% Ausbeute) der Titelverbindung erhalten.

¹H-NMR (400 MHz, DMSO-d₆, δ/ppm): 12.45 (1H, breit), 7.85 (2H, d), 7.89 (2H, d), 3.66 (2H, s), 1.54 (9H, s).

### Beispiel 22A

### 4-(2-Hydroxyethyl)benzoesäure-tert.-butylester

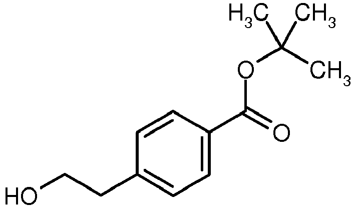

Zu einer Lösung von 3.6 g (15.24 mmol) [4-(*tert*.-Butoxycarbonyl)phenyl]essigsäure in 100 ml THF werden bei -10°C unter Sauerstoffausschluss 30.47 ml (30.47 mmol) 1 M Boran-THF-Komplex-Lösung langsam zugetropft. Der Ansatz wird 4 Stunden bei 0°C nachgerührt. Es wird anschließend mit Ammoniumchlorid-Lösung versetzt und mit Diethylether extrahiert. Die vereinigten organischen Phasen werden mit Natriumchlorid-Lösung gewaschen, über Natriumsulfat getrocknet und eingeengt. Es werden 3.2 g (14.4 mmol, 92% Ausbeute) der Titelverbindung erhalten.

¹H-NMR (400 MHz, DMSO-d₆, δ/ppm): 7.81 (2H, d), 7.34 (2H, d), 4.69 (1H, t), 3.62 (2H, q).

### Beispiel 23A

### 4-(2-Bromethyl)benzoesäure-tert.-butylester

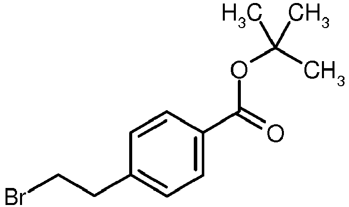

7.16 g (21.59 mmol) Tetrabromkohlenstoff und 5.66 g (21.59 mmol) Triphenylphosphin werden unter Sauerstoffausschluss in 70 ml THF gelöst und 30 Minuten bei Raumtemperatur gerührt. Anschließend werden 3.2 g (14.4 mmol) 4-(2-Hydroxyethyl)benzoesäure-*tert*.-butylester, in 30 ml THF gelöst, zugetropft und die Reaktionsmischung 12 Stunden bei Raumtemperatur gerührt. Der Ansatz wird nach vollständigem Umsatz zur Trockene eingeengt und der erhaltene Rückstand über Flash-Chromatographie an Kieselgel gereinigt (Laufmittel: Cyclohexan/Essigsäureethylester 10:1). Es werden 3.9 g (13.7 mmol, 87% Ausbeute) der Titelverbindung als farbloses Öl erhalten.

¹H-NMR (300 MHz, DMSO-d₆, δ/ppm): 7.85 (2H, d), 7.4 (2H, d), 3.76 (2H, t), 3.2 (2H, t), 1.55 (9H, s).

### Beispiel 24A

### Diallyl-{2-[4-(tert.-butoxycarbonyl)phenyl]ethyl}[4-(niethoxycarbonyl)benzyl]malonat

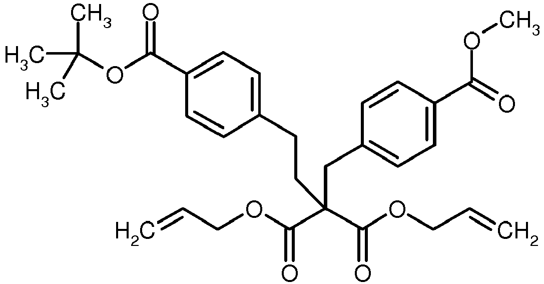

Eine Lösung von 4.13 g (12.43 mmol) 2-(4-Methoxycarbonylbenzyl)malonsäurediallylester in 40 ml DMF wird bei 0°C portionsweise mit 0.6 g (14.92 mmol) Natriumhydrid versetzt. Anschließend lässt man die Reaktionslösung auf Raumtemperatur kommen und rührt 1 Stunde lang nach. Danach wird die Reaktionslösung wieder auf 0°C abgekühlt, mit 3.9 g (13.68 mmol) 4-(2-Bromethyl)-benzoesäure-*tert*.-butylester in 10 ml DMF versetzt und 30 min bei dieser Temperatur nachgerührt. Der Ansatz wird dann bei Raumtemperatur über Nacht gerührt. Das Reaktionsgemisch wird auf Eiswasser gegeben, dreimal mit Essigsäureethylester extrahiert, die vereinigten organischen Phasen mit gesättigter Kochsalz-Lösung gewaschen und über Natriumsulfat getrocknet. Nach Filtration wird das Lösungsmittel im Vakuum bis zur Trockene eingeengt und das erhaltene Rohprodukt über präparative HPLC gereinigt. Es werden 933 mg (1.7 mmol, 14% Ausbeute) eines farblosen Feststoffes erhalten.

¹H-NMR (300 MHz, CDCl₃, δ/ppm): 7.91 (4H, dd), 7.2 (4H, dd), 5.95-5.8 (2H, m), 5.79 (4H, dd), 4.63 (4H, d), 3.9 (3H, s), 3.4 (2H, s), 2.7-2.62 (2H, m), 2.13-2.05 (2H, m), 1.58 (9H, s).

### Beispiel 25A

### 4-[4-(tert.-Butoxycarbonyl)phenyl]-2-[4-(methoxycarbonyl)benzyl]butansäure

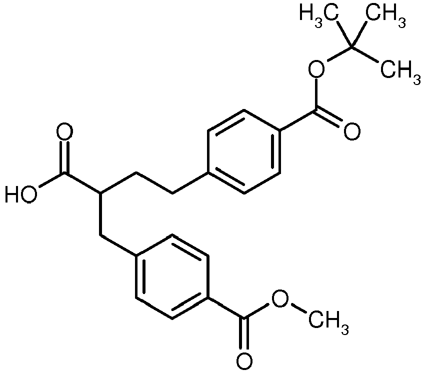

Eine Lösung von 920 mg (1.71 mmol) Diallyl-{2-[4-(*tert*.-butoxycarbonyl)phenyl]ethyl}[4-(methoxycarbonyl)benzyl]malonat, 31.5 mg (0.12 mmol) Triphenylphosphin und 7.7 mg (0.03 mmol) Palladiumacetat in 10 ml Dioxan wird bei Raumtemperatur mit einer Lösung von 0.79 ml (5.66 mmol) Trimethylamin und 0.16 ml (4.29 mmol) Ameisensäure in 10 ml Dioxan versetzt. Das Reaktionsgemisch wird anschließend 12 Stunden bei 100°C gerührt. Nach vollständigem Umsatz wird die Reaktionslösung abgekühlt und das Lösungsmittel im Vakuum entfernt. Der erhaltene Rückstand wird durch Flash-Chromatographie an Kieselgel (Laufmittel: Cyclohexan/Essigsäureethylester 4:1 → 1:1) gereinigt. Es werden 497 mg (1.2 mmol, 61% Ausbeute) eines farblosen Feststoffs erhalten.

¹H-NMR (400 MHz, DMSO-d₆, δ/ppm): 12.3 (1H, s), 7.86 (2H, d), 7.81 (2H, d), 7.32 (2H, d), 7.28 (2H, d), 3.82 (3H, s), 2.98-2.82 (2H, m), 2.74-2.56 (2H, m), 1.9-1.59 (3H, m), 1.53 (9H, s).

### Beispiel 26A

### tert.-Butyl-methyl-4,4'-[2-(hydroxymethyl)butan-1,4-diyl]dibenzoat

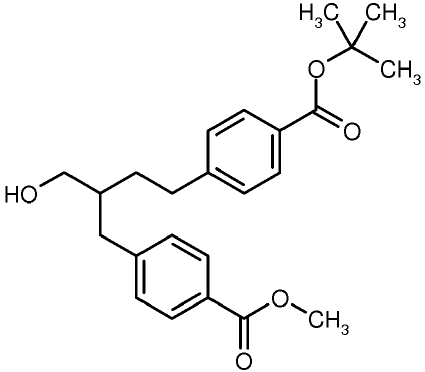

Zu einer Lösung von 497 mg (1.2 mmol) 4-[4-(*tert*.-Butoxycarbonyl)phenyl]-2-[4-(methoxycarbonyl)benzyl]butansäure in 10 ml THF werden 1.81 ml (1.81 mmol) einer 1 M Boran-THF-Komplex-Lösung bei -15°C zugetropft und die Reaktionsmischung bei dieser Temperatur noch 3 Stunden nachgerührt. Nach vollständiger Umsetzung wird das Reaktionsgemisch mit gesättigter Ammoniumchlorid-Lösung versetzt und mit Essigsäureethylester extrahiert. Die organische Phase wird mit Natriumchlorid-Lösung gewaschen, über Natriumsulfat getrocknet und eingeengt. Das erhaltene Rohprodukt wird durch Flash-Chromatographie an Kieselgel gereinigt (Laufmittel: Cyclohexan/Essigsäureethylester 4:1). Es werden 403 mg (1.0 mmol, 81% Ausbeute) eines farblosen Feststoffs erhalten.

¹H-NMR (300 MHz, DMSO-d₆, δ/ppm): 7.87 (2H, d), 7.78 (2H, d), 7.31 (2H, d), 7.25 (2H, d), 4.5 (1H, t), 3.84 (3H, s), 3.32 (2H, t), 2.8-2.57 (4H, m), 1.76-1.57 (3H, m), 1.53 (9H, s).

### Beispiel 27A

### tert.-Butyl-methyl-4,4'-(2-formylbutan-1,4-diyl)dibenzoat

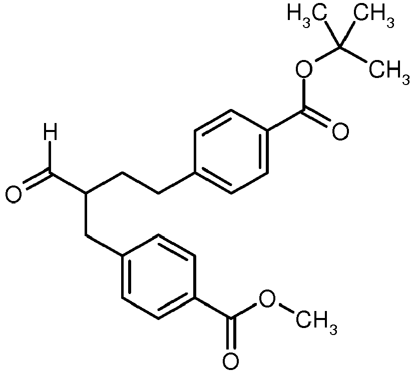

Eine Lösung von 400 mg (1 mmol) *tert*.-Butyl-methyl-4,4'-[2-(hydroxymethyl)butan-1,4-diyl]dibenzoat in 20 ml Dichlormethan wird mit 5 g 4Å-Molekularsieb und 176.4 mg (1.51 mmol) *N*-Methylmorpholin-*N*-oxid versetzt und 10 Minuten bei Raumtemperatur gerührt. Anschließend werden 17.6 mg (0.05 mmol) Tetrapropylammoniumperruthenat zugegeben und die Reaktionsmischung 5 Stunden bei Raumtemperatur gerührt. Das Molekularsieb wird über Kieselgur abfiltriert und das Filtrat eingeengt. Der erhaltene Rückstand wird über Flash-Chromatographie an Kieselgel gereinigt (Laufmittel: Cyclohexan/Essigsäureethylester 4:1). Es werden 200 mg (0.5 mmol, 50% Ausbeute) eines farblosen Feststoffs erhalten, welcher direkt weiter umgesetzt wird.

### Beispiel 28A

### 4-[(3E/Z)-2-{2-[4-(tert.-Butoxycarbonyl)phenyl]ethyl}-4-(2-{[4'-(trifluormethyl)biphenyl-4-yl]-methoxy}phenyl)but-3-en-1-yl]benzoesäuremethylester

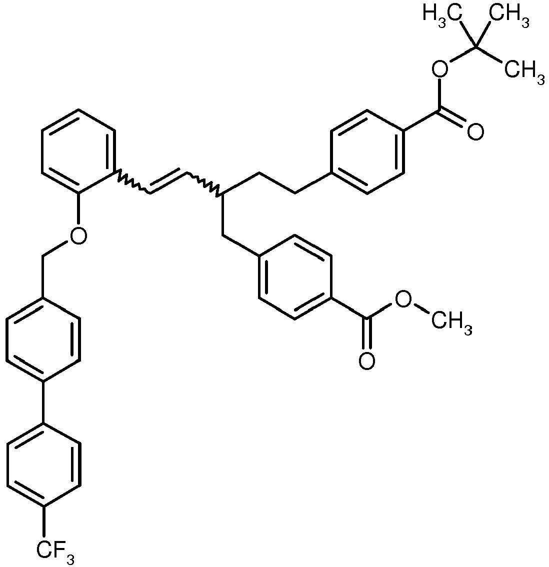

Zu einer Lösung von 412 mg (0.61 mmol) Triphenyl-(2-{[4'-(trifluormethyl)biphenyl-4-yl]methoxy}benzyl)phosphoniumbromid in 10 ml THF werden bei 0°C 0.47 ml (0.76 mmol) einer 1.6 M Lösung von n-Butyllithium in Hexan langsam hinzugefügt. Anschließend werden bei dieser Temperatur 200 mg (0.5 mmol) *tert*.-Butyl-methyl-4,4'-(2-formylbutan-1,4-diyl)dibenzoat zudosiert. Nach Erwärmung auf Raumtemperatur wird die Reaktionslösung 12 Stunden nachgerührt, dann mit etwas Wasser versetzt und bis zur Trockene eingeengt. Der Rückstand wird in Essigsäureethylester aufgenommen, mit Wasser und mit gesättigter Natriumchlorid-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/ Essigsäureethylester 50:1 → 20:1) gereinigt. Es werden 270 mg (0.37 mmol, 70% Ausbeute) eines weißen Schaums erhalten.

¹H-NMR (300 MHz, DMSO-d₆, δ/ppm): 7.88-7.6 (10H, m), 7.55-7.38 (2H, m), 7.32-7.15 (7H, m), 7.15-7.0 (1H, m), 6.92 (1H, t), 6.5 (1H, d), 6.12 (1H, dd), 5.15 (2H, s), 3.76 (3H, s), 2.92-2.56 (5H, m), 1.88-1.56 (2H, m), 1.5 (9H, s).

### Beispiel 29A

### 4-[(4E/Z)-3-[4-(Methoxycarbonyl)benzyl]-5-(2-{[4'-(trifluormethyl)biphenyl-4-yl]methoxy}-phenyl)pent-4-en-1-yl]benzoesäure

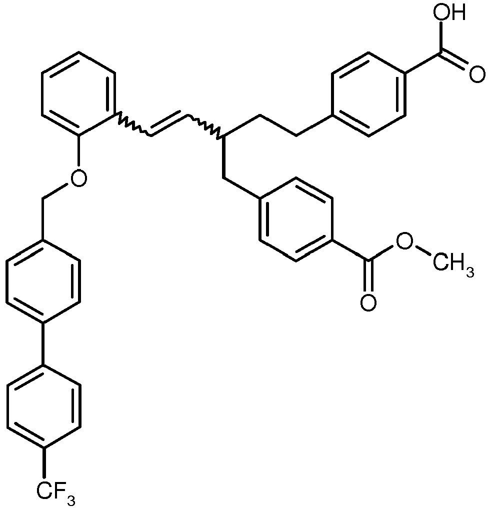

Eine Lösung von 100 mg (0.14 mmol) 4-[(3*E*/*Z*)-2-{2-[4-(*tert*.-Butoxycarbonyl)phenyl]ethyl}-4-(2-{[4'-(trifluormethyl)biphenyl-4-yl]methoxy}phenyl)but-3-en-1-yl]benzoesäuremethylester in 4 ml Dichlormethan wird mit 1 ml (13 mmol) Trifluoressigsäure versetzt und 1 Stunde bei Raumtemperatur gerührt. Der Ansatz wird eingeengt und der Rückstand zwischen Wasser und Essigsäureethylester verteilt. Die organische Phase wird über Natriumsulfat getrocknet und eingeengt. Der erhaltene Rückstand wird über Flash-Chromatographie an Kieselgel gereinigt (Laufmittel: Cyclohexan/Essigsäureethylester 4:1). Es werden 56 mg (0.08 mmol, 55% Ausbeute) der Titelverbindung erhalten.

LC-MS (Methode 2): Rₜ = 3.28 min; MS (ESIpos): m/z = 665 (M+H)⁺.

### Beispiel 30A

### 4-{(3E)-2-[2-(4-Cyanophenyl)ethyl]-4-[2-(4,4,4-trifluorbutoxy)phenyl]but-3-en-1-yl}benzoesäure-methylester

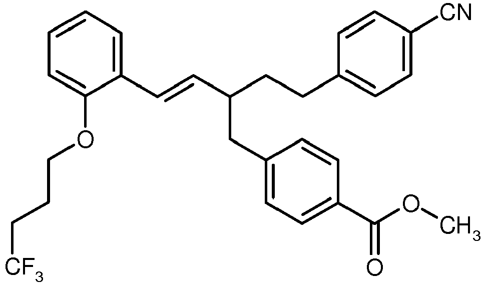

Eine Lösung von 200 mg (0.486 mmol) 4-[(3*E*)-2-[2-(4-Cyanophenyl)ethyl]-4-(2-hydroxyphenyl)-but-3-en-1-yl]benzoesäuremethylester in 5 ml Acetonitril wird mit 101 mg (0.73 mmol) Kaliumcarbonat und 139 mg (0.73 mmol) 4,4,4-Trifluor-1-brombutan versetzt und 12 h unter Rückfluss gerührt. Nach vollständigem Umsatz werden die Salze abfiltriert und die Mutterlauge eingeengt. Es werden 253 mg (0.49 mmol, 100% d. Th.) der Titelverbindung erhalten.

LC-MS (Methode 2): Rₜ = 3.21 min; MS (ESIpos): m/z = 522 (M+H)⁺.

### Beispiel 31A

### 4-[(3E)-2-[2-(4-Cyanophenyl)ethyl]-4-(2-methoxyphenyl)but-3-en-1-yl]benzoesäuremethylester

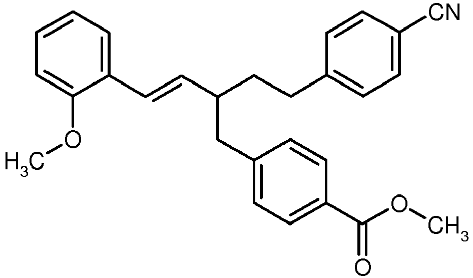

Ausgehend von 2 g (4.9 mmol) 4-[(3*E*)-2-[2-(4-Cyanophenyl)ethyl]-4-(2-hydroxyphenyl)but-3-en-1-yl]benzoesäuremethylester, 1 g (7.3 mmol) Kaliumcarbonat und 3.63 ml (58 mmol) Iodmethan werden nach dem in Beispiel 30A beschriebenen Verfahren 1.9 g (4.5 mmol, 92% d. Th.) der Titelverbindung erhalten.

MS (DCI): m/z = 443 (M+NH₄)⁺.

### Beispiel 32A

### 4-[(3E)-2-[2-(4-Cyanophenyl)ethyl]-4-(2-methoxyphenyl)but-3-en-1-yl]benzoesäure

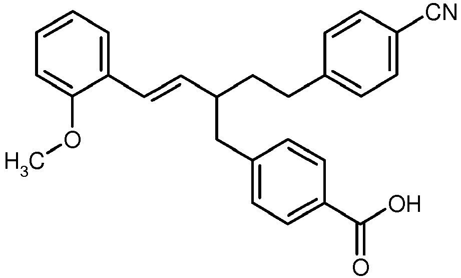

Eine Lösung von 744 mg (1.75 mmol) 4-[(3E)-2-[2-(4-Cyanophenyl)ethyl]-4-(2-methoxyphenyl)-but-3-en-1-yl]benzoesäuremethylester in 24 ml THF und 24 ml Wasser wird mit 84 mg (3.5 mmol) Lithiumhydroxid versetzt und 12 h bei 50°C gerührt. Nach vollständigem Umsatz wird die Reaktionslösung mit Diethylether und Wasser verdünnt und die Phasen getrennt. Die wässrige Phase wird mit 1 M Salzsäure sauer gestellt und mit Diethylether extrahiert. Die erhaltene organische Phase wird mit Natriumchlorid-Lösung gewaschen, über Natriumsulfat getrocknet und eingeengt. Es werden 606 mg (1.5 mmol, 97% d. Th.) der Titelverbindung erhalten.

LC-MS (Methode 2): Rt = 2.68 min; MS (ESIpos): m/z = 412 (M+H)⁺.

### Beispiel 33A

### 4-{(3E)-4-(2-Butoxyphenyl)-2-[2-(4-cyanophenyl)ethyl]but-3-en-1-yl}benzoesäuremethylester

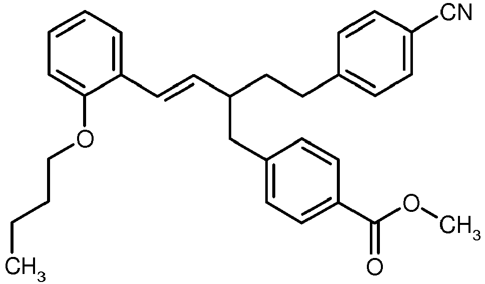

Ausgehend von 200 mg (0.49 mmol) 4-[(3*E*)-2-[2-(4-Cyanophenyl)ethyl]-4-(2-hydroxyphenyl)but-3-en-1-yl]benzoesäuremethylester, 101 mg (0.73 mmol) Kaliumcarbonat und 100 mg (0.73 mmol) 1-Brombutan werden nach dem in Beispiel 30A beschriebenen Verfahren 224 mg (0.48 mmol, 85% d. Th.) der Titelverbindung erhalten.

LC-MS (Methode 1): Rₜ = 3.53 min; MS (ESIpos): m/z = 468 (M+H)⁺.

### Beispiel 34A

### 4-{(3E)-2-[2-(4-Cyanophenyl)ethyl]-4-[2-(pentyloxy)phenyl]but-3-en-1-yl}benzoesäuremethyl-ester

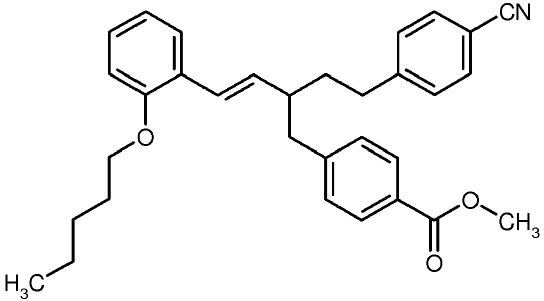

Ausgehend von 200 mg (0.49 mmol) 4-[(3*E*)-2-[2-(4-Cyanophenyl)ethyl]-4-(2-hydroxyphenyl)but-3-en-1-yl]benzoesäuremethylester, 101 mg (0.73 mmol) Kaliumcarbonat und 110 mg (0.73 mmol) 1-Pentylbromid werden nach dem in Beispiel 30A beschriebenen Verfahren 196 mg (0.41 mmol, 70% d. Th.) der Titelverbindung erhalten.

LC-MS (Methode 1): Rt = 3.58 min; MS (ESIpos): m/z = 482 (M+H)⁺.

### Beispiel 35A

### 4-{(3E)-2-[2-(4-Cyanophenyl)ethyl]-4-[2-(hexyloxy)phenyl]but-3-en-1-yl}benzoesäuremethylester

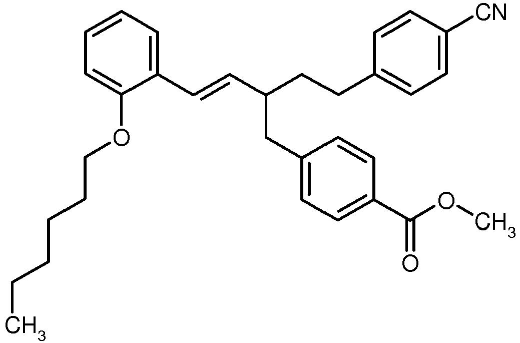

Ausgehend von 200 mg (0.49 mmol) 4-[(3*E*)-2-[2-(4-Cyanophenyl)ethyl]-4-(2-hydroxyphenyl)but-3-en-1-yl]benzoesäuremethylester, 101 mg (0.73 mmol) Kaliumcarbonat und 120 mg (0.73 mmol) 1-Hexylbromid werden nach dem in Beispiel 30A beschriebenen Verfahren 190 mg (0.37 mmol, 67% d. Th.) der Titelverbindung erhalten.

LC-MS (Methode 2): Rₜ = 3.65 min; MS (ESIpos): m/z = 496 (M+H)⁺.

### Beispiel 36A

### 4-{(3E)-2-[2-(4-Cyanophenyl)ethyl]-4-[2-(heptyloxy)phenyl]but-3-en-1-yl}benzoesäuremethyl-ester

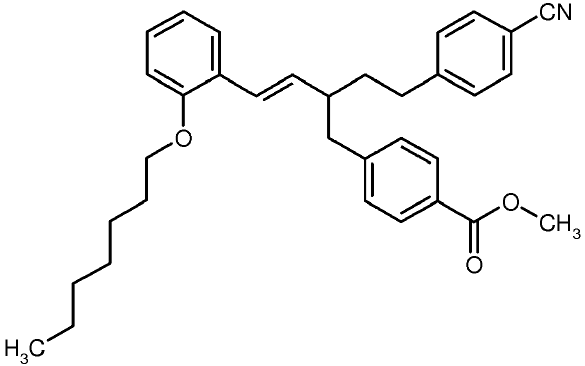

Ausgehend von 200 mg (0.49 mmol) 4-[(3*E*)-2-[2-(4-Cyanophenyl)ethyl]-4-(2-hydroxyphenyl)but-3-en-1-yl]benzoesäuremethylester, 101 mg (0.73 mmol) Kaliumcarbonat und 131 mg (0.73 mmol) 1-Heptylbromid werden nach dem in Beispiel 30A beschriebenen Verfahren 190 mg (0.37 mmol, 67% d. Th.) der Titelverbindung erhalten.

LC-MS (Methode 6): Rₜ = 8.0 min; MS (DCI): m/z = 527 (M+NH₄)⁺.

### Beispiel 37A

### 4-{(3E/Z)-2-[2-(4-Cyanophenyl)ethyl]-4-phenylbut-3-en-1-yl}benzoesäuremethylester

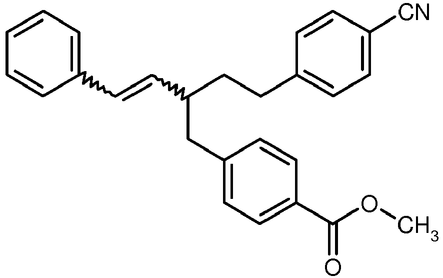

809 mg (1.87 mmol) Benzyltriphenylphosphin werden in 30 ml THF suspendiert und unter Sauerstoffausschluss auf 0°C abgekühlt. Bei dieser Temperatur werden 1.46 ml (2.33 mmol) *n*-Butyllithium (1.6 M Lösung in Hexan) zugetropft und die Mischung anschließend 45 Minuten nachgerührt. Danach werden 500 mg (1.56 mmol) 4-[4-(4-Cyanophenyl)-2-formylbutyl]benzoesäuremethylester, gelöst in 10 ml THF, schnell zu der Reaktionslösung getropft. Anschließend wird 1 h bei 0°C gerührt, dann langsam auf Raumtemperatur erwärmt und weitere 3 h nachgerührt. Die Reaktionslösung wird mit Ammoniumchlorid-Lösung versetzt und eingeengt. Der Rückstand wird in Essigsäureethylester aufgenommen und mit Wasser ausgeschüttelt. Die organische Phase wird mit Natriumchlorid-Lösung gewaschen, über Natriumsulfat getrocknet und eingeengt. Das erhaltene Rohprodukt wird mittels Flash-Chromatographie an Kieselgel gereinigt (Laufmittel: Cyclohexan/Essigsäureethylester 10:1). Es werden 484 mg (1.2 mmol, 79% d. Th.) der Titelverbindung erhalten, welche direkt ohne weitere Charakterisierung umgesetzt wird.

### Beispiel 38A

### Triphenyl-[2-(trifluormethoxy)benzyl]phosphoniumbromid

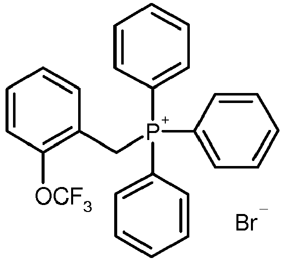

5 g (26 mmol) 2-Trifluormethoxybenzylbromid und 8.5 g (24.7 mmol) Triphenylphosphoniumbromid werden in 100 ml Acetonitril 3 h unter Rückfluss gerührt. Nach dem Abkühlen wird der entstandene Niederschlag abgesaugt und getrocknet. Es werden 13.5 g (26 mmol, 100% d. Th.) der Titelverbindung erhalten.

LC-MS (Methode 3): Rt = 1.91 min; MS (ESIpos): m/z = 437 (M-Br)⁺.

### Beispiel 39A

### 4-{(3E/Z)-2-[2-(4-Cyanophenyl)ethyl]-4-[2-(trifluormethoxy)phenyl]but-3-en-1-yl}benzoesäuremethylester

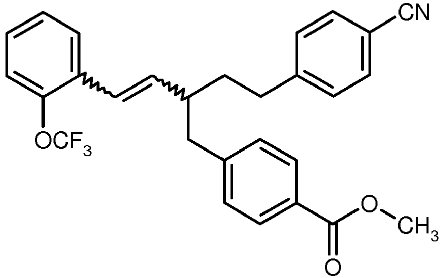

3.38 g (6.5 mmol) Triphenyl-[2-(trifluormethoxy)benzyl]phosphoniumbromid werden in 80 ml THF suspendiert und unter Sauerstoffausschluss auf 0°C abgekühlt. Bei dieser Temperatur werden 5.1 ml (8.2 mmol) *n*-Butyllithium (1.6 M Lösung in Hexan) zugetropft und anschließend die Mischung 45 Minuten nachgerührt. Danach werden 1.75 g (5.45 mmol) 4-[4-(4-Cyanophenyl)-2-formylbutyl]benzoesäuremethylester, gelöst in 20 ml THF, schnell zur Reaktionslösung getropft. Es wird 1 h bei 0°C gerührt, dann langsam auf Raumtemperatur erwärmt und weitere 3 h nachgerührt. Die Reaktionslösung wird mit Ammoniumchlorid-Lösung versetzt und eingeengt. Der Rückstand wird in Essigsäureethylester aufgenommen und mit Wasser ausgeschüttelt. Die organische Phase wird mit Natriumchlorid-Lösung gewaschen, über Natriumsulfat getrocknet und eingeengt. Das erhaltene Rohprodukt wird mittels Flash-Chromatographie an Kieselgel gereinigt (Laufmittel: Cyclohexan/Essigsäureethylester 10:1). Es werden 2.065 g (4.3 mmol, 79% d. Th.) der Titelverbindung erhalten.

LC-MS (Methode 3): Rt = 3.33 min; MS (ESIpos): m/z = 480 (M+H)⁺.

### Beispiel 40A

### 4-[(3E)-2-[2-(4-Cyanophenyl)ethyl]-4-{2-[(5,5,5-trifluorpentyl)oxy]phenyl}but-3-en-1-yl]benzoesäuremethylester

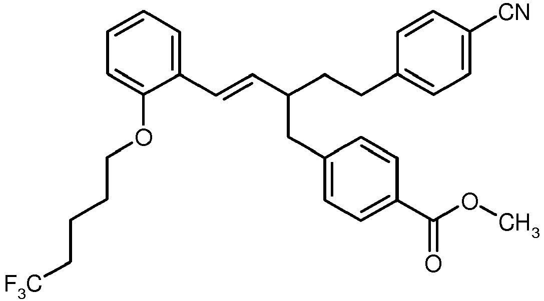

Eine Lösung von 200 mg (0.486 mmol) 4-[(3*E*)-2-[2-(4-Cyanophenyl)ethyl]-4-(2-hydroxyphenyl)-but-3-en-1-yl]benzoesäuremethylester in 10 ml Acetonitril wird mit 101 mg (0.73 mmol) Kaliumcarbonat sowie 149 mg (0.73 mmol) 1,1,1-Trifluor-5-brompentan [CAS Reg.-Nr. 54932-74-00] versetzt und 12 h unter Rückfluss gerührt. Nach vollständigem Umsatz werden die Salze abfiltriert und die Mutterlauge eingeengt. Es werden 205 mg (0.28 mmol, 79% d. Th.) der Titelverbindung erhalten.

LC-MS (Methode 2): Rₜ = 3.25 min; MS (ESIpos): m/z = 536 (M+H)⁺.

### Beispiel 41A

### 4-{(3E)-2-[2-(4-Cyanophenyl)ethyl]-4-[2-(3-phenylpropoxy)phenyl]but-3-en-1-yl}benzoesäuremethylester

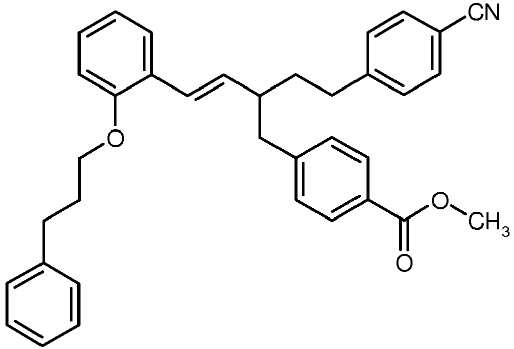

Eine Lösung von 200 mg (0.486 mmol) 4-[(3*E*)-2-[2-(4-Cyanophenyl)ethyl]-4-(2-hydroxyphenyl)-but-3-en-1-yl]benzoesäuremethylester in 5 ml Acetonitril wird mit 145 mg (0.73 mmol) (3-Brompropyl)benzol sowie 100.76 mg (0.73 mmol) Kaliumcarbonat versetzt und dann 12 h unter Rückfluss gerührt. Nach dem Abkühlen wird das Kaliumcarbonat abfiltriert und das Filtrat eingeengt. Es werden 278 mg (0.45 mmol, 93% d. Th., Gehalt 86%) der Titelverbindung erhalten.

MS (DCI): m/z = 547 (M+NH₄⁺)

HPLC (Methode 2): Rₜ = 5.53 min.

### Beispiel 42A

### 4-{(3E)-2-[2-(4-Cyanophenyl)ethyl]-4-[2-(4-phenylbutoxy)phenyl]but-3-en-1-yl} benzoesäuremethylester

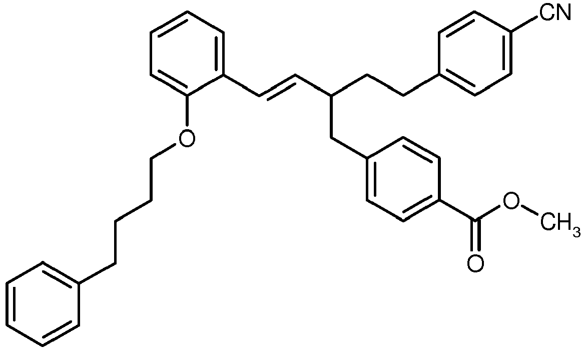

Eine Lösung von 200 mg (0.486 mmol) 4-[(3*E*)-2-[2-(4-Cyanophenyl)ethyl]-4-(2-hydroxyphenyl)-but-3-en-1-yl]benzoesäuremethylester in 5 ml Acetonitril wird mit 155 mg (0.73 mmol) (4-Brombutyl)benzol sowie 100.76 mg (0.73 mmol) Kaliumcarbonat versetzt und dann 12 h unter Rückfluss gerührt. Nach dem Abkühlen wird das Kaliumcarbonat abfiltriert und das Filtrat eingeengt. Es werden 308 mg (0.41 mmol, 85% d. Th., Gehalt 73%) der Titelverbindung erhalten.

LC-MS (Methode 2): Rt = 3.38 min; MS (ESIpos): m/z = 544 (M+H)⁺.

### Beispiel 43A

### 4-[(3E)-2-[2-(4-Cyanophenyl)ethyl]-4-(2-ethoxyphenyl)but-3-en-1-yl]benzoesäuremethylester

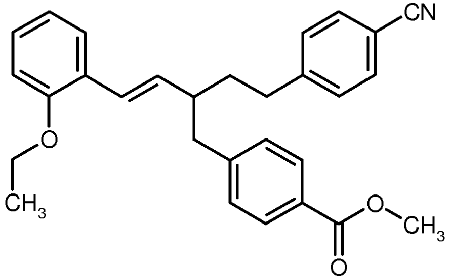

Ausgehend von 200 mg (0.486 mmol) 4-[(3*E*)-2-[2-(4-Cyanophenyl)ethyl]-4-(2-hydroxyphenyl)-but-3-en-1-yl]benzoesäuremethylester, 100.76 mg (0.73 mmol) Kaliumcarbonat und 79.44 mg (0.73 mmol) Bromethan werden nach dem in Beispiel 30A beschriebenen Verfahren 196 mg (0.44 mmol, 85% d. Th., Gehalt 93%) der Titelverbindung erhalten.

LC-MS (Methode 4): Rt = 3.35 min; MS (ESIpos): m/z = 440 (M+H)⁺.

### Beispiel 44A

### 4-[(3E)-2-[2-(4-Cyanophenyl)ethyl]-4-(2-propoxyphenyl)but-3-en-1-yl]benzoesäuremethylester

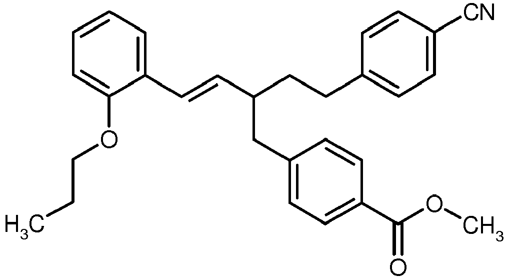

Ausgehend von 200 mg (0.486 mmol) 4-[(3*E*)-2-[2-(4-Cyanophenyl)ethyl]-4-(2-hydroxyphenyl)-but-3-en-1-yl]benzoesäuremethylester, 100.76 mg (0.73 mmol) Kaliumcarbonat und 89.67 mg (0.73 mmol) 1-Brompropan werden nach dem in Beispiel 30A beschriebenen Verfahren 201 mg (0.35 mmol, 72% d. Th., Gehalt 79%) der Titelverbindung erhalten.

LC-MS (Methode 4): Rₜ = 3.41 min; MS (ESIpos): m/z = 454 (M+H)⁺.

### Beispiel 45A

### 2-(4-Cyanobenzyl)-malonsäurediallylester

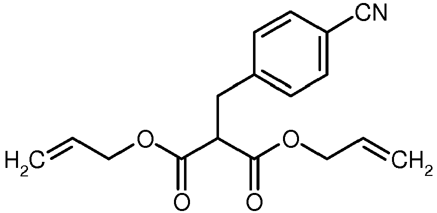

Eine Lösung von 121.5 g (0.659 mol) Malonsäurediallylester in 1.5 Liter Dioxan wird bei 0°C portionsweise mit 19.79 g (0.494 mol) Natriumhydrid versetzt (Vorsicht: Wasserstoffentwicklung). Nach Erwärmen auf Raumtemperatur wird der Ansatz 1 Stunde bei 40°C gerührt. Anschließend werden 50 g (0.329 mol) 4-Chlormethylbenzonitril, gelöst in 500 ml Dioxan, langsam bei 40°C zugetropft und die Reaktionslösung über Nacht bei 110°C gerührt. Nach Abkühlen auf Raumtemperatur wird das Reaktionsgemisch auf 1200 ml Wasser gegeben. Dabei ist darauf zu achten, dass der pH-Wert <7 bleibt (gegebenenfalls werden wenige ml 1 M Salzsäure bis ca. pH 2 zudosiert). Der Ansatz wird dann dreimal mit Essigsäureethylester extrahiert, die vereinigten organischen Phasen mit gesättigter Natriumchlorid-Lösung gewaschen und über Natriumsulfat getrocknet. Nach der Filtration wird das Lösungsmittel im Vakuum bis zur Trockene eingeengt. Anschließend wird durch Hochvakuum-Destillation überschüssiger Malonsäurediallylester entfernt (Siedepunkt: 57°C, 0.074 mbar). Der Destillationsrückstand wird durch Flash-Chromatographie an Kieselgel (Laufmittel: Petrolether/Essigsäureethylester 20:1) gereinigt. Es werden 67 g (0.22 mol, 67% d. Th.) eines farblosen Feststoffes erhalten.

¹H-NMR (300 MHz, DMSO-d₆, δ/ppm): 7.77 (2H, d), 7.48 (2H, d), 5.90-5.73 (2H, m), 5.29-5.13 (4H, m), 4.64-4.50 (4H, m), 4.09 (1H, t), 3.21 (2H, d).

MS (DCI): m/z = 317 (M+NH₄⁺).

### Beispiel 46A

### 2-(4-Cyanobenzyl)-2-[2-(4-methoxycarbonylphenyl)ethyl]-malonsäurediallylester

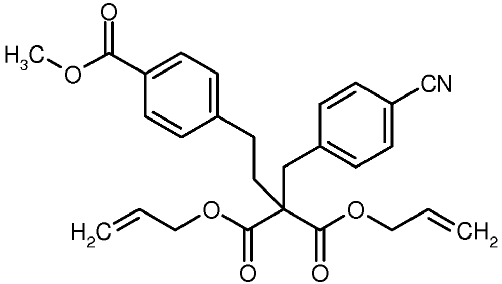

Eine Lösung von 48.53 g (162.14 mmol) 2-(4-Cyanobenzyl)-malonsäurediallylester aus Beispiel 45A in 180 ml DMF wird bei 0°C portionsweise mit 7.13 g (178.36 mmol) Natriumhydrid versetzt. Anschließend lässt man die Reaktionslösung auf Raumtemperatur kommen und rührt 30 min nach. Dann wird die Reaktionslösung wieder auf 0°C abgekühlt, mit 55 g (194.57 mmol) 4-(2-Bromethyl)-benzoesäuremethylester [CAS Reg.-Nr. 136333-97-6] in 195 ml DMF versetzt und 30 min bei dieser Temperatur nachgerührt. Der Ansatz wird danach bei Raumtemperatur über Nacht gerührt. Das Reaktionsgemisch wird tropfenweise mit Wasser versetzt, dreimal mit Essigsäureethylester extrahiert, die vereinigten organischen Phasen mit gesättigter Kochsalz-Lösung gewaschen und über Natriumsulfat getrocknet. Nach der Filtration wird das Lösungsmittel im Vakuum bis zur Trockene eingeengt. Das erhaltene Rohprodukt wird durch Flash-Chromatographie an Kieselgel (Laufmittel: Petrolether/Essigsäureethylester 10:1) gereinigt. Es werden 33.4 g (72.37 mmol, 44% d. Th.) eines farblosen Feststoffes erhalten.

¹H-NMR (300 MHz, CDCl₃, δ/ppm): 7.89 (2H, d), 7.79 (2H, d), 7.38 (2H, d), 7.32 (2H, d), 5.97-5.81 (2H, m), 5.38-5.20 (4H, m), 4.61 (4H, d), 3.82 (3H, s), 3.39 (2H, s), 2.77-2.61 (2H, m), 1.99-1.84 (2H, m).

MS (DCI): m/z = 479 (M+NH₄⁺).

### Beispiel 47A

### 4-[3-Carboxy-4-(4-cyanophenyl)butyl]-benzoesäuremethylester

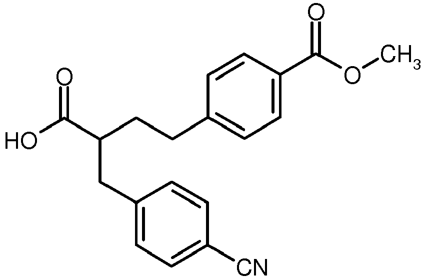

Eine Lösung von 7.5 g (16.25 mmol) 2-(4-Cyanobenzyl)-2-[2-(4-methoxycarbonylphenyl)ethyl]-malonsäurediallylester aus Beispiel 46A, 0.3 g (1.14 mmol) Triphenylphosphin und 70 mg Palladiumacetat in 170 ml Dioxan wird bei Raumtemperatur mit einer Lösung von 7.47 ml (53.63 mmol) Trimethylamin und 1.53 ml (40.63 mmol) Ameisensäure in 170 ml Dioxan versetzt. Das Reaktionsgemisch wird anschließend 2 h bei 100°C gerührt. Nach vollständigem Umsatz wird die Reaktionslösung abgekühlt und das Lösungsmittel im Vakuum entfernt. Der Rückstand wird in Essigsäureethylester und Wasser aufgenommen, mit 1 N Salzsäure angesäuert und die organische Phase abgetrennt. Die wässrige Phase wird noch dreimal mit Essigsäureethylester extrahiert, die organischen Phasen werden anschließend vereinigt, mit gesättigter Kochsalz-Lösung gewaschen und über Natriumsulfat getrocknet. Nach Filtration wird die Lösung im Vakuum eingeengt. Es werden 5.48 g (89% d. Th., 90% Reinheit) eines farblosen Feststoffes erhalten.

¹H-NMR (400 MHz, DMSO-d₆, δ/ppm): 12.46-12.29 (1H, breit), 7.88 (2H, d), 7.74 (2H, d), 7.39 (2H, d), 7.31 (2H, d), 3.83 (3H, s), 2.99-2.83 (2H, m), 2.79-2.56 (3H, m), 1.93-1.67 (2H, m).

MS (DCI): m/z = 355 (M+NH₄⁺).

### Beispiel 48A

### 4-[3-(4-Cyanobenzyl)-4-hydroxybutyl]-benzoesäuremethylester

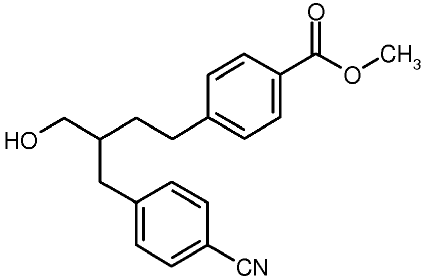

Zu einer Lösung von 8 g (23.71 mmol) 4-[3-Carboxy-4-(4-cyanophenyl)butyl]-benzoesäuremethylester aus Beispiel 47A in 200 ml THF werden 47.43 ml (47.43 mmol) einer 1 M Boran-THF-Komplex-Lösung bei -10°C zugetropft. Nach Erwärmen auf -5°C wird noch 4 h bei dieser Temperatur nachgerührt. Nach vollständiger Umsetzung wird das Reaktionsgemisch mit gesättigter Natriumhydrogencarbonat-Lösung versetzt und das Lösungsmittel bis zur Trockene eingeengt. Der Rückstand wird in Dichlormethan aufgenommen, über Natriumsulfat getrocknet und nach Filtration wiederum vom Lösungsmittel befreit. Das erhaltene Rohprodukt wird durch Flash-Chromatographie an Kieselgel (Laufmittel: Essigsäureethylester/Cyclohexan 1:10) gereinigt. Es werden 5.8 g (98% Reinheit, 74% d. Th.) eines farblosen Feststoffes erhalten.

¹H-NMR (300 MHz, DMSO-d₆, δ/ppm): 7.86 (2H, d), 7.73 (2H, d), 7.38 (2H, d), 7.30 (2H, d), 4.60 (1H, t), 3.83 (3H, s), 3.32 (2H, t), 2.81-2.57 (4H, m), 1.79-1.56 (2H, m), 1.54-1.39 (1H, m).

MS (DCI): m/z = 341 (M+NH₄⁺).

### Beispiel 49A

### 4-[3-(4-Cyanobenzyl)-4-oxobutyl]-benzoesäuremethylester

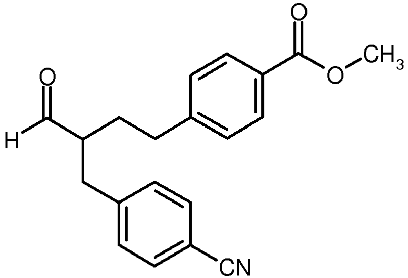

Eine Lösung von 400 mg (1.24 mmol) 4-[3-(4-Cyanobenzyl)-4-hydroxybutyl]-benzoesäuremethylester aus Beispiel 48A in 7 ml Dichlormethan wird mit 320 mg (1.48 mmol) Pyridiniumchlorochromat (PCC) versetzt und 5 Stunden bei Raumtemperatur gerührt. Nach vollständigem Umsatz wird ca. 1 g Kieselgel zugegeben und das Lösungsmittel im Vakuum bis zur Trockene entfernt. Der Rückstand wird durch Flash-Chromatographie an Kieselgel (Laufmittel: Cyclohexan/Essigsäureethylester 10:1 → 4:1) gereinigt. Es werden 302 mg (90% Reinheit, 69% d. Th.) eines farblosen Feststoffes erhalten.

¹H-NMR (300 MHz, DMSO-d₆, δ/ppm): 9.68 (1H, s), 7.87 (2H, d), 7.77 (2H, d), 7.43 (2H, d), 7.31 (2H, d), 3.86 (3H, s), 3.16-3.03 (1H, m), 2.94-2.81 (1H, m), 2.80-2.55 (3H, m), 1.99-1.81 (1H, m), 1.78-1.61 (1H, m).

MS (DCI): m/z = 339 (M+NH₄⁺).

### Beispiel 50A

### E-4-[3-(4-Cyanobenzyl)-5-(2-hydroxyphenyl)-pent-4-enyl]-benzoesäuremethylester

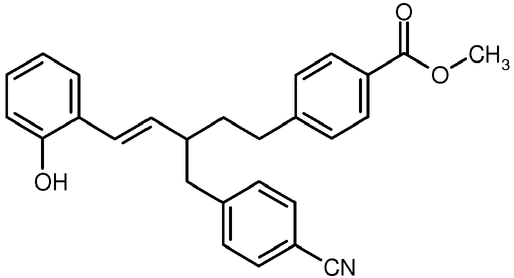

Zu einer Lösung von 23.22 g (51.7 mmol) (2-Hydroxybenzyl)-triphenylphosphoniumbromid in 130 ml wasserfreiem THF werden bei 0°C 73.8 ml (118 mmol) einer 1.6 M Lösung von n-Butyllithium in Hexan langsam hinzugetropft. Anschließend werden bei dieser Temperatur 15.5 g (42 mmol, Gehalt 87%) 4-[3-(4-Cyanobenzyl)-4-oxobutyl]-benzoesäuremethylester aus Beispiel 49A, gelöst in 130 ml THF, langsam zudosiert. Nach Erwärmung auf Raumtemperatur wird die Reaktionslösung 12 h nachgerührt, anschließend mit etwas Wasser versetzt und bis zur Trockene eingeengt. Der Rückstand wird in Essigsäureethylester aufgenommen, mit Wasser und gesättigter Kochsalz-Lösung gewaschen und über Natriumsulfat getrocknet. Nach Filtration wird das Lösungsmittel bis zur Trockene eingeengt. Das erhaltene Rohprodukt wird durch Flash-Chromatographie an Kieselgel (Laufmittel: Cyclohexan/Essigsäureethylester 4:1 → 2:1) gereinigt. Es werden 11.3 g (27.5 mmol, 65% d. Th.) eines farblosen Feststoffes erhalten.

¹H-NMR (300 MHz, DMSO-d₆, δ/ppm): 9.47 (1H, s), 7.88 (2H, d), 7.71 (2H, d), 7.42-7.27 (5H, m), 7.01 (1H, t), 6.81-6.68 (2H, m), 6.45 (1H, d), 6.12-6.00 (1H, m), 3.84 (3H, s), 3.42 (2H, m), 2.95-2.56 (3H, m), 1.88-1.56 (2H, m).

MS (DCI): m/z = 429 (M+NH₄⁺).

11.3 g (27.5 mmol) des so erhaltenen racemischen E-4-[3-(4-Cyanobenzyl)-5-(2-hydroxyphenyl)-pent-4-enyl]-benzoesäuremethylesters werden mittels präparativer HPLC an chiraler Phase weiter aufgetrennt. Es werden jeweils enantiomerenrein 4.14 g bzw. 3.69 g der beiden *E*-Isomere als farblose Feststoffe erhalten (siehe Beispiele 51A und 52A).

### Beispiel 51A

### E-4-[3-(4-Cyanobenzyl)-5-(2-hydroxyphenyl)-pent-4-enyl]-benzoesäuremethylester

### (Enantiomer 1)

### Methode Enantiomerentrennung:

Säule: Daicel Chiralpak AD-H 250 mm x 4.6 mm, 5 µm; Elutionsmittel: Isohexan / Isopropanol 50:50 (v/v); Fluss: 1 ml/min; UV-Detektion: 210 nm; Temperatur: 25°C.

Rₜ 6.77 min; Reinheit 96.85%; >99.5% ee
Ausbeute: 4.14 g
LC-MS (Methode 7): Rt = 3.03 min; MS (ESIpos): m/z = 412 (M+H)⁺.

### Beispiel 52A

### E-4-[3-(4-Cyanobenzyl)-5-(2-hydroxyphenyl)-pent-4-enyl]-benzoesäuremethylester

### (Enantiomer 2)

Methode Enantiomerentrennung: siehe Beispiel 51A.

Rₜ 7.82 min; Reinheit 96%; >99% ee
Ausbeute: 3.69 g
LC-MS (Methode 7): Rt = 3.03 min; MS (ESIpos): m/z = 412 (M+H)⁺.

### Beispiel 53A

### 1-(5-Brompentyl)-4-(trifluormethyl)benzol

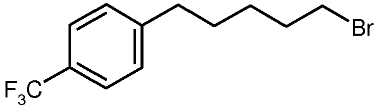

Eine Lösung von 11.25 g (50 mmol) 1-Brom-4-(trifluormethyl)benzol in 50 ml THF wird bei Raumtemperatur mit 1.34 g (55 mmol) aktivierten Magnesiumspänen versetzt. Die Reaktionslösung erwärmt sich zum Rückfluss (exotherme Reaktion), dabei gehen die Magnesiumspäne fast vollständig in Lösung. Nach Abklingen der exothermen Reaktion wird die dunkelbraune Lösung weitere 15 min unter Rückfluss gerührt. Anschließend wird die so erhaltene, ca. 1 M Grignard-Lösung wieder auf Raumtemperatur abgekühlt.

Parallel dazu wird eine hellgrüne Lösung von 359 mg (2.5 mmol) Kupfer(I)bromid und 435 mg (5 mmol) wasserfreiem Lithiumbromid in 10 ml THF bei Raumtemperatur mit einer Lösung von 20.5 ml (150 mmol) 1,5-Dibrompentan in 40 ml THF versetzt. Das Reaktionsgemisch wird auf 40°C Badtemperatur erwärmt und dann langsam mit der frisch hergestellten Grignard-Lösung versetzt. Nach vollständiger Zugabe der Grignard-Lösung wird das Reaktionsgemisch 1.5 h bei 50-55°C gerührt und danach über Nacht bei Raumtemperatur nachgerührt. Das Reaktionsgemisch wird tropfenweise mit Ammoniumchlorid-Lösung (20 g Ammoniumchlorid in 100 ml Wasser) versetzt und unter Eiskühlung hydrolysiert. Die Mischung wird dreimal mit Diethylether extrahiert, die vereinigten organischen Phasen mit gesättigter Kochsalz-Lösung gewaschen und über Natriumsulfat getrocknet. Nach der Filtration wird das Lösungsmittel im Vakuum bis zur Trockene eingeengt. Das erhaltene Rohprodukt wird durch fraktionierte Destillation gereinigt. Es werden 7.11 g (24.1 mmol, 48% d. Th.; Siedepunkt: 80°C, 0.15 Torr) eines farblosen Öls erhalten.

¹H-NMR (200 MHz, DMSO-d₆, δ/ppm): 7.62 (2H, d), 7.43 (2H, d), 3.53 (2H, t), 2.68 (2H, t), 1.92-1.72 (2H, m), 1.71-1.51 (2H, m), 1.49-1.29 (2H, m).

MS (EI): m/z = 294 (M⁺).

### Beispiel 54A

### 1-(Difluormethyl)-4-(trifluormethoxy)benzol

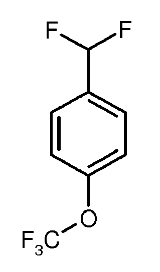

Eine Lösung von 2.527 g (13.29 mmol) Trifluormethoxybenzaldehyd in 6 ml Dichlormethan wird bei Raumtemperatur unter Argon langsam mit 5 g (22.6 mmol) 2-Methoxy-*N*-(2-methoxyethyl)-*N-*(trifluor-λ⁴-sulfanyl)ethanamin, gelöst in 6 ml Dichlormethan, versetzt. Anschließend werden 155 µl Ethanol zur Reaktionslösung dosiert, wobei die Temperatur leicht auf 29°C ansteigt. Der Ansatz wird dann bei Raumtemperatur über Nacht nachgerührt. Nach vollständigem Umsatz (Reaktionskontrolle mittels GC-MS) wird die Reaktionslösung langsam mit gesättigter Natriumhydrogencarbonat-Lösung hydrolysiert (starke Gasentwicklung) und in Wasser / Dichlormethan aufgenommen. Die organische Phase wird abgetrennt, über Magnesiumsulfat getrocknet und nach Filtration im Vakuum eingeengt. Es werden 2.38 g (11.22 mmol, 84.4% d. Th.) einer farblosen Flüssigkeit erhalten.

GC-MS (Methode 1): Rt = 2.46 min; MS (ESIpos): m/z = 212 (M⁺).

### Beispiel 55A

### 1-[Brom(difluor)methyl]-4-(trifluormethoxy)benzol

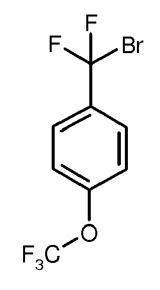

Unter Sauerstoffausschluss wird eine Lösung von 2.38 g (11.22 mmol) 1-(Difluormethyl)-4-(trifluormethoxy)benzol und 6.19 g (34.78 mmol) *N*-Bromsuccinimid (NBS) in 25 ml Tetrachlorkohlenstoff mit einer Tageslichtlampe bestrahlt. Dabei erreicht das Lösungsmittel seine Siedetemperatur. Es wird 48 Stunden lang unter Rückfluss bestrahlt. Anschließend lässt man auf Raumtemperatur abkühlen, filtriert ausgefallenes Succinimid ab und engt das Filtrat zur Trockene ein. Das erhaltene Rohprodukt wird durch Flash-Chromatographie an Kieselgel (Laufmittel: Cyclohexan) gereinigt. Es werden 1.73 g (5.94 mmol, 53% d. Th.) eines gelblichen Öls erhalten.

GC-MS (Methode 1): Rt = 2.93 min; MS (ESIpos): m/z = 211 (M-Br)⁺.

### Beispiel 56A

### 4-{(4E)-3-(4-Cyanobenzyl)-5-[2-({4-[1,2,2,2-tetrafluor-1-(trifluormethyl)ethyl]benzyl}oxy)-phenyl]pent-4-en-1-yl}benzoesäuremethylester

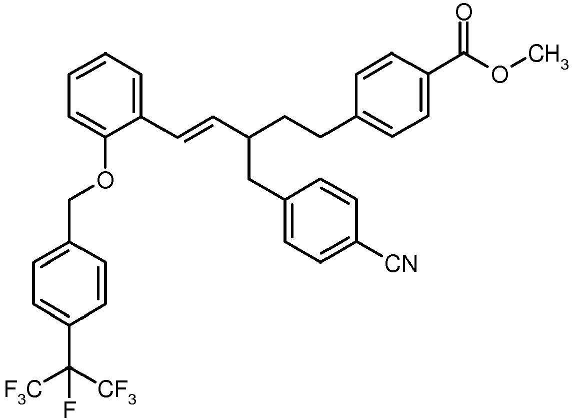

Eine Lösung von 150 mg (0.365 mmol) *E*-4-[3-(4-Cyanobenzyl)-5-(2-hydroxyphenyl)-pent-4-enyl]-benzoesäuremethylester (Enantiomer 1, Beispiel 51A) in 3 ml trockenem Acetonitril wird mit 139 mg (0.47 mmol) 1-(Chlormethyl)-4-[1,2,2,2-tetrafluor-1-(trifluormethyl)ethyl]benzol [hergestellt nach EP 0 009 787-A2] sowie 100 mg (0.73 mmol) wasserfreiem Kaliumcarbonat versetzt und dann 12 h unter Rückfluss erhitzt. Anschließend wird der Ansatz filtriert und das Filtrat bis zur Trockene eingeengt. Der Rückstand wird chromatographisch an Kieselgel (Laufmittel: Cyclohexan/Essigsäureethylester 4:1) aufgereinigt. Es werden 210 mg (0.31 mmol, 86% d. Th.) eines Öls isoliert.

¹H-NMR (400 MHz, DMSO-d₆, δ/ppm): 7.83 (2H, d), 7.70-7.60 (6H, m), 7.42 (1H, d), 7.36 (3H, d), 7.30 (2H, d), 7.20 (1H, t), 7.04 (1H, d), 6.93 (1H, t), 6.49 (1H, d), 6.17-6.06 (1H, m), 5.20 (2H, s), 3.82 (3H, s), 2.95-2.85 (1H, m), 2.80-2.69 (2H, m), 2.68-2.58 (1H, m), 2.57-2.46 (1H, m), 1.88-1.74 (1H, m), 1.73-1.60 (1H, m).

LC-MS (Methode 2): Rₜ = 3.41 min; m/z = 670 (M+H)⁺.

### Beispiel 57A

### 4-Fluor-2-(hydroxymethyl)phenol

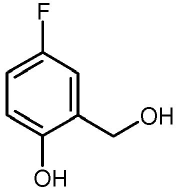

Unter Sauerstoffausschluss werden 27.1 g (159.28 mmol) 5-Fluor-2-hydroxybenzoesäuremethylester in 500 ml trockenem THF vorgelegt und auf 0°C abgekühlt. Anschließend tropft man langsam unter Kühlung 238 ml (238 mmol) einer 1 M Lösung von Lithiumaluminiumhydrid in THF hinzu und rührt 1 Stunde bei 0°C und dann über Nacht bei RT nach. Nach vollständiger Umsetzung wird der Ansatz mit gesättigter Ammoniumchlorid-Lösung versetzt und in Dichlormethan aufgenommen. Die organische Phase wird abgetrennt und über Natriumsulfat getrocknet. Nach Filtration wird das Lösungsmittel im Vakuum entfernt. Das Rohprodukt wird über Kieselgel chromatographisch (Laufmittel: Cyclohexan/Essigsäureethylester 20:1) gereinigt. Es werden 18.0 g (126.6 mmol, 79% d. Th.) eines farblosen Feststoffes isoliert.

¹H-NMR (400 MHz, DMSO-d₆, δ/ppm): 9.32 (1H, s), 7.06-7.03 (1H, m), 6.86-6.81 (1H, m), 6.74-6.71 (1H, m), 5.09 (1H, t), 4.45 (2H, d).

### Beispiel 58A

### (5-Fluor-2-hydroxybenzyl)(triphenyl)phosphoniumbromid

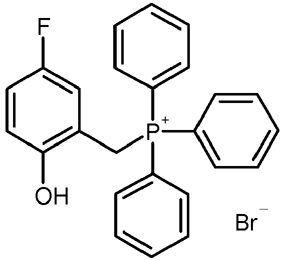

18.6 g (130.87 mmol) 4-Fluor-2-(hydroxymethyl)phenol und 42.67 g (124.32 mmol) Triphenylphosphoniumhydrobromid werden in 186 ml Acetonitril 3 h lang unter Rückfluss gerührt. Nach dem Abkühlen wird der entstandene Niederschlag abgesaugt und getrocknet. Es werden 58 g (124 mmol, 100% d. Th.) der Titelverbindung erhalten.

¹H-NMR (400 MHz, DMSO-d₆, δ/ppm): 9.82 (1H, s), 7.95-7.84 (3H, m), 7.79-7.62 (12H, m), 7.02-6.91 (1H, m), 6.75-6.67 (1H, m), 6.66-6.58 (1H, m), 4.90 (2H, d).

### Beispiel 59A

### 2-[4-(Methoxycarbonyl)benzyl]-2-{2-[4-(methoxycarbonyl)phenyl]ethyl}-malonsäurediallylester

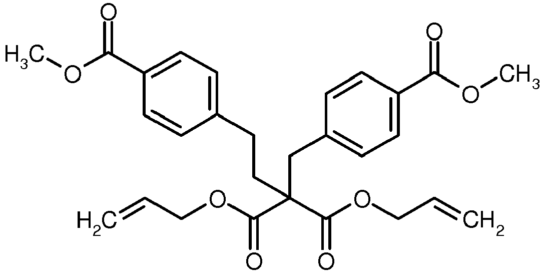

Eine Lösung von 42.61 g (128 mmol) 2-(4-Methoxycarbonylbenzyl)-malonsäurediallylester in 450 ml DMF wird bei Raumtemperatur mit 62.66 g (192 mmol) Caesiumcarbonat sowie 46.75 g (154 mmol) 4-(2-Bromethyl)-benzoesäuremethylester [CAS Reg.-Nr. 136333-97-6] versetzt. Der Ansatz wird danach bei Raumtemperatur über Nacht gerührt. Nach vollständigem Umsatz wird die Reaktionslösung bis zur Trockene eingeengt, der Rückstand in Wasser aufgenommen und dreimal mit Essigsäureethylester extrahiert. Die vereinigten organischen Phasen werden anschließend mit gesättigter Kochsalz-Lösung gewaschen und über Natriumsulfat getrocknet. Nach Filtration wird das Lösungsmittel im Vakuum bis zur Trockene eingeengt. Das erhaltene Rohprodukt wird durch Flash-Chromatographie an Kieselgel (Laufmittel: Cyclohexan/Essigsäureethylester 13:1) gereinigt. Es werden 41.35 g (83.6 mmol, 65% d. Th.) eines farblosen Feststoffes erhalten.

LC-MS (Methode 2): Rₜ = 2.92 min; m/z = 495 (M+H⁺).

### Beispiel 60A

### 2-[4-(Methoxycarbonyl)benzyl]-4-[4-(methoxycarbonyl)phenyl]-butansäure

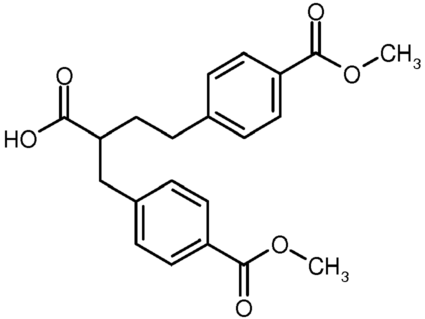

Eine Lösung von 40 g (80.9 mmol) 2-[4-(Methoxycarbonyl)benzyl]-2-{2-[4-(methoxycarbonyl)-phenyl]ethyl}-malonsäurediallylester, 1.49 g (5.67 mmol) Triphenylphosphin und 363 mg Palladiumacetat in 500 ml Dioxan wird bei Raumtemperatur mit einer Lösung von 37.2 ml (267 mmol) Triethylamin und 7.6 ml (202 mmol) Ameisensäure in 100 ml Dioxan versetzt. Das Reaktionsgemisch wird anschließend über Nacht bei 110°C gerührt. Nach vollständigem Umsatz wird die Reaktionslösung abgekühlt und das Lösungsmittel im Vakuum entfernt. Der erhaltene Rückstand wird durch Flash-Chromatographie an Kieselgel (Laufmittel: Cyclohexan/Essigsäureethylester 4:1) gereinigt. Es werden 20.98 g (56.6 mmol, 70% d. Th.) eines farblosen Feststoffes erhalten.

¹H-NMR (400 MHz, DMSO-d₆, δ/ppm): 12.32 (1H, s), 7.91-7.82 (4H, m), 7.37-7.27 (4H, m), 3.83 (6H, s), 2.99-2.81 (2H, m), 2.77-2.56 (3H, m), 1.92-1.67 (2H, m).

LC-MS (Methode 7): Rₜ = 2.45 min; m/z = 371 (M+H⁺).

### Beispiel 61A

### Dimethyl 4,4'-[2-(hydroxymethyl)butan-1,4-diyl]dibenzoat

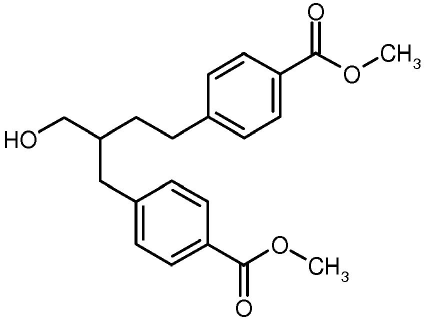

Zu einer Lösung von 20.9 g (56.4 mmol) 2-[4-(Methoxycarbonyl)benzyl]-4-[4-(methoxycarbonyl)-phenyl]butansäure in 600 ml THF werden 112.8 ml (112.8 mmol) einer 1 M Boran-THF-Komplex-Lösung bei -10°C zugetropft. Anschließend wird das Reaktionsgemisch auf 0°C erwärmt und 4 h bei dieser Temperatur nachgerührt. Nach vollständiger Umsetzung wird das Reaktionsgemisch mit gesättigter Ammoniumchlorid-Lösung versetzt, mit Wasser und Essigsäureethylester verdünnt, die Phasen getrennt und die wässrige Phase zweimal mit Essigsäureethylester nachextrahiert. Die vereinigten organischen Phasen werden über Natriumsulfat getrocknet und nach Filtration im Vakuum vom Lösungsmittel befreit. Es werden 20.11 g (94% Reinheit, 100% d. Th.) eines farblosen Feststoffes erhalten.

LC-MS (Methode 2): Rₜ = 2.27 min; m/z = 357 (M+H⁺).

### Beispiel 62A

### Dimethyl 4,4'-(2-formylbutan-1,4-diyl)dibenzoat

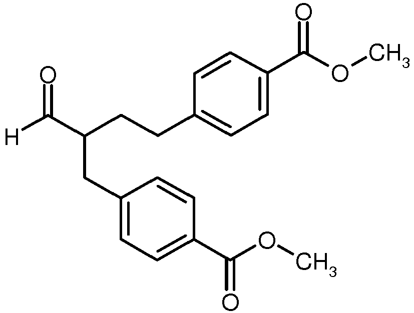

Eine Lösung von 18.02 g (50.5 mmol) Dimethyl 4,4'-[2-(hydroxymethyl)butan-1,4-diyl]dibenzoat in 350 ml Dichlormethan wird mit 13.07 g (60.6 mmol) Pyridiniumchlorochromat (PCC) versetzt und 12 Stunden bei Raumtemperatur gerührt. Nach vollständigem Umsatz werden ca. 60 g Kieselgel zugesetzt und das Lösungsmittel im Vakuum bis zur Trockene entfernt. Der Rückstand wird durch Flash-Chromatographie an Kieselgel (Laufmittel: Cyclohexan/Essigsäureethylester 5:1 → 4:1) gereinigt. Es werden 14.73 g (41.46 mmol, 82% d. Th.) eines farblosen Öls erhalten.

LC-MS (Methode 7): Rₜ = 2.61 min; m/z = 355 (M+H⁺).

### Beispiel 63A

### 4-{(4E)-5-(5-Fluor-2-hydroxyphenyl)-3-[4-(methoxycarbonyl)benzyl]pent-4-en-1-yl}benzoesäuremethylester

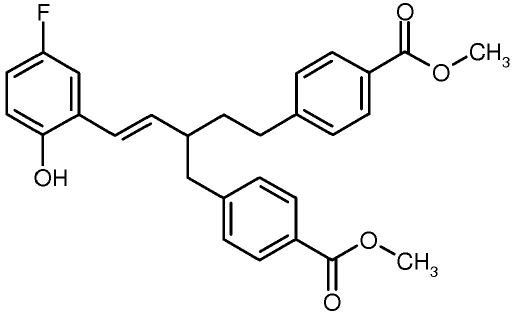

Zu einer Lösung von 10.84 g (23.2 mmol) (5-Fluor-2-hydroxybenzyl)(triphenyl)phosphoniumbromid in 150 ml THF werden bei 0°C 21.65 ml (54.12 mmol) einer 2.5 M Lösung von n-Butyllithium in Hexan langsam zugetropft und das Gemisch 45 min bei dieser Temperatur nachgerührt. Anschließend werden bei dieser Temperatur 6.85 g (19.33 mmol) Dimethyl 4,4'-(2-formylbutan-1,4-diyl)dibenzoat in 100 ml THF langsam zudosiert. Die Reaktionslösung wird 3 h bei 0°C nachgerührt, anschließend mit gesättigter Ammoniumchlorid-Lösung versetzt, mit Wasser und Essigsäureethylester verdünnt, die organische Phase abgetrennt und die wässrige Phase noch zweimal mit Essigsäureethylester nachextrahiert. Die vereinigten organischen Phasen werden über Natriumsulfat getrocknet, filtriert und das Lösungsmittel bis zur Trockene entfernt. Das erhaltene Rohprodukt wird durch Flash-Chromatographie an Kieselgel (Laufmittel: Cyclohexan/Essigsäureethylester 4:1) gereinigt. Es werden 8.21 g (17.75 mmol, 76% d. Th.) eines gelblichen Öls erhalten.

LC-MS (Methode 7): Rₜ = 3.14 min; m/z = 463 (M+H⁺).

### Beispiel 64A

### 4-{(4E)-5-(5-Fluor-2-{[4-(trifluormethyl)benzyl]oxy}phenyl)-3-[4-(methoxycarbonyl)benzyl]pent-4-en-1-yl}benzoesäuremethylester

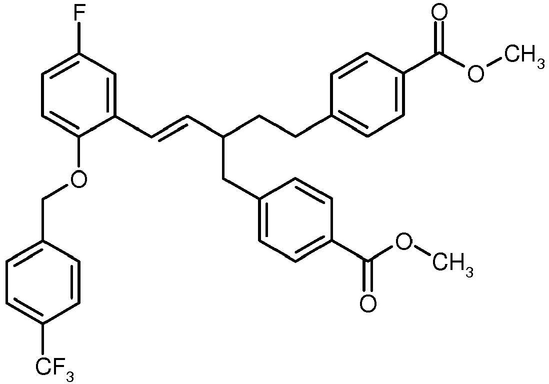

Eine Lösung von 1.4 g (3.03 mmol) 4-{(4*E*)-5-(5-Fluor-2-hydroxyphenyl)-3-[4-(methoxycarbonyl)benzyl]pent-4-en-1-yl}benzoesäuremethylester in 30 ml trockenem Acetonitril wird mit 868 mg (3.6 mmol) 1-(Brommethyl)-4-(trifluormethyl)benzol sowie 627 mg (4.5 mmol) wasserfreiem Kaliumcarbonat versetzt und dann 12 h unter Rückfluss erhitzt. Anschließend wird der Ansatz filtriert und das Filtrat bis zur Trockene eingeengt. Der Rückstand wird chromatographisch an Kieselgel (Laufmittel: Cyclohexan/Essigsäureethylester 5:1) aufgereinigt. Es werden 1.23 g (1.98 mmol, 65% d. Th.) eines farblosen Öls isoliert.

¹H-NMR (400 MHz, DMSO-d₆, δ/ppm): 7.88-7.79 (4H, m), 7.70 (2H, d), 7.54 (2H, d), 7.33-7.24 (5H, m), 7.06-6.97 (2H, m), 6.44 (1H, d), 6.25-6.15 (1H, m), 5.14 (2H, s), 3.82 (3H, s), 3.79 (3H, s), 2.93-2.82 (1H, m), 2.79-2.68 (2H, m), 2.67-2.57 (1H, m), 2.56-2.45 (1H, m), 1.89-1.76 (1H, m), 1.75-1.62 (1H, m).

LC-MS (Methode 8): Rₜ = 5.01 min; m/z = 621 (M+H⁺).

412 mg (0.66 mmol) des so erhaltenen racemischen 4-{(4*E*)-5-(5-Fluor-2-{[4-(trifluormethyl)-benzyl]oxy}phenyl)-3-[4-(methoxycarbonyl)benzyl]pent-4-en-1-yl}benzoesäuremethylesters werden mittels präparativer HPLC an chiraler Phase weiter aufgetrennt. Es werden jeweils enantiomerenrein 93 mg bzw. 87 mg der beiden *E*-Isomere als farblose Feststoffe erhalten (siehe Beispiele 65A und 66A).

### Beispiel 65A

### 4-{(4E)-5-(5-Fluor-2-{[4-(trifluormethyl)benzyl]oxy}phenyl)-3-[4-(methoxycarbonyl)benzyl]pent-4-en-1-yl}benzoesäuremethylester (Enantiomer 1)

### Methode Enantiomerentrennung:

Säule: Daicel Chiralpak AD-H 250 mm x 20 mm, 5 µm; Elutionsmittel: Isohexan / Isopropanol 60:40 (v/v); Fluss: 15 ml/min; UV-Detektion: 220 nm; Temperatur: 28°C.

Rₜ 7.55 min; Reinheit 99%; >99.5% ee
Ausbeute: 93 mg.

### Beispiel 66A

### 4-{(4E)-5-(5-Fluor-2-{[4-(trifluormethyl)benzyl]oxy}phenyl)-3-[4-(methoxycarbonyl)benzyl]pent-4-en-1-yl}benzoesäuremethylester (Enantiomer 2)

Methode Enantiomerentrennung: siehe Beispiel 65A.
Rₜ 8.48 min; Reinheit >98.5%; >97.5% ee
Ausbeute: 87 mg.

### Beispiel 67A

### 4-{(4E)-5-(5-Fluor-2-{[4-(trifluormethoxy)benzyl]oxy}phenyl)-3-[4-(methoxycarbonyl)benzyl]-pent-4-en-1-yl}benzoesäuremethylester

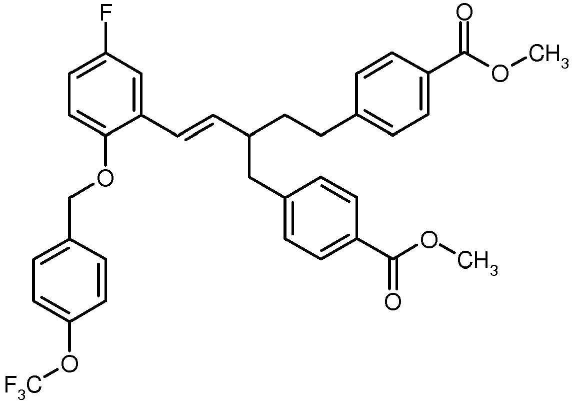

Eine Lösung von 750 mg (1.62 mmol) 4-{(4*E*)-5-(5-Fluor-2-hydroxyphenyl)-3-[4-(methoxycarbonyl)benzyl]pent-4-en-1-yl}benzoesäuremethylest in 19 ml trockenem Acetonitril wird mit 496 mg (1.95 mmol) 1(Brommethyl)-4-(trifluormethoxy)benzol sowie 336 mg (2.43 mmol) wasserfreiem Kaliumcarbonat versetzt und dann 12 h unter Rückfluss erhitzt. Anschließend wird der Ansatz filtriert und das Filtrat bis zur Trockene eingeengt. Der Rückstand wird chromatographisch an Kieselgel (Laufmittel: Cyclohexan/Essigsäureethylester 5:1) aufgereinigt. Es werden 883 mg (1.39 mmol, 85% d. Th.) eines farblosen Öls isoliert.

LC-MS (Methode 7): Rₜ = 3.54 min; m/z = 637 (M+H⁺).

880 mg (1.38 mmol) des so erhaltenen racemischen 4-{(4*E*)-5-(5-Fluor-2-{[4-(trifluormethoxy)-benzyl]oxy}phenyl)-3-[4-(methoxycarbonyl)benzyl]pent-4-en-1-yl}benzoesäuremethylesters werden mittels präparativer HPLC an chiraler Phase weiter aufgetrennt. Es werden jeweils enantiomerenrein 444 mg bzw. 320 mg der beiden *E*-Isomere als farblose Feststoffe erhalten (siehe Beispiele 68A und 69A).

### Beispiel 68A

### 4-{(4E)-5-(5-Fluor-2-{[4-(trifluormethoxy)benzyl]oxy}phenyl)-3-[4-(methoxycarbonyl)benzyl]-pent-4-en-1-yl}benzoesäuremethylester (Enantiomer 1)

### Methode Enantiomerentrennung:

Säule: Daicel Chiralpak AD-H 250 mm x 20 mm, 5 µm; Elutionsmittel: Isohexan / Isopropanol 70:30 (v/v); Fluss: 15 ml/min; UV-Detektion: 220 nm; Temperatur: 30°C.

Rₜ 6.05 min; Reinheit >99.5%; >99.5% ee
Ausbeute: 444 mg
¹H-NMR (400 MHz, DMSO-d₆, δ/ppm): 7.88-7.78 (4H, m), 7.48 (2H, d), 7.37-7.21 (7H, m), 7.09-6.95 (2H, m), 6.41 (1H, d), 6.25-6.14 (1H, m), 5.06 (2H, s), 3.82 (3H, s), 3.79 (3H, s), 2.93-2.82 (1H, m), 2.79-2.67 (2H, m), 2.66-2.56 (1H, m), 2.55-2.42 (1H, m), 1.88-1.75 (1H, m), 1.74-1.61 (1H, m).

LC-MS (Methode 2): Rₜ = 3.37 min; m/z = 637 (M+H⁺).

### Beispiel 69A

### 4-{(4E)-5-(5-Fluor-2-{[4-(trifluormethoxy)benzyl]oxy}phenyl)-3-[4-(methoxycarbonyl)benzyl]-pent-4-en-1-yl}benzoesäuremethylester (Enantiomer 2)

Methode Enantiomerentrennung: siehe Beispiel 68A.

Rₜ 6.35 min; Reinheit >99.5%; >98.8% ee Ausbeute: 320 mg

¹H-NMR: siehe Beispiel 68A
LC-MS (Methode 2): Rₜ = 3.37 min; m/z = 637 (M+H⁺).

### Beispiel 70A

### 4-[(3E)-2-[2-(4-Cyanophenyl)ethyl]-4-{2-[(6-phenylhexyl)oxy]phenyl}but-3-en-1-yl]benzoesäuremethylester

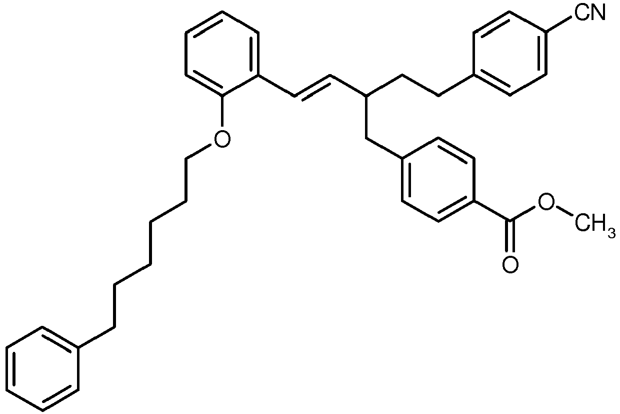

Eine Lösung von 200 mg (0.49 mmol) 4-[(3*E*)-2-[2-(4-Cyanophenyl)ethyl]-4-(2-hydroxyphenyl)-but-3-en-1-yl]benzoesäuremethylester in 25 ml Acetonitril wird mit 176 mg (0.73 mmol) (6-Bromhexyl)benzol sowie 101 mg (0.73 mmol) Kaliumcarbonat versetzt und dann 12 h unter Rückfluss gerührt. Nach dem Abkühlen wird das Kaliumcarbonat abfiltriert und das Filtrat eingeengt. Es werden 364 mg (0.48 mmol, 98% d. Th., Reinheit 76%) eines gelblichen Öls erhalten.

LC-MS (Methode 2): Rₜ = 3.47 min; MS (ESIpos): m/z = 572 (M+H⁺).

### Beispiel 71A

### {2-[4-(Methoxycarbonyl)phenyl]ethyl}-malonsäurediallylester

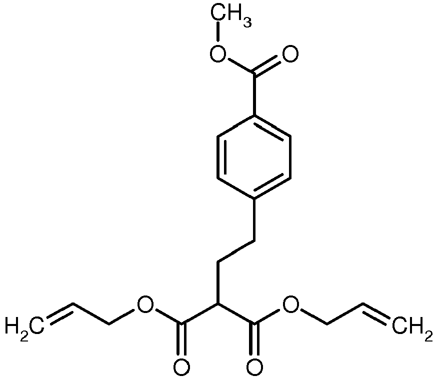

Eine Lösung von 6.91 g (37.52) mmol) Malonsäurediallylester in 50 ml Dioxan wird bei 0°C portionsweise mit 1.125 g (28.14 mmol) Natriumhydrid versetzt (Vorsicht: Wasserstoffentwicklung). Nach Erwärmen auf Raumtemperatur wird der Ansatz 1 h bei 40°C nachgerührt. Anschließend werden 4.56 g (18.76 mmol) 4-(2-Bromethyl)-benzoesäuremethylester [CAS Reg.-Nr. 136333-97-6], gelöst in 20 ml Dioxan, langsam bei 40°C zugetropft und die Reaktionslösung über Nacht bei 110°C gerührt. Nach Abkühlen auf Raumtemperatur wird das Reaktionsgemisch auf 1200 ml Wasser gegeben. Dabei ist darauf zu achten, dass der pH-Wert <7 bleibt (gegebenenfalls werden wenige ml 1 M Salzsäure bis ca. pH 2 zudosiert). Der Ansatz wird dann dreimal mit Essigsäureethylester extrahiert, die vereinigten organischen Phasen mit gesättigter Natriumchlorid-Lösung gewaschen und über Natriumsulfat getrocknet. Nach Filtration wird das Lösungsmittel im Vakuum bis zur Trockene eingeengt. Anschließend wird durch Hochvakuum-Destillation überschüssiger Malonsäurediallylester entfernt (Siedepunkt: 57°C, 0.074 mbar). Der Destillationsrückstand wird durch Flash-Chromatographie an Kieselgel (Laufmittel: Petrolether/Essigsäureethylester 10:1) gereinigt. Es werden 6.01 g (17.35 mmol, 92% d. Th.) eines farblosen Feststoffes erhalten.

LC-MS (Methode 7): Rₜ = 2.75 min; m/z = 347 (M+H)⁺.

### Beispiel 72A

### 4-[(4E)-5-[2-(4-Brombutoxy)phenyl]-3-(4-cyanobenzyl)pent-4-en-1-yl]benzoesäuremethylester

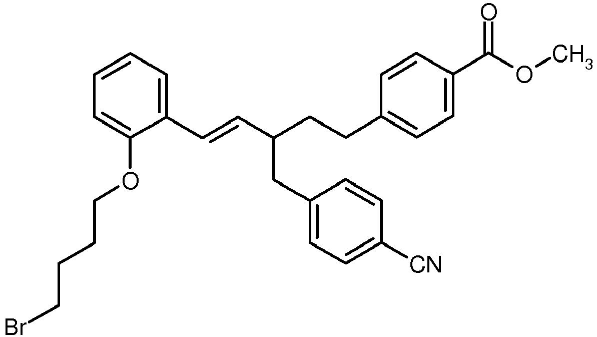

Eine Lösung von 664 mg (1.62 mmol) *E*-4-[3-(4-Cyanobenzyl)-5-(2-hydroxyphenyl)pent-4-enyl]-benzoesäuremethylester (Enantiomer 1, Beispiel 51A) in 28 ml trockenem Acetonitril wird mit 1743 mg (8.07 mmol) 1,4-Dibrombutan sowie 446 mg (3.23 mmol) wasserfreiem Kaliumcarbonat versetzt und dann 12 h unter Rückfluss erhitzt. Anschließend wird der Ansatz filtriert und das Filtrat zur Trockene eingeengt. Der Rückstand wird chromatographisch an Kieselgel (Laufmittel: Cyclohexan/Essigsäureethylester 10:1) aufgereinigt. Es werden 790 mg (1.44 mmol, 89% d. Th.) eines farblosen Öls isoliert.

¹H-NMR (400 MHz, CDCl₃, δ/ppm): 7.95 (2H, d), 7.53 (2H, d), 7.36 (1H, d), 7.29-7.14 (5H, m), 6.91 (1H, t), 6.83 (1H, d), 6.54 (1H, d), 6.01-5.91 (1H, m), 4.05-3.95 (2H, m), 3.91 (3H, s), 3.47 (2H, t), 2.89-2.72 (2H, m), 2.70-2.58 (1H, m), 2.54-2.42 (1H, m), 2.09-1.90 (4H, m), 1.89-1.79 (1H, m), 1.78-1.59 (2H, m).

LC-MS (Methode 7): Rₜ = 3.40 min; m/z = 547 (M+H)⁺.

### Beispiel 73A

### 4-[(4E)-3-(4-Cyanobenzyl)-5-(2-{4-[4-(trifluormethyl)phenoxy]butoxy}phenyl)pent-4-en-1-yl]-benzoesäuremethylester

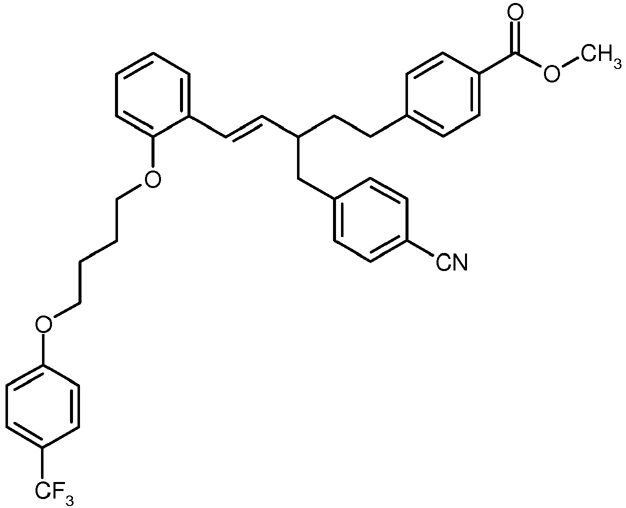

Eine Lösung von 392 mg (0.72 mmol) 4-[(4*E*)-5-[2-(4-Brombutoxy)phenyl]-3-(4-cyanobenzyl)-pent-4-en-1-yl]benzoesäuremethylester in 10 ml trockenem Acetonitril wird mit 174 mg (1.08 mmol) 4-Trifluormethylphenol sowie 148 mg (1.08 mmol) wasserfreiem Kaliumcarbonat versetzt und dann 12 h unter Rückfluss erhitzt. Anschließend wird der Ansatz filtriert und das Filtrat zur Trockene eingeengt. Es werden 450 mg (0.61 mmol, Reinheit 86%, 86% d. Th.) eines gelblichen Öls isoliert.

LC-MS (Methode 2): Rₜ = 3.38 min; m/z = 628 (M+H)⁺.

### Beispiel 74A

### 4-[(4E)-3-(4-Cyanobenzyl)-5-{2-[4-(4-fluorphenoxy)butoxy]phenyl}pent-4-en-1-yl]benzoesäuremethylester

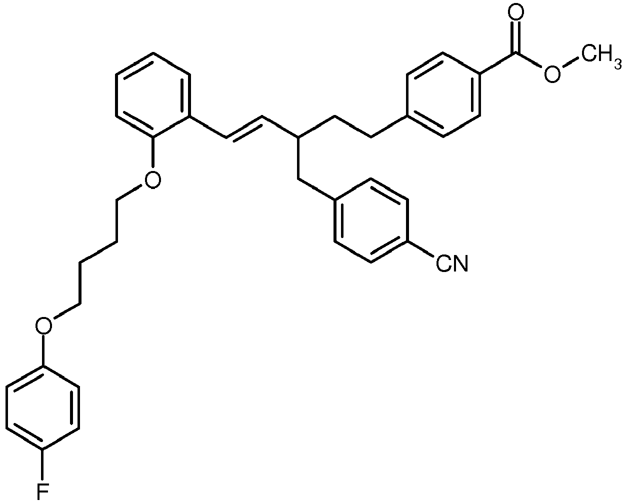

Eine Lösung von 392 mg (0.72 mmol) 4-[(4*E*)-5-[2-(4-Brombutoxy)phenyl]-3-(4-cyanobenzyl)-pent-4-en-1-yl]benzoesäuremethyleste in 10 ml trockenem Acetonitril wird mit 120 mg (1.08 mmol) 4-Fluorphenol sowie 148 mg (1.08 mmol) wasserfreiem Kaliumcarbonat versetzt und dann 12 h unter Rückfluss erhitzt. Anschließend wird der Ansatz filtriert und das Filtrat zur Trockene eingeengt. Es werden 445 mg (0.71 mmol, Reinheit 93%, 99% d. Th.) eines gelblichen Öls isoliert.

LC-MS (Methode 2): Rₜ = 3.33 min; m/z = 578 (M+H)⁺.

### Ausführungsbeispiele:

### Beispiel 1

### 4-[(4E)-5-{2-[(4-tert.-Butylbenzyl)oxy]phenyl}-3-(4-carboxybenzyl)pent-4-en-1-yl]benzoesäure

### (Racemat)

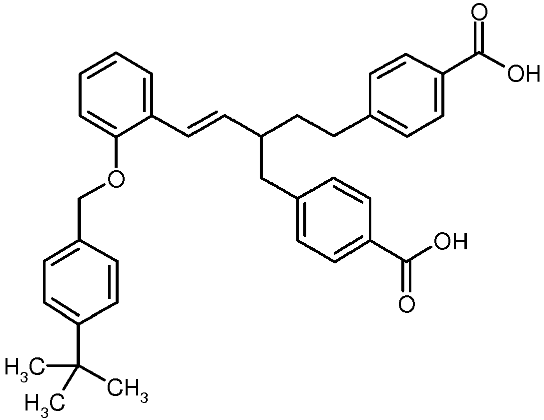

Eine Lösung von 430 mg (0.77 mmol) 4-{(3*E*)-4-{2-[(4-*tert*.-butylbenzyl)oxy]phenyl}-2-[2-(4-cyanophenyl)ethyl]but-3-en-1-yl}benzoesäuremethylester in 20 ml 1-Propanol wird mit 1.73 g (30.84 mmol) Kaliumhydroxid versetzt und 5 Stunden bei 110°C gerührt. Der Ansatz wird mit 1 M Salzsäure auf pH 2 gestellt und eingeengt. Der erhaltene Rückstand wird direkt über präparative HPLC gereinigt. Es werden 290 mg (0.52 mmol, 53% Ausbeute) der Titelverbindung erhalten.

¹H-NMR (400 MHz, DMSO-d₆, δ/ppm): 12.78 (2H, breit), 7.8 (4H, d), 7.4 (1H, d), 7.38 (2H, d), 7.32-7.21 (6H, m), 7.18 (1H, t), 7.05 (1H, d), 6.91 (1H, t), 6.5 (1H, d), 6.12 (1H, dd), 5.05 (2H, s), 2.89-2.81 (1H, m), 2.78-2.67 (2H, m), 2.67-2.56 (1H, m), 2.56-2.5 (1H, m), 1.86-1.72 (1H, m), 1.72-1.59 (1H, m), 1.25 (9H, s).

290 mg (0.52 mmol) der so erhaltenen racemischen 4-[(4*E*)-5-{2-[(4-*tert*.-Butylbenzyl)oxy]-phenyl}-3-(4-carboxybenzyl)pent-4-en-1-yl]benzoesäure werden mittels präparativer HPLC an chiraler Phase weiter aufgetrennt. Es werden jeweils enantiomerenrein 90 mg bzw. 102 mg der beiden E-Isomere als farblose Feststoffe erhalten (siehe Beispiele 2 und 3).

### Beispiel 2

### 4-[(4E)-5-{2-[(4-tert.-Butylbenzyl)oxy]phenyl}-3-(4-carboxybenzyl)pent-4-en-1-yl]benzoesäure

### (Enantiomer 1)

### Methode Enantiomerentrennung:

Säule: Daicel Chiralpak AD-H 250 mm x 20 mm; Elutionsmittel: Isohexan (mit 1% Wasser und 0.2% Essigsäure) / Isopropanol 70:30 (v/v); Fluss: 15 ml/min; UV-Detektion: 220 nm; Temperatur: 25°C.

Rₜ 8.84 min; Reinheit 99%; >99% ee
Ausbeute: 90 mg
LC-MS (Methode 4): Rₜ 2.89 min; MS (ESIpos): m/z 563 (M+H)⁺.

### Beispiel 3

### 4-[(4E)-5-{2-[(4-tert.-Butylbenzyl)oxy]phenyl}-3-(4-carboxybenzyl)pent-4-en-1-yl]benzoesäure

### (Enantiomer 2)

Methode Enantiomerentrennung: siehe Beispiel 2.
Rₜ 9.71 min; Reinheit 99%; >98.5% ee
Ausbeute: 102 mg
LC-MS (Methode 4): Rₜ = 2.89 min; MS (ESIpos): m/z 563 (M+H)⁺.

### Beispiel 4

### 4-((4E)-3-(4-Carboxybenzyl)-5-{2-[(5-phenylpentyl)oxy]phenyl}pent-4-en-1-yl)benzoesäure

### (Racemat)

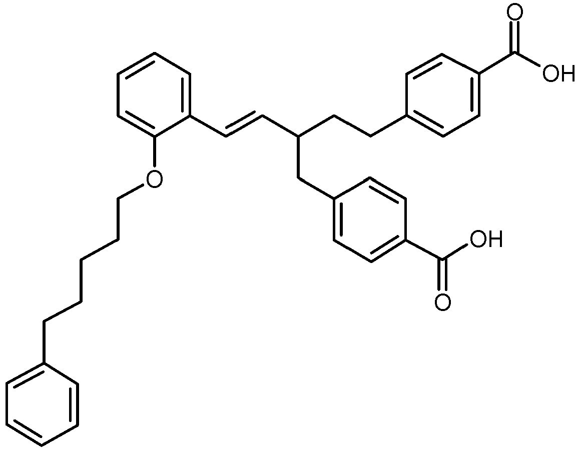

Eine Lösung von 600 mg (1.08 mmol) 4-((3*E*)-2-[2-(4-Cyanophenyl)ethyl]-4-{2-[(5-phenylpentyl)-oxy]phenyl}but-3-en-1-yl)benzoesäuremethylester in 15 ml 1-Propanol wird mit 2.4 g (43 mmol) Kaliumhydroxid versetzt und 7 Stunden bei 110°C gerührt. Der Ansatz wird mit 1 M Salzsäure auf pH 2 gestellt und eingeengt. Der erhaltene Rückstand wird direkt über präparative HPLC gereinigt. Es werden 235 mg (0.42 mmol, 38% Ausbeute) der Titelverbindung als farbloser Feststoff erhalten.

¹H-NMR (300 MHz, DMSO-d₆, δ/ppm): 12.8 (2H, breit), 7.88-7.78 (4H, m), 7.39 (1H, d), 7.32-7.1 (10H, m), 6.98-6.82 (2H, m), 6.41 (1H, d), 6.1 (1H, dd), 3.97-3.82 (2H, m), 2.9-2.8 (1H, m), 2.8-2.69 (2H, m), 2.69-2.58 (1H, m), 1.88-1.52 (9H, m), 1.46-1.32 (2H, m).

235 mg (0.42 mmol) der so erhaltenen racemischen 4-((4*E*)-3-(4-Carboxybenzyl)-5-{2-[(5-phenylpentyl)oxy]phenyl}pent-4-en-1-yl)benzoesäure werden mittels präparativer HPLC an chiraler Phase weiter aufgetrennt. Es werden jeweils enantiomerenrein 90 mg bzw. 91 mg der beiden E-Isomere als farblose Feststoffe erhalten (siehe Beispiele 5 und 6).

### Beispiel 5

### 4-((4E)-3-(4-Carboxybenzyl)-5-{2-[(5-phenylpentyl)oxy]phenyl}pent-4-en-1-yl)benzoesäure

### (Enantiomer 1)

### Methode Enantiomerentrennung:

Säule: Daicel Chiralpak AD-H 250 mm x 20 mm; Elutionsmittel: Isohexan (mit 1% Wasser und 0.2% Essigsäure) / Isopropanol 70:30 (v/v); Fluss: 15 ml/min; UV-Detektion: 220 nm; Temperatur: 27°C.

Rₜ 6.93 min; Reinheit 97.5%; >99.5% ee
Ausbeute: 90 mg.

### Beispiel 6

### 4-((4E)-3-(4-Carboxybenzyl)-5-{2-[(5-phenylpentyl)oxy]phenyl}pent-4-en-1-yl)benzoesäure

### (Enantiomer 2)

Methode Enantiomerentrennung: siehe Beispiel 5.

Rₜ 8.43 min; Reinheit 99.5%; >99.5% ee
Ausbeute: 91 mg.

### Beispiel 7

### (Vergleichsbeispiel)

### 4-[(4E)-3-(4-Carboxybenzyl)-5-(2-{[4-(2-phenylethyl)benzyl]oxy}phenyl)pent-4-en-1-yl]benzoesäure (Racemat)

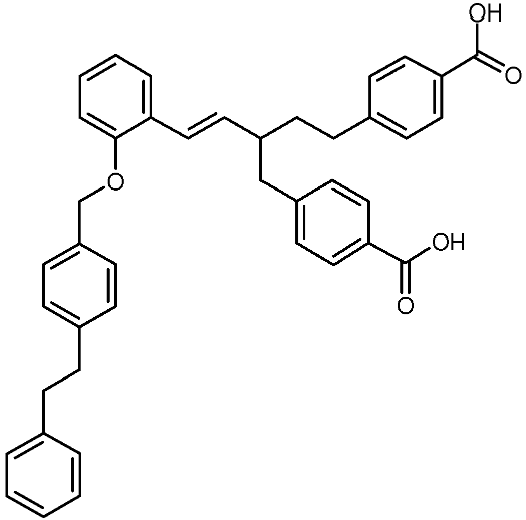

Eine Lösung von 400 mg (0.66 mmol) 4-[(3*E*)-2-[2-(4-Cyanophenyl)ethyl]-4-(2-{[4-(2-phenylethyl)benzyl]oxy}phenyl)but-3-en-1-yl]benzoesäuremethylester in 20 ml 1-Propanol wird mit 1.48 g (26.4 mmol) Kaliumhydroxid versetzt und 5 Stunden bei 110°C gerührt. Der Ansatz wird mit 1 M Salzsäure auf pH 2 gestellt und eingeengt. Der erhaltene Rückstand wird direkt über präparative HPLC gereinigt. Es werden 280 mg (0.46 mmol, 58% Ausbeute) der Titelverbindung als farbloser Feststoff erhalten.

¹H-NMR (300 MHz, DMSO-d₆, δ/ppm): 12.79 (2H, breit), 7.82 (4H, d), 7.42 (1H, d), 7.31-7.12 (14H, m), 7.03 (1H, d), 6.9 (1H, t), 6.49 (1H, d), 6.12 (1H, dd), 5.05 (2H, s), 2.9-2.78 (3H, m), 2.78-2.67 (2H, m), 1.87-1.58 (2H, m).

280 mg (0.46 mmol) der so erhaltenen racemischen 4-[(4*E*)-3-(4-Carboxybenzyl)-5-(2-{[4-(2-phenylethyl)benzyl]oxy}phenyl)pent-4-en-1-yl]benzoesäure werden mittels präparativer HPLC an chiraler Phase weiter aufgetrennt. Es werden jeweils enantiomerenrein 111 mg bzw. 123 mg der beiden *E*-Isomere als farblose Feststoffe erhalten (siehe Beispiele 8 und 9).

### Beispiel 8

### (Vergleichsbeispiel)

### 4-[(4E)-3-(4-Carboxybenzyl)-5-(2-{[4-(2-phenylethyl)benzyl]oxy}phenyl)pent-4-en-1-yl]benzoesäure (Enantiomer 1)

### Methode Enantiomerentrennung:

Säule: Daicel Chiralpak AD-H 250 mm x 20 mm; Elutionsmittel: Isohexan (mit 1% Wasser und 0.2% Essigsäure) / Isopropanol 70:30 (v/v); Fluss: 15 ml/min; UV-Detektion: 220 nm; Temperatur: 25°C.

Rₜ 8.40 min; Reinheit 98.5%; >99.5% ee
Ausbeute: 111 mg.

### Beispiel 9

### (Vergleichsbeispiel)

### 4-[(4E)-3-(4-Carboxybenzyl)-5-(2-{[4-(2-phenylethyl)benzyl]oxy}phenyl)pent-4-en-1-yl]benzoesäure (Enantiomer 2)

Methode Enantiomerentrennung: siehe Beispiel 8.
Rₜ 10.19 min; Reinheit 99%; >99.5% ee
Ausbeute: 123 mg.

### Beispiel 10

### (Vergleichsbeispiel)

### 4-[(4E/Z)-3-(4-Carboxybenzyl)-5-(2-{[4'-(trifluormethyl)biphenyl-4-yl]methoxy}phenyl)pent-4-en-1-yl]benzoesäure

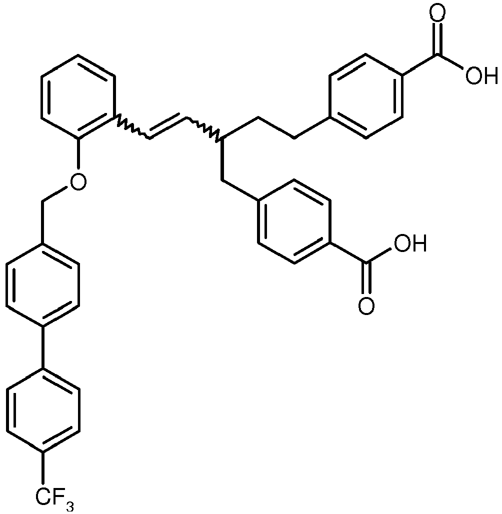

Eine Lösung von 56 mg (0.08 mmol) 4-[(4*E*/*Z*)-3-[4-(Methoxycarbonyl)benzyl]-5-(2-{[4'-(trifluormethyl)biphenyl-4-yl]methoxy}phenyl)pent-4-en-1-yl]benzoesäuremethylester in 2 ml Wassser und 2 ml THF wird mit 4 mg (0.17 mmol) Lithiumhydroxid versetzt und 12 Stunden bei 50°C gerührt. Der Ansatz wird mit Wasser verdünnt, mit 1 M Salzsäure auf pH 5 gestellt und zweimal mit Essigsäureethylester extrahiert. Die vereinigten organischen Phasen werden über Natriumsulfat getrocknet und eingeengt. Es werden 44.6 mg (0.07 mmol, 78% Ausbeute) der Titelverbindung als farbloser Feststoff erhalten.

¹H-NMR (300 MHz, DMSO-d₆, δ/ppm): 12.76 (2H, breit), 7.9-7.78 (8H, m), 7.78-7.6 (3H, m), 7.53-7.38 (3H, m), 7.3-7.14 (5H, m), 6.92 (1H, t), 6.5 (1H, d), 6.12 (1H, dd), 5.18 (2H, s), 2.9-2.58 (5H, m), 1.88-1.55 (2H, m).

### Beispiel 11

### 4-{(4E)-3-(4-Carboxybenzyl)-5-[2-(4,4,4-trifluorbutoxy)phenyl]pent-4-en-1-yl}benzoesäure

### (Racemat)

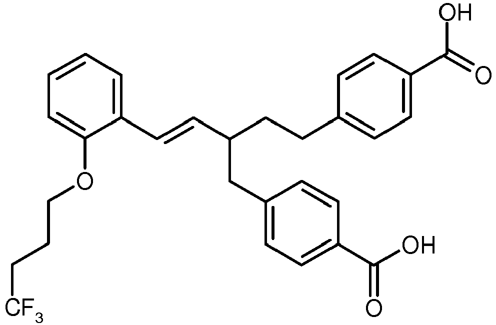

Eine Lösung von 265 mg (0.51 mmol) 4-{(3*E*)-2-[2-(4-Cyanophenyl)ethyl]-4-[2-(4,4,4-trifluorbutoxy)phenyl]but-3-en-1-yl}benzoesäuremethylester in 15 ml 1-Propanol wird mit 1.14 g (20.3 mmol) Kaliumhydroxid versetzt und 12 h bei 110°C gerührt. Nach dem Abkühlen wird der Ansatz mit 1 M Salzsäure sauer gestellt und die ausgefallenen Kristalle abgesaugt und getrocknet. Es werden 250 mg (0.47 mmol, 93% d. Th.) der Titelverbindung erhalten.

LC-MS (Methode 2): Rₜ = 2.63 min; MS (ESIpos): m/z = 527 (M+H)⁺.

### Beispiel 12

### 4-[(4E)-3-(4-Carboxybenzyl)-5-(2-methoxyphenyl)pent-4-en-1-yl]benzoesäure (Racemat)

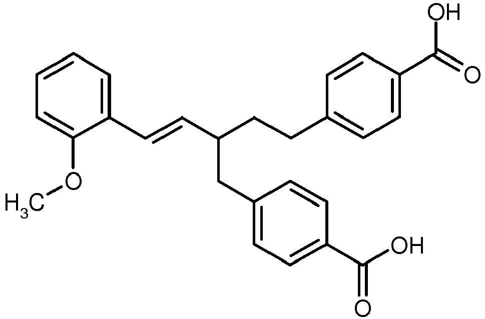

Ausgehend von 632 mg (1.54 mmol) 4-[(3*E*)-2-[2-(4-Cyanophenyl)ethyl]-4-(2-methoxyphenyl)but-3-en-1-yl]benzoesäure und 3.45 g (61.4 mmol) Kaliumhydroxid werden nach dem in Beispiel 11 beschriebenen Verfahren 654 mg (1.5 mmol, 99% d. Th.) der Titelverbindung erhalten.

LC-MS (Methode 4): Rₜ = 2.58 min; MS (ESIpos): m/z = 431 (M+H)⁺.

163 mg (0.34 mmol) der so erhaltenen racemischen 4-[(4*E*)-3-(4-Carboxybenzyl)-5-(2-methoxyphenyl)pent-4-en-1-yl]benzoesäure werden mittels präparativer HPLC an chiraler Phase weiter aufgetrennt. Es werden jeweils enantiomerenrein 69 mg bzw. 49 mg der beiden *E*-Isomere erhalten (siehe Beispiele 13 und 14).

### Beispiel 13

### 4-[(4E)-3-(4-Carboxybenzyl)-5-(2-methoxyphenyl)pent-4-en-1-yl]benzoesäure (Enantiomer 1)

### Methode Enantiomerentrennung:

Säule: Daicel Chiralcel OD-H 250 mm x 20 mm; Elutionsmittel: Isopropanol (mit 1% Wasser und 0.2% Trifluoressigsäure) / Isohexan 30:70 (v/v); Fluss: 15 ml/min; UV-Detektion: 220 nm; Temperatur: 30°C.

Rₜ 5.97 min; Reinheit >99%; >99% ee
Ausbeute: 69 mg
LC-MS (Methode 3): Rt = 2.63 min; MS (ESIpos): m/z = 431 (M+H)⁺
¹H-NMR (400 MHz, DMSO-d₆, δ/ppm): 12.89-12.69 (2H, breit), 7.89-7.78 (4H, m), 7.42 (1H, d), 7.30 (4H, d), 7.20 (1H, t), 6.95 (1H, d), 6.90 (1H, t), 6.46 (1H, d), 6.15-6.02 (1H, m), 3.76 (3H, s), 2.94-2.81 (1H, m), 2.80-2.54 (4H, m), 1.85-1.72 (1H, m), 1.71-1.59 (1H, m).

### Beispiel 14

### 4-[(4E)-3-(4-Carboxybenzyl)-5-(2-methoxyphenyl)pent-4-en-1-yl]benzoesäure (Enantiomer 2)

Methode Enantiomerentrennung: siehe Beispiel 13.
Rₜ 6.98 min; Reinheit >99%; >98% ee
Ausbeute: 49 mg.

### Beispiel 15

### 4-[(4E)-5-(2-Butoxyphenyl)-3-(4-carboxybenzyl)pent-4-en-1-yl]benzoesäure (Racemat)

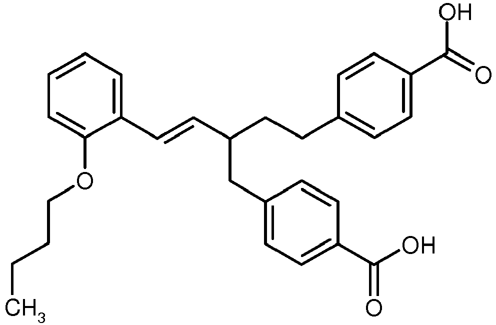

Ausgehend von 220 mg (0.47 mmol) 4-{(3*E*)-4-(2-Butoxyphenyl)-2-[2-(4-cyanophenyl)ethyl]but-3-en-1-yl}benzoesäuremethylester und 1 g (18.8 mmol) Kaliumhydroxid werden nach dem in Beispiel 11 beschriebenen Verfahren 169 mg (0.36 mmol, 76% d. Th.) der Titelverbindung erhalten.

LC-MS (Methode 2): Rt = 2.72 min; MS (ESIpos): m/z = 472 (M+H)⁺
¹H-NMR (400 MHz, DMSO-d₆, δ/ppm): 12.75 (s, 2H), 7.9-7.75 (m, 4H), 7.4 (d, 1H), 7.28 (t, 4H), 7.16 (t, 1H), 6.95-6.83 (m, 2H), 6.42 (d, 1H), 6.1 (dd, 1H), 3.98-3.85 (m, 2H), 2.92-2.82 (m, 1H), 2.8-2.4 (m, 5H), 1.88-1.75 (m, 1H), 1.75-1.6 (m, 3H), 1.45-1.32 (m, 2H), 0.9 (t, 3H).

### Beispiel 16

### 4-{(4E)-3-(4-Carboxybenzyl)-5-[2-(pentyloxy)phenyl]pent-4-en-1-yl}benzoesäure (Racemat)

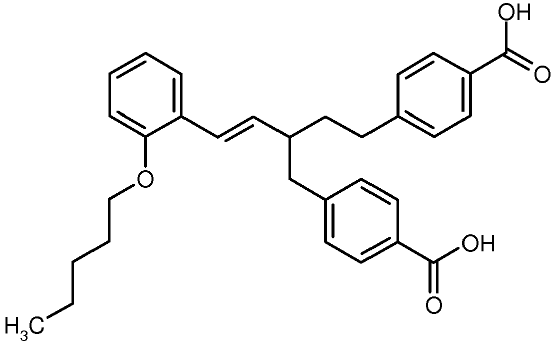

Ausgehend von 195 mg (0.41 mmol) 4-{(3*E*)-2-[2-(4-Cyanophenyl)ethyl]-4-[2-(pentyloxy)phenyl]-but-3-en-1-yl}benzoesäuremethylester und 909 mg (16.2 mmol) Kaliumhydroxid werden nach dem in Beispiel 11 beschriebenen Verfahren 160 mg (0.33 mmol, 74% d. Th.) der Titelverbindung erhalten.

LC-MS (Methode 1): Rₜ = 3.09 min; MS (ESIpos): m/z = 486 (M+H)⁺

¹H-NMR (400 MHz, DMSO-d₆, δ/ppm): 12.75 (s, 2H), 7.9-7.86 (m, 4H), 7.38 (d, 1H), 7.3-7.25 (m, 4H), 7.15 (t, 1H), 6.95-6.83 (m, 2H), 6.44 (d, 1H), 6.1 (dd, 1H), 3.98-3.84 (m, 2H), 2.92-2.81 (m, 1H), 2.8-2.58 (m, 3H), 2.58-2.43 (m, 2H), 1.88-1.75 (m, 1H), 1.75-1.6 (m, 3H), 1.4-1.22 (m, 3H), 0.84 (t, 3H).

160 mg (0.33 mmol) der so erhaltenen racemischen 4-{(4*E*)-3-(4-Carboxybenzyl)-5-[2-(pentyloxy)phenyl]pent-4-en-1-yl}benzoesäure werden mittels präparativer HPLC an chiraler Phase weiter aufgetrennt. Es werden jeweils enantiomerenrein 55 mg bzw. 49 mg der beiden *E*-Isomere erhalten (siehe Beispiele 17 und 18).

### Beispiel 17

### 4-{(4E)-3-(4-Carboxybenzyl)-5-[2-(pentyloxy)phenyl]pent-4-en-1-yl}benzoesäure (Enantiomer 1)

### Methode Enantiomerentrennung:

Säule: Daicel Chiralpak AD-H 250 mm x 20 mm; Elutionsmittel: Isopropanol (mit 1% Wasser und 0.2% Trifluoressigsäure) / Isohexan 25:75 (v/v); Fluss: 15 ml/min; UV-Detektion: 220 nm; Temperatur: 30°C.

Rₜ 6.57 min; Reinheit >94.5%; >99% ee
Ausbeute: 55 mg.

### Beispiel 18

### 4-{(4E)-3-(4-Carboxybenzyl)-5-[2-(pentyloxy)phenyl]pent-4-en-1-yl}benzoesäure (Enantiomer 2)

Methode Enantiomerentrennung: siehe Beispiel 17.
Rₜ 7.41 min; Reinheit >99%; >97% ee
Ausbeute: 49 mg.

### Beispiel 19

### 4-{(4E)-3-(4-Carboxybenzyl)-5-[2-(hexyloxy)phenyl]pent-4-en-1-yl}benzoesäure (Racemat)

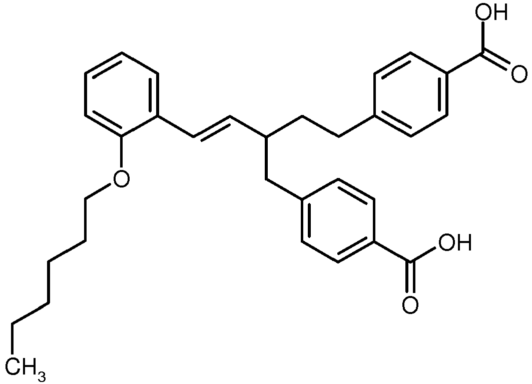

Ausgehend von 190 mg (0.38 mmol) 4-{(3*E*)-2-[2-(4-Cyanophenyl)ethyl]-4-[2-(hexyloxy)phenyl]-but-3-en-1-yl}benzoesäuremethylester und 860 mg (15.3 mmol) Kaliumhydroxid werden nach dem in Beispiel 11 beschriebenen Verfahren 159 mg (0.32 mmol, 83% d. Th.) der Titelverbindung erhalten.

LC-MS (Methode 6): Rt = 6.66 min; MS (ESIpos): m/z = 501 (M+H)⁺

¹H-NMR (400 MHz, DMSO-d₆, δ/ppm): 12.76 (s, 2H), 7.9-7.77 (m, 4H), 7.38 (d, 1H), 7.32-7.23 (m, 4H), 7.16 (t, 1H), 6.94-6.85 (m, 2H), 6.44 (d, 1H), 6.11 (dd, 1H), 3.97-3.84 (m, 2H), 2.91-2.82 (m, 1H), 2.79-2.42 (m, 5H), 1.87-1.74 (m, 1H), 1.74-1.6 (m, 3H), 1.44-1.17 (m, 5H), 0.82 (t, 3H).

159 mg (0.32 mmol) der so erhaltenen racemischen 4-{(4*E*)-3-(4-Carboxybenzyl)-5-[2-(hexyloxy)-phenyl]pent-4-en-1-yl}benzoesäure werden mittels präparativer HPLC an chiraler Phase weiter aufgetrennt. Es werden jeweils enantiomerenrein 56 mg bzw. 46 mg der beiden *E*-Isomere erhalten (siehe Beispiele 20 und 21).

### Beispiel 20

### 4-{(4E)-3-(4-Carboxybenzyl)-5-[2-(hexyloxy)phenyl]pent-4-en-1-yl}benzoesäure (Enantiomer 1)

### Methode Enantiomerentrennung:

Säule: Daicel Chiralpak AD-H 250 mm x 20 mm; Elutionsmittel: Isopropanol (mit 1% Wasser und 0.2% Trifluoressigsäure) / Isohexan 25:75 (v/v); Fluss: 15 ml/min; UV-Detektion: 220 nm; Temperatur: 30°C.

Rₜ 6.05 min; Reinheit >97%; >99% ee
Ausbeute: 56 mg.

### Beispiel 21

### 4-{(4E)-3-(4-Carboxybenzyl)-5-[2-(hexyloxy)phenyl]pent-4-en-1-yl}benzoesäure (Enantiomer 2)

Methode Enantiomerentrennung: siehe Beispiel 20.
Rₜ 6.8 min; Reinheit >98.5%; >99% ee
Ausbeute: 46 mg.

### Beispiel 22

### 4-{(4E)-3-(4-Carboxybenzyl)-5-[2-(heptyloxy)phenyl]pent-4-en-1-yl}benzoesäure (Racemat)

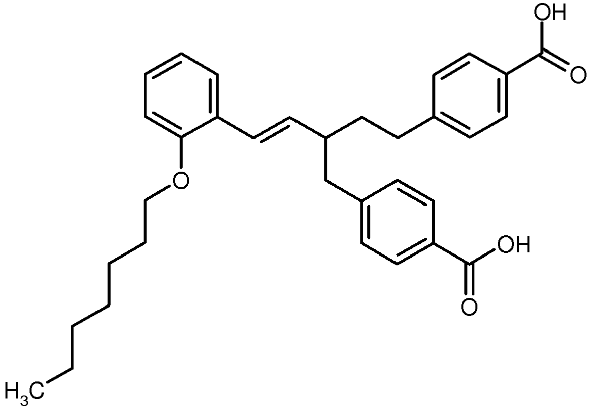

Ausgehend von 190 mg (0.37 mmol) 4-{(3*E*)-2-[2-(4-Cyanophenyl)ethyl]-4-[2-(heptyloxy)phenyl]-but-3-en-1-yl}benzoesäuremethylester und 837 mg (14.9 mmol) Kaliumhydroxid werden nach dem in Beispiel 11 beschriebenen Verfahren 190 mg (0.37 mmol, 99% d. Th.) der Titelverbindung erhalten.

LC-MS (Methode 1): Rₜ = 3.3 min; MS (ESIpos): m/z = 515 (M+H)⁺

¹H-NMR (400 MHz, DMSO-d₆, δ/ppm): 12.75 (s, 2H), 7.9-7.75 (m, 4H), 7.38 (d, 1H), 7.32-7.2 (m, 4H), 7.17 (t, 1H), 6.95-6.82 (m, 2H), 6.45 (d, 1H), 6.12 (dd, 1H), 3.98-3.85 (m, 2H), 2.9-2.8 (m, 1H), 2.8-2.4 (m, 4H), 1.88-1.75 (m, 1H), 1.75-1.6 (m, 3H), 1.42-1.12 (m, 8H), 0.8 (t, 3H).

### Beispiel 23

### (Vergleichsbeispiel)

### 4-[(4E/Z)-3-(4-Carboxybenzyl)-5-phenylpent-4-en-1-yl]benzoesäure (Racemat)

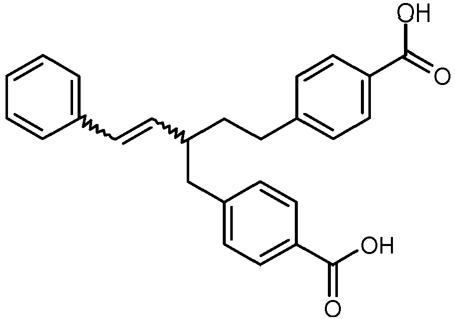

Ausgehend von 484 mg (1.22 mmol) 4-{(3*E*/*Z*)-2-[2-(4-Cyanophenyl)ethyl]-4-phenylbut-3-en-1-yl}benzoesäuremethylester und 2.75 g (48.95 mmol) Kaliumhydroxid werden nach dem in Beispiel 11 beschriebenen Verfahren 453 mg (1.13 mmol, 92% d. Th.) der Titelverbindung erhalten. LC-MS (Methode 1): Rₜ = 2.88 min; MS (ESIpos): m/z = 485 (M+H)⁺.

450 mg (1.12 mmol) der so erhaltenen racemischen 4-[(4*E*/*Z*)-3-(4-Carboxybenzyl)-5-phenylpent-4-en-1-yl]benzoesäure werden mittels präparativer HPLC an chiraler Phase weiter aufgetrennt. Es werden jeweils enantiomerenrein 123 mg bzw. 127 mg der beiden *E*-Isomere erhalten (siehe Beispiele 24 und 25).

### Beispiel 24

### (Vergleichsbeispiel)

### 4-[(4E)-3-(4-Carboxybenzyl)-5-phenylpent-4-en-1-yl]benzoesäure (Enantiomer 1)

### Methode Enantiomerentrennung:

Säule: Daicel Chiralcel OD-H 250 mm x 20 mm; Elutionsmittel: Isopropanol (mit 1% Wasser und 0.2% Trifluoressigsäure) / Isohexan 50:50 (v/v); Fluss: 15 ml/min; UV-Detektion: 220 nm; Temperatur: 40°C.

Rₜ 21.37 min; Reinheit >99.5%; >99% ee
Ausbeute: 123 mg
¹H-NMR (400 MHz, DMSO-d₆, δ/ppm): 12.75 (br. s, 2H), 7.82 (t, 4H), 7.38-7.23 (m, 8H), 7.19 (t, 1H), 6.3-6.12 (m, 2H), 2.92-2.82 (m, 1H), 2.8-2.55 (m, 4H), 1.85-1.62 (m, 2H).

### Beispiel 25

### (Vergleichsbeispiel)

### 4-[(4E)-3-(4-Carboxybenzyl)-5-phenylpent-4-en-1-yl]benzoesäure (Enantiomer 2)

Methode Enantiomerentrennung: siehe Beispiel 24.
Rₜ 25.6 min; Reinheit >99.5%; >99% ee
Ausbeute: 127 mg.

### Beispiel 26

### (Vergleichsbeispiel)

### 4-{(4E/Z)-3-(4-Carboxybenzyl)-5-[2-(trifluormethoxy)phenyl]pent-4-en-1-yl}benzoesäure

### (Racemat)

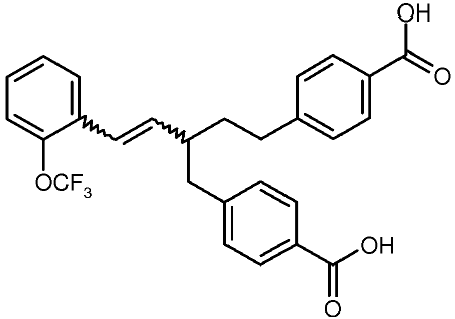

Ausgehend von 500 mg (1.043 mmol) 4-{(3*E*/*Z*)-2-[2-(4-Cyanophenyl)ethyl]-4-[2-(trifluormethoxy)phenyl]but-3-en-1-yl}benzoesäuremethylester und 2.34 g (41.7 mmol) Kaliumhydroxid werden nach dem in Beispiel 11 beschriebenen Verfahren 440 mg (0.91 mmol, 87% d. Th.) der Titelverbindung erhalten.

LC-MS (Methode 1): Rₜ = 2.73 min; MS (ESIpos): m/z = 485 (M+H)⁺
440 mg (0.91 mmol) der so erhaltenen racemischen 4-{(4*E*/*Z*)-3-(4-Carboxybenzyl)-5-[2-(trifluormethoxy)phenyl]pent-4-en-1-yl}benzoesäure werden mittels präparativer HPLC an chiraler Phase weiter aufgetrennt. Es werden jeweils enantiomerenrein 108 mg bzw. 107 mg der beiden *E*-Isomere erhalten (siehe Beispiele 27 und 28).

### Beispiel 27

### (Vergleichsbeispiel)

### 4-{(4E)-3-(4-Carboxybenzyl)-5-[2-(trifluormethoxy)phenyl]pent-4-en-1-yl}benzoesäure

### (Enantiomer 1)

### Methode Enantiomerentrennung:

Säule: Daicel Chiralpak AD-H 250 mm x 20 mm; Elutionsmittel: Isopropanol (mit 1% Wasser und 0.2% Trifluoressigsäure) / Isohexan 25:75 (v/v); Fluss: 15 ml/min; UV-Detektion: 220 nm; Temperatur: 29°C.

Rₜ 5.85 min; Reinheit >98.8%; >99% ee
Ausbeute: 108 mg
¹H-NMR (400 MHz, DMSO-d₆, δ/ppm): 12.8 (br. s, 2H), 7.88-7.8 (m, 4H), 7.7-7.65 (m, 1H), 7.39-7.32 (m, 2H), 7.29 (d, 5H), 6.37-6.21 (m, 2H), 2.9 (dd, 1H), 2.8-2.54 (m, 4H), 1.89-1.65 (m, 2H).

### Beispiel 28

### (Vergleichsbeispiel)

### 4-{(4E)-3-(4-Carboxybenzyl)-5-[2-(trifluormethoxy)phenyl]pent-4-en-1-yl}benzoesäure

### (Enantiomer 2)

Methode Enantiomerentrennung: siehe Beispiel 27.
Rₜ 6.78 min; Reinheit >99.5%; >98% ee

Ausbeute: 107 mg.

### Beispiel 29

### 4-[(4E)-3-(4-Carboxybenzyl)-5-(2-propoxyphenyl)pent-4-en-1-yl]benzoesäure (Racemat)

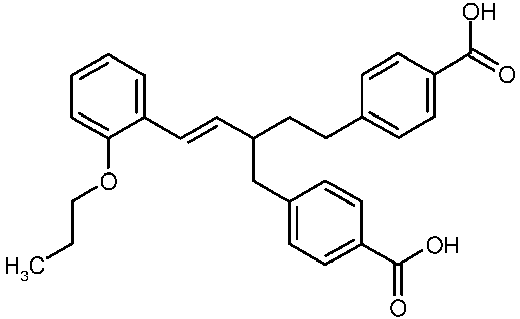

Ausgehend von 200 mg (0.44 mmol) 4-[(3*E*)-2-[2-(4-Cyanophenyl)ethyl]-4-(2-propoxyphenyl)but-3-en-1-yl]benzoesäuremethylester und 989.5 mg (17.6 mmol) Kaliumhydroxid werden nach dem in Beispiel 11 beschriebenen Verfahren 21 mg (0.05 mmol, 10% d. Th.) der Titelverbindung erhalten.

LC-MS (Methode 1): Rₜ = 2.87 min; MS (ESIpos): m/z = 459 (M+H)⁺.

11 mg (0.02 mmol) der so erhaltenen racemischen 4-[(4E)-3-(4-Carboxybenzyl)-5-(2-propoxyphenyl)pent-4-en-1-yl]benzoesäure werden mittels präparativer HPLC an chiraler Phase weiter aufgetrennt. Es werden jeweils enantiomerenrein 6 mg bzw. 4 mg der beiden *E*-Isomere erhalten (siehe Beispiele 30 und 31).

### Beispiel 30

### 4-[(4E)-3-(4-Carboxybenzyl)-5-(2-propoxyphenyl)pent-4-en-1-yl]benzoesäure (Enantiomer 1)

### Methode Enantiomerentrennung:

Säule: Daicel Chiralpak AD-H 250 mm x 20 mm, 5 µm; Elutionsmittel: Isopropanol (mit 1% Wasser und 0.2% Trifluoressigsäure) / Isohexan 28:72 (v/v); Fluss: 15 ml/min; UV-Detektion: 220 nm; Temperatur: 25°C.

Rₜ 8.27 min; Reinheit >97%; >99% ee
Ausbeute: 6 mg
LC-MS (Methode 1): Rₜ = 2.87 min; MS (ESIpos): m/z = 459 (M+H)⁺.

### Beispiel 31

### 4-[(4E)-3-(4-Carboxybenzyl)-5-(2-propoxyphenyl)pent-4-en-1-yl]benzoesäure (Enantiomer 2)

Methode Enantiomerentrennung: siehe Beispiel 30.
Rₜ 9.21 min; Reinheit >94.5%; >99% ee
Ausbeute: 4 mg
LC-MS (Methode 1): Rₜ = 2.87 min; MS (ESIpos): m/z = 459 (M+H)⁺.

### Beispiel 32

### 4-[(4E)-3-(4-Carboxybenzyl)-5-(2-ethoxyphenyl)pent-4-en-1-yl]benzoesäure (Racemat)

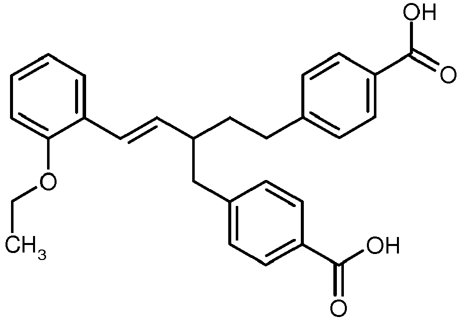

Ausgehend von 195 mg (0.44 mmol) 4-[(3*E*)-2-[2-(4-Cyanophenyl)ethyl]-4-(2-ethoxyphenyl)but-3-en-1-yl]benzoesäuremethylester und 995.6 mg (17.7 mmol) Kaliumhydroxid werden nach dem in Beispiel 11 beschriebenen Verfahren 60 mg (0.13 mmol, 30% d. Th.) der Titelverbindung erhalten.

LC-MS (Methode 1): Rₜ = 2.76 min; MS (ESIpos): m/z = 445 (M+H)⁺.

52 mg (0.12 mmol) der so erhaltenen racemischen 4-[(4E)-3-(4-Carboxybenzyl)-5-(2-ethoxyphenyl)pent-4-en-1-yl]benzoesäure werden mittels präparativer HPLC an chiraler Phase weiter aufgetrennt. Es werden jeweils enantiomerenrein 24 mg bzw. 25 mg der beiden *E*-Isomere erhalten (siehe Beispiele 33 und 34).

### Beispiel 33

### 4-[(4E)-3-(4-Carboxybenzyl)-5-(2-ethoxyphenyl)pent-4-en-1-yl]benzoesäure (Enantiomer 1)

### Methode Enantiomerentrennung:

Säule: Daicel Chiralpak AD-H 250 mm x 20 mm, 5 µm; Elutionsmittel: Ethanol (mit 1% Wasser und 0.2% Trifluoressigsäure) / Isohexan 20:80 (v/v); Fluss: 15 ml/min; UV-Detektion: 220 nm; Temperatur: 25°C.

Rₜ 8.28 min; Reinheit >99.5%; >99.5% ee
Ausbeute: 24 mg
¹H-NMR (400 MHz, DMSO-d₆, δ/ppm): 12.78 (br. s, 2H), 7.92-7.73 (m, 4H), 7.41 (d, 1H), 7.36-7.22 (m, 4H), 7.16 (t, 1H), 6.99-6.82 (m, 2H), 6.44 (d, 1H), 6.18-6.01 (m, 1H), 4.06-3.89 (m, 2H), 2.94-2.81 (m, 1H), 2.80-2.69 (m, 2H), 2.68-2.57 (m, 1H), 2.56-2.41 (m, 1H), 1.89-1.74 (m, 1H), 1.73-1.60 (m, 1H), 1.27 (t, 3H).

LC-MS (Methode 1): Rₜ = 2.76 min; MS (ESIpos): m/z = 445 (M+H)⁺.

### Beispiel 34

### 4-[(4E)-3-(4-Carboxybenzyl)-5-(2-ethoxyphenyl)pent-4-en-1-yl]benzoesäure (Enantiomer 2)

Methode Enantiomerentrennung: siehe Beispiel 33.
Rₜ 9.34 min; Reinheit >99.5%; >99.5% ee
Ausbeute: 25 mg
¹H-NMR: siehe Beispiel 33
LC-MS (Methode 1): Rₜ = 2.76 min; MS (ESIpos): m/z = 445 (M+H)⁺.

### Beispiel 35

### 4-{(4E)-3-(4-Carboxybenzyl)-5-[2-(3-phenylpropoxy)phenyl]pent-4-en-1-yl}benzoesäure

### (Racemat)

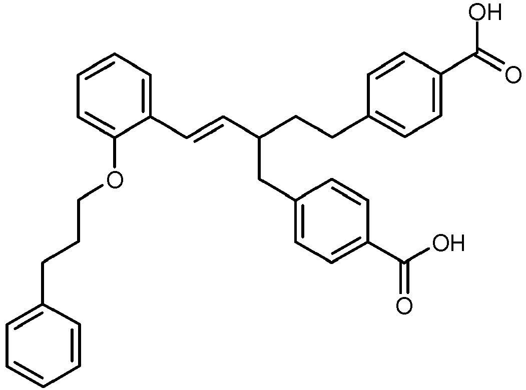

Ausgehend von 270 mg (0.51 mmol) 4-{(3*E*)-2-[2-(4-Cyanophenyl)ethyl]-4-[2-(3-phenylpropoxy)-phenyl]but-3-en-1-yl}benzoesäuremethylester und 1143 mg (20.4 mmol) Kaliumhydroxid werden nach dem in Beispiel 11 beschriebenen Verfahren 194 mg (0.34 mmol, Reinheit 94%, 67% d. Th.) der Titelverbindung erhalten.

¹H-NMR (400 MHz, DMSO-d₆, δ/ppm): 12.77 (br. s, 2H), 7.89-7.75 (4H, m), 7.40 (1H, d), 7.33-7.20 (6H, m), 7.19-7.10 (4H, m), 6.94-6.83 (2H, m), 6.49 (1H, d), 6.21-6.07 (1H, m), 3.98-3.82 (2H, m), 2.94-2.83 (1H, m), 2.82-2.58 (6H, m), 2.05-1.93 (2H, m), 1.89-1.75 (1H, m), 1.74-1.60 (1H, m).

LC-MS (Methode 4): Rₜ = 3.02 min; MS (ESIpos): m/z = 535 (M+H)⁺.

190 mg (0.35 mmol) der so erhaltenen 4-{(4*E*)-3-(4-Carboxybenzyl)-5-[2-(3-phenylpropoxy)-phenyl]pent-4-en-1-yl}benzoesäure werden mittels präparativer HPLC an chiraler Phase weiter aufgetrennt. Es werden jeweils enantiomerenrein 93 mg bzw. 90 mg der beiden *E*-Isomere erhalten (siehe Beispiele 36 und 37).

### Beispiel 36

### 4-{(4E)-3-(4-Carboxybenzyl)-5-[2-(3-phenylpropoxy)phenyl]pent-4-en-1-yl}benzoesäure

### (Enantiomer 1)

### Methode Enantiomerentrennung:

Säule: Daicel Chiralpak AD 250 mm x 20 mm; Elutionsmittel: Isopropanol (mit 1% Wasser und 0.2% Trifluoressigsäure) / Isohexan 23:77 (v/v); Fluss: 15 ml/min; UV-Detektion: 215 nm; Temperatur: 30°C.

Rₜ 11.91 min; Reinheit >99.5%; >99.5% ee
Ausbeute: 93 mg
LC-MS (Methode 2): Rₜ = 2.85 min; MS (ESIpos): m/z = 535 (M+H)⁺.

### Beispiel 37

### 4-{(4E)-3-(4-Carboxybenzyl)-5-[2-(3-phenylpropoxy)phenyl]pent-4-en-1-yl}benzoesäure

### (Enantiomer 2)

Methode Enantiomerentrennung: siehe Beispiel 36.
Rₜ 14.72 min; Reinheit >99.5%; >99.5% ee
Ausbeute: 90 mg
LC-MS (Methode 2): Rₜ = 2.85 min; MS (ESIpos): m/z = 535 (M+H)⁺.

### Beispiel 38

### 4-{(4E)-3-(4-Carboxybenzyl)-5-[2-(4-phenylbutoxy)phenyl]pent-4-en-1-yl}benzoesäure (Racemat)

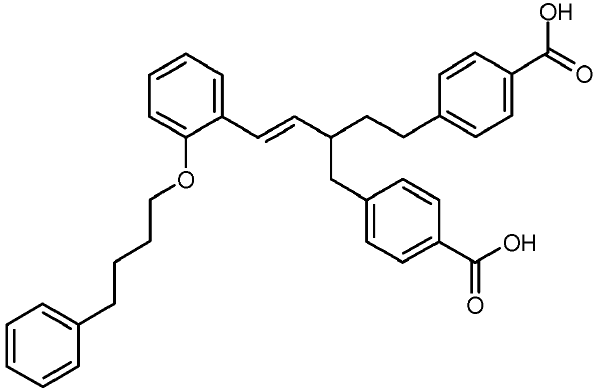

Ausgehend von 300 mg (0.55 mmol) 4-{(3*E*)-2-[2-(4-Cyanophenyl)ethyl]-4-[2-(4-phenylbutoxy)-phenyl]but-3-en-1-yl}benzoesäuremethylester und 1238 mg (22 mmol) Kaliumhydroxid werden nach dem in Beispiel 11 beschriebenen Verfahren 74 mg (0.13 mmol, 24% d. Th.) der Titelverbindung erhalten.

LC-MS (Methode 1): Rₜ = 3.15 min; MS (ESIpos): m/z = 549 (M+H)⁺.

60 mg (0.11 mmol) der so erhaltenen 4-{(4*E*)-3-(4-Carboxybenzyl)-5-[2-(4-phenylbutoxy)phenyl]-pent-4-en-1-yl}benzoesäure werden mittels präparativer HPLC an chiraler Phase weiter aufgetrennt. Es werden jeweils enantiomerenrein 20 mg bzw. 28 mg der beiden *E*-Isomere erhalten (siehe Beispiele 39 und 40).

### Beispiel 39

### 4-{(4E)-3-(4-Carboxybenzyl)-5-[2-(4-phenylbutoxy)phenyl]pent-4-en-1-yl}benzoesäure

### (Enantiomer 1)

### Methode Enantiomerentrennung:

Säule: Daicel Chiralpak AD 250 mm x 20 mm; Elutionsmittel: Isopropanol (mit 1% Wasser und 0.2% Trifluoressigsäure) / Isohexan 23:77 (v/v); Fluss: 15 ml/min; UV-Detektion: 215 nm; Temperatur: 30°C.

Rt 11.36 min; >99.5% ee
Ausbeute: 20 mg
¹H-NMR (400 MHz, DMSO-d₆, δ/ppm): 12.78 (br. s, 2H), 7.89-7.77 (4H, m), 7.39 (1H, d), 7.31-7.19 (6H, m), 7.19-7.09 (4H, m), 6.96-6.82 (2H, m), 6.44 (1H, d), 6.16-6.04 (1H, m), 4.02-3.87 (2H, m), 2.90-2.80 (1H, m), 2.78-2.66 (2H, m), 2.65-2.56 (3H, m), 2.55-2.41 (1H, m), 1.88-1.73 (1H, m), 1.72-1.58 (5H, m).

LC-MS (Methode 4): Rₜ = 3.14 min; MS (ESIpos): m/z = 549 (M+H)⁺.

### Beispiel 40

### 4-{(4E)-3-(4-Carboxybenzyl)-5-[2-(4-phenylbutoxy)phenyl]pent-4-en-1-yl}benzoesäure

### (Enantiomer 2)

Methode Enantiomerentrennung: siehe Beispiel 39.
Rt 13.29 min; >99.5% ee
Ausbeute: 28 mg
¹H-NMR: siehe Beispiel 39
LC-MS (Methode 4): Rₜ = 3.14 min; MS (ESIpos): m/z = 549 (M+H)⁺.

### Beispiel 41

### 4-[(4E)-3-(4-Carboxybenzyl)-5-{2-[(5,5,5-trifluorpentyl)oxy]phenyl}pent-4-en-1-yl]benzoesäure

### (Racemat)

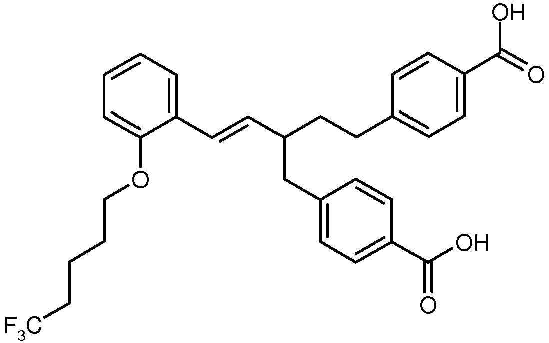

Ausgehend von 280 mg (0.52 mmol) 4-[(3*E*)-2-[2-(4-Cyanophenyl)ethyl]-4-{2-[(5,5,5-trifluorpentyl)oxy]phenyl}but-3-en-1-yl]benzoesäuremethylester und 1173 mg (20.9 mmol) Kaliumhydroxid werden nach dem in Beispiel 11 beschriebenen Verfahren 148 mg (0.27 mmol, 52% d. Th.) der Titelverbindung erhalten.

LC-MS (Methode 1): Rₜ = 2.97 min; MS (ESIpos): m/z = 541 (M+H)⁺.

148 mg (0.27 mmol) der so erhaltenen 4-[(4*E*)-3-(4-Carboxybenzyl)-5-{2-[(5,5,5-trifluorpentyl)-oxy]phenyl}pent-4-en-1-yl]benzoesäure werden mittels präparativer HPLC an chiraler Phase weiter aufgetrennt. Es werden jeweils enantiomerenrein 71 mg bzw. 55 mg der beiden *E*-Isomere erhalten (siehe Beispiele 42 und 43).

### Beispiel 42

### 4-[(4E)-3-(4-Carboxybenzyl)-5-{2-[(5,5,5-trifluorpentyl)oxy]phenyl}pent-4-en-1-yl]benzoesäure

### (Enantiomer 1)

### Methode Enantiomerentrennung:

Säule: Daicel Chiralpak AD-H 250 mm x 20 mm, 5 µm; Elutionsmittel: Isopropanol (mit 1% Wasser und 0.2% Trifluoressigsäure) / Isohexan 23:77 (v/v); Fluss: 15 ml/min; UV-Detektion: 215 nm; Temperatur: 30°C.

Rₜ 9.58 min; Reinheit: >99.5%; >99.5% ee
Ausbeute: 71 mg
¹H-NMR (400 MHz, DMSO-d₆, δ/ppm): 12.77 (br. s, 2H), 7.90-7.77 (4H, m), 7.39 (1H, d), 7.33-7.22 (4H, m), 7.17 (1H, t), 6.94 (1H, d), 6.88 (1H, t), 6.42 (1H, d), 6.17-6.03 (1H, m), 4.02-3.89 (2H, m), 2.91-2.82 (1H, m), 2.79-2.68 (2H, m), 2.67-2.56 (1H, m), 2.55-2.41 (1H, m), 2.37-2.20 (2H, m), 1.88-1.72 (3H, m), 1.71-1.55 (3H, m).

### Beispiel 43

### 4-[(4E)-3-(4-Carboxybenzyl)-5-{2-[(5,5,5-trifluorpentyl)oxy]phenyl}pent-4-en-1-yl]benzoesäure

### (Enantiomer 2)

Methode Enantiomerentrennung: siehe Beispiel 42.

Rₜ 11.18 min; Reinheit: 99.5%; >99.5% ee
Ausbeute: 55 mg
¹H-NMR: siehe Beispiel 42.

### Beispiel 44

### (Vergleichsbeispiel)

### 4-{(4E)-3-(4-Carboxybenzyl)-5-[2-({4-[1,2,2,2-tetrafluor-1-(trifluormethyl)ethyl]benzyl}oxy)-phenyl]pent-4-en-1-yl}benzoesäure

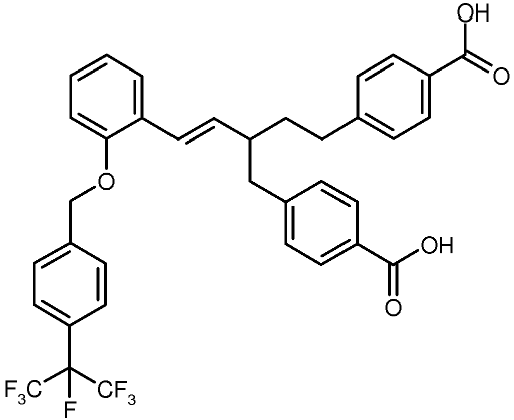

Eine Lösung von 170 mg (0.25 mmol) 4-{(4*E*)-3-(4-Cyanobenzyl)-5-[2-({4-[1,2,2,2-tetrafluor-1-(trifluormethyl)ethyl]benzyl}oxy)phenyl]pent-4-en-1-yl}benzoesäuremethylester in 3 ml 1-Propanol und 2 ml Wasser wird mit 427 mg (7.6 mmol) Kaliumhydroxid versetzt und 12 h bei 110°C gerührt. Nach dem Abkühlen wird der Ansatz mit 1 M Salzsäure sauer gestellt und die ausgefallenen Kristalle abgesaugt, mehrfach mit Wasser gewaschen und getrocknet. Es werden 135 mg (0.2 mmol, 79% d. Th.) der Titelverbindung erhalten.

¹H-NMR (400 MHz, DMSO-d₆, δ/ppm): 12.75 (br. s, 2H), 7.88-7.76 (4H, m), 7.70-7.59 (4H, m), 7.43 (1H, d), 7.31-7.21 (4H, m), 7.18 (1H, t), 7.04 (1H, d), 6.92 (1H, t), 6.51 (1H, d), 6.19-6.07 (1H, m), 5.20 (2H, s), 2.92-2.81 (1H, m), 2.79-2.68 (2H, m), 2.67-2.58 (1H, m), 2.57-2.42 (1H, m), 1.85-1.72 (1H, m), 1.71-1.58 (1H, m).

LC-MS (Methode 7): Rₜ = 3.18 min; MS (ESIpos): m/z = 675 (M+H)⁺.

Auf analoge Weise werden die in der folgenden Tabelle aufgeführten Beispiele erhalten:

| **Beispiel Nr.** | **Beispiel-Struktur** | **Analytische Daten** |
|---|---|---|
| | ***[Edukte]*** | |
| **45** | 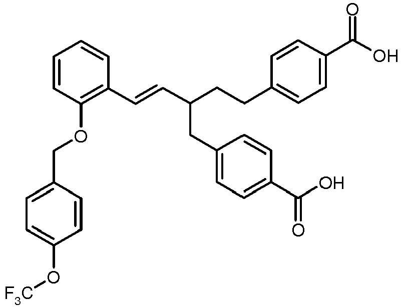 | ¹H-NMR (400 MHz, DMSO-d₆, δ/ppm): 13.10-12.00 (2H, breit), 7.89-7.75 (4H, m), 7.50 (2H, d), 7.43 (1H, d), 7.38 (2H, d), 7.31-7.21 (4H, m), 7.18 (1H, t), 7.01 (1H, d), 6.91 (1H, t), 6.49 (1H, d), 6.19-6.03 (1H, m), 5.11 (2H, s), 2.92-2.81 (1H, m), 2.79-2.66 (2H, m), 2.65-2.58 (1H, m), 2.57-2.42 (1H, m), 1.88-1.73 (1H, m), 1.72-1.59 (1H, m). |
| | *[ausgehend von 1-(Brommethyl)-4-(trifluormethoxy)benzol und Bsp. 51A]* | |
| | | LC-MS (Methode 7): Rₜ = 3.06 min; m/z = 591 (M+H⁺). |
| **46** | 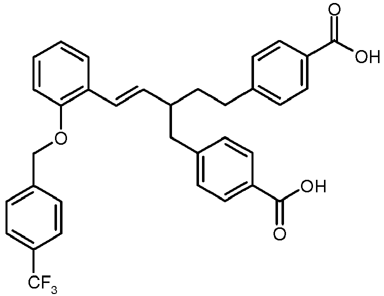 | ¹H-NMR (400 MHz, DMSO-d₆, δ/ppm): 12.77 (2H, br. s), 7.82 (4H, d), 7.71 (2H, d), 7.59 (2H, d), 7.44 (1H, d), 7.32-7.21 (4H, m), 7.19 (1H, t), 7.01 (1H, d), 6.92 (1H, t), 6.50 (1H, d), 6.19-6.05 (1H, m), 5.20 (2H, s), 2.91-2.81 (1H, m), 2.79-2.67 (2H, m), 2.66-2.57 (1H, m), 2.56-2.44 (1H, m), 1.88-1.74 (1H, m), 1.73-1.58 (1H, m). |
| | *[ausgehend von 1-(Brommethyl)-4-(trifluormethyl)benzol und Bsp. 51A]* | |
| | | LC-MS (Methode 2): Rₜ = 2.85 min; m/z = 575 (M+H⁺). |
| **47** (Vergleichsbeispiel) | 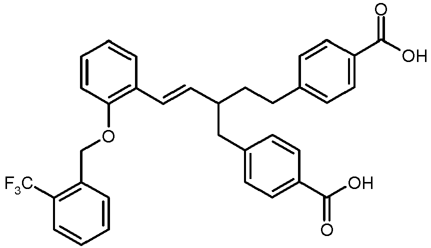 | ¹H-NMR (400 MHz, DMSO-d₆, δ/ppm): 12.74 (2H, br. s), 7.81-7.72 (5H, m), 7.71-7.62 (2H, m), 7.60-7.52 (1H, m), 7.43 (1H, d), 7.28-7.15 (5H, m), 7.01 (1H, d), 6.95 (1H, t), 6.45 (1H, d), 6.15-6.04 (1H, m), 5.21 (2H, s), 2.89-2.79 (1H, m), 2.76-2.62 (2H, m), 2.61-2.39 (2H, m), 1.84-1.70 (1H, m), 1.69-1.56 (1H, m). |
| | [*ausgehend von 1-(Brommethyl)-2-(trifluormethyl)benzol und Bsp. 51A]* | |
| | | LC-MS (Methode 2): Rₜ = 2.86 min; m/z = 575 (M+H⁺). |
| **48** (Vergleichsbeispiel) | 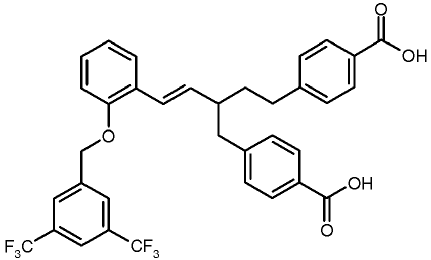 | ¹H-NMR (400 MHz, DMSO-d₆, δ/ppm): 12.74 (2H, br. s), 8.14 (2H, s), 8.07 (1H, s), 7.82.-7.71 (4H, m), 7.44 (1H, d), 7.29-7.17 (5H, m), 7.06 (1H, d), 6.95 (1H, t), 6.51 (1H, d), 6.21-6.08 (1H, m), 5.38-5.25 (2H, m), 2.91-2.79 (1H, m), 2.78-2.64 (2H, m), 2.63-2.41 (2H, m), 1.85-1.71 (1H, m), 1.70-1.56 (1H, m). |
| | [*ausgehend von 1-(Brommethyl)-3,5-bis(trifluormethyl)benzol und Bsp. 51A]* | |
| | | LC-MS (Methode 2): Rₜ = 2.94 min; m/z = 643 (M+H⁺). |
| **49** (Vergleichsbeispiel) | 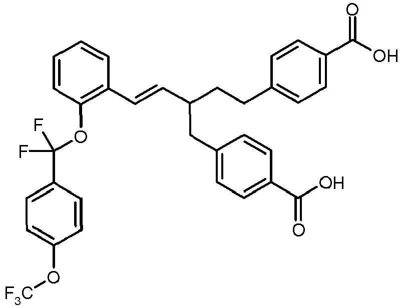 | ¹H-NMR (400 MHz, DMSO-d₆, δ/ppm): 12.76 (2H, br. s), 7.84-7.72 (6H, m), 7.63 (1H, d), 7.52 (2H, d), 7.37-7.17 (7H, m), 6.39 (1H, d), 6.26-6.13 (1H, m), 2.92-2.81 (1H, m), 2.79-2.63 (2H, m), 2.62-2.44 (2H, m), 1.87-1.74 (1H, m), 1.73-1.60 (1H, m). |
| | *[ausgehend von Beispiel 55A und Bsp. 51A]* | LC-MS (Methode 4): Rₜ = 3.08 min; m/z = 625 (M-H⁻). |
| **50** (Vergleichsbeispiel) | 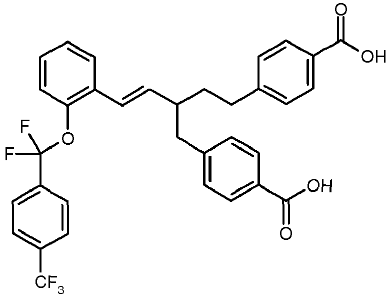 | LC-MS (Methode 7): Rₜ = 3.07 min; m/z = 609 (M-H⁻). |
| | *[ausgehend von 1-[Chlor(difluor)methyl]-4-(trifluormethyl)benzol (CAS Reg.-Nr. 13947-94-9) und Bsp. 51A]* | |
| **51** (Vergleichsbeispiel) | 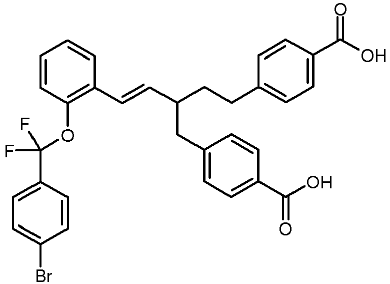 | ¹H-NMR (400 MHz, DMSO-d₆, δ/ppm): 12.78 (2H, br. s), 7.80 (4H, d), 7.73 (2H, d), 7.62 (1H, d), 7.57 (2H, d), 7.35-7.17 (7H, m), 6.37 (1H, d), 6.25-6.12 (1H, m), 2.93-2.80 (1H, m), 2.79-2.63 (2H, m), 2.62-2.44 (2H, m), 1.87-1.73 (1H, m), 1.73-1.60 (1H, m). |
| | *[ausgehend von 1-Brom-4-[brom(difluor)-methyl]benzol (CAS Reg.-Nr. 2358-32-9) und Bsp. 51A]* | LC-MS (Methode 2): Rₜ = 3.03 min; m/z = 619 (M-H⁻). |
| **52** (Vergleichsbeispiel) | 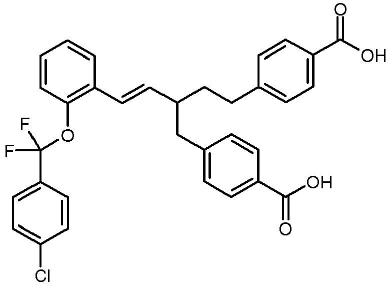 | ¹H-NMR (400 MHz, DMSO-d₆, δ/ppm): 12.78 (2H, br. s), 7.83-7.75 (4H, m), 7.68-7.54 (5H, m), 7.36-7.18 (7H, m), 6.36 (1H, d), 6.25-6.13 (1H, m), 2.92-2.81 (1H, m), 2.79-2.64 (2H, m), 2.63-2.44 (2H, m), 1.87-1.73 (1H, m), 1.73-1.61 (1H, m). |
| | *[ausgehend von 1-[Brom(difluor)methyl]-4-chlorbenzol (CAS Reg.-Nr. 6987-14-0) und Bsp. 51A]* | LC-MS (Methode 4): Rₜ = 3.00 min; m/z = 575 (M-H⁻). |
| **53** | 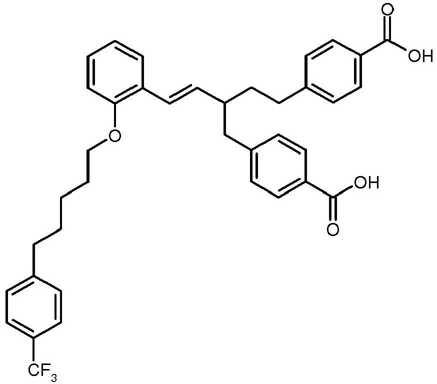 | ¹H-NMR (400 MHz, DMSO-d₆, δ/ppm): 12.78 (2H, br. s), 7.89-7.74 (4H, m), 7.59 (2H, d), 7.40-7.32 (3H, m), 7.32-7.20 (4H, m), 7.16 (1H, t), 6.96-6.83 (2H, m), 6.42 (1H, d), 6.14-6.01 (1H, m), 3.99-3.84 (2H, m), 2.91-2.80 (1H, m), 2.79-2.68 (2H, m), 2.68-2.58 (2H, m), 2.57-2.40 (1H, m), 1.88-1.55 (7H, m), 1.48-1.31 (2H, m). |
| | *[ausgehend von Bsp. 53A und Bsp. 51A]* | LC-MS (Methode 4): Rₜ = 3.29 min; m/z = 629 (M-H⁻). |

### Beispiel 54

### 4-[(4E)-3-(4-Carboxybenzyl)-5-(5-fluor-2-{[4-(trifluormethyl)benzyl]oxy}phenyl)pent-4-en-1-yl]-benzoesäure (Enantiomer 1)

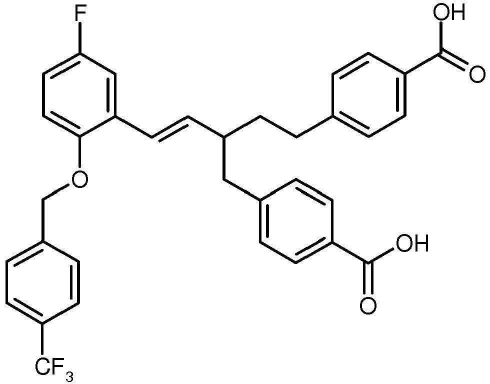

Eine Lösung von 523 mg (0.84 mmol) 4-{(4*E*)-5-(5-Fluor-2-{[4-(trifluormethyl)benzyl]oxy}-phenyl)-3-[4-(methoxycarbonyl)benzyl]pent-4-en-1-yl}benzoesäuremethylester (Enantiomer 1, Beispiel 65A) in 8 ml THF und 4 ml Wasser wird mit 101 mg (4.2 mmol) Lithiumhydroxid versetzt und 12 h bei 50°C gerührt. Nach dem Abkühlen wird der Ansatz mit 1 M Salzsäure sauer gestellt und die ausgefallenen Kristalle abgesaugt, mehrfach mit Wasser gewaschen und getrocknet. Es werden 457 mg (0.77 mmol, 91% d. Th.) der Titelverbindung erhalten.

¹H-NMR (400 MHz, DMSO-d₆, δ/ppm): 12.76 (br. s, 2H), 7.81 (4H, d), 7.21 (2H, d), 7.57 (2H, d), 7.33-7.21 (5H, m), 7.08-6.96 (2H, m), 6.47 (1H, d), 6.29-6.17 (1H, m), 5.17 (2H, s), 2.92-2.82 (1H, m), 2.79-2.66 (2H, m), 2.65-2.57 (1H, m), 2.56-2.45 (1H, m), 1.88-1.75 (1H, m), 1.74-1.61 (1H, m).

LC-MS (Methode 8): Rₜ = 4.23 min; MS (ESIpos): m/z = 593 (M+H)⁺.

Auf analoge Weise werden die in der folgenden Tabelle aufgeführten Beispiele erhalten:

| **Beispiel Nr.** | **Beispiel-Struktur** | **Analytische Daten** |
|---|---|---|
| | ***[Edukt]*** | |
| **55** | 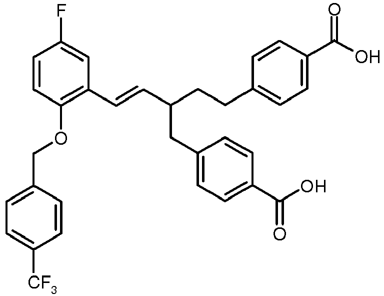 | ¹H-NMR: siehe Bsp. 54 |
| | | LC-MS (Methode 2): Rₜ = 2.82 min; m/z = 593 (M+H⁺). |
| | (Enantiomer 2) | |
| | *[ausgehend von Bsp. 66A]* | |
| **56** | 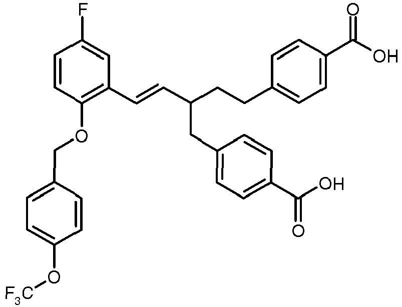 | ¹H-NMR (400 MHz, DMSO-d₆, δ/ppm): 12.78 (2H, br. s), 7.89-7.74 (4H, m), 7.48 (2H, d), 7.33 (2H, d), 7.31-7.19 (5H, m), 7.10-6.94 (2H, m), 6.43 (1H, d), 6.28-6.14 (1H, m), 5.08 (2H, s), 3.93-2.80 (1H, m), 2.79-2.67 (2H, m), 2.66-2.58 (1H, m), 2.57-2.41 (1H, m), 1.87-1.74 (1H, m), 1.73-1.60 (1H, m). |
| | (Enantiomer 1) | |
| | *[ausgehend von Bsp. 68A]* | |
| | | LC-MS (Methode 2): Rₜ = 2.84 min; m/z = 607 (M-H⁻). |
| **57** | 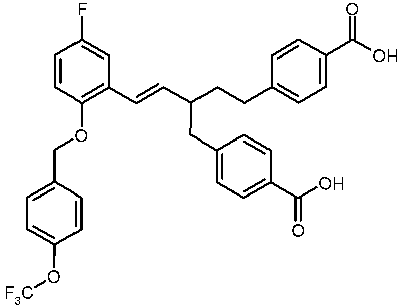 | ¹H-NMR: siehe Bsp. 56 |
| | | LC-MS (Methode 2): Rₜ = 2.84 min; m/z = 607 (M-H⁻). |
| | (Enantiomer 2) | |
| | *[ausgehend von Bsp. 69A]* | |

### Beispiel 58

### 4-[(4E)-3-(4-Carboxybenzyl)-5-{2-[(6-phenylhexyl)oxy]phenyl}pent-4-en-1-yl]benzoesäure

### (Racemat)

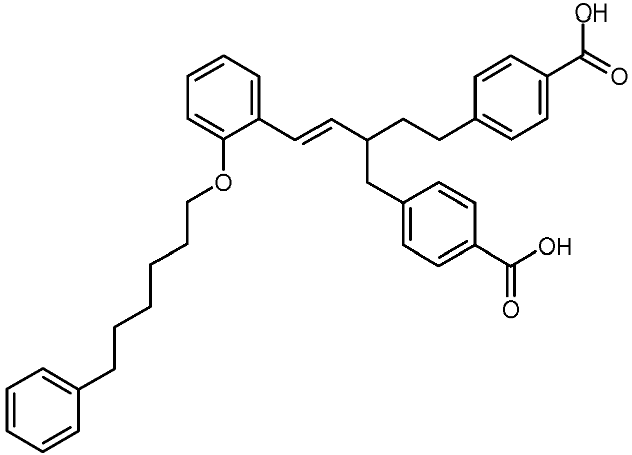

Eine Lösung von 360 mg (0.48 mmol; Reinheit 76%) 4-[(3*E*)-2-[2-(4-Cyanophenyl)ethyl]-4-{2-[(6-phenylhexyl)oxy]phenyl}but-3-en-1-yl]benzoesäuremethylester in 15 ml 1-Propanol wird mit 1452 mg (25.9 mmol) Kaliumhydroxid versetzt und 12 h bei 110°C gerührt. Nach dem Abkühlen wird der Ansatz mit 1 M Salzsäure sauer gestellt und die ausgefallenen Kristalle abgesaugt, mehrfach mit Wasser gewaschen und getrocknet. Es werden 22 mg (0.04 mmol, 8% d. Th.) der Titelverbindung erhalten.

LC-MS (Methode 6): Rt = 6.97 min; MS (ESIpos): m/z = 577 (M+H)⁺.

### Beispiel 59

### (Vergleichsbeispiel)

### 4-[(4E)-3-(4-Carboxybenzyl)-5-(2-{4-[4-(trifluormethyl)phenoxy]butoxy}phenyl)pent-4-en-1-yl]-benzoesäure

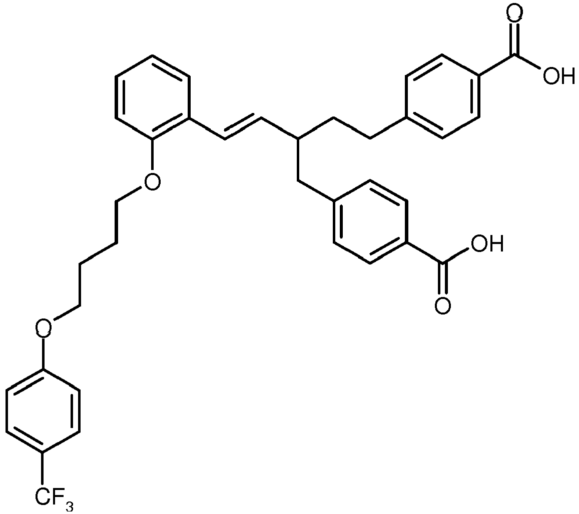

Eine Lösung von 450 mg (0.72 mmol) 4-[(4*E*)-3-(4-Cyanobenzyl)-5-(2-{4-[4-(trifluormethyl)phenoxy]butoxy}phenyl)pent-4-en-1-yl]benzoesäuremethylester in 15 ml 1-Propanol und 2 ml Wasser wird mit 1207 mg (21.5 mmol) Kaliumhydroxid versetzt und 12 h bei 110°C gerührt. Nach dem Abkühlen wird der Ansatz mit 1 M Salzsäure sauer gestellt und die ausgefallenen Kristalle abgesaugt, mehrfach mit Wasser gewaschen und getrocknet. Es werden 447 mg (0.65 mmol, 90% d. Th., Reinheit 92%) der Titelverbindung erhalten.

¹H-NMR (400 MHz, DMSO-d₆, δ/ppm): 12.78 (br. s, 2H), 7.91-7.75 (4H, m), 7.60 (2H, d), 7.39 (1H, d), 7.33-7.20 (4H, m), 7.19 (1H, t), 7.07 (2H, d), 6.94 (1H, d), 6.88 (1H, t), 6.44 (1H, d), 6.18-6.03 (1H, m), 4.12-4.05 (2H, m), 4.04-3.91 (2H, m), 2.91-2.79 (1H, m), 2.78-2.64 (2H, m), 2.63-2.40 (2H, m), 1.95-1.72 (5H, m), 1.72-1.56 (1H, m).

LC-MS (Methode 4): Rₜ = 3.16 min; MS (ESIpos): m/z = 633 (M+H)⁺.

### Beispiel 60

### (Vergleichsbeispiel)

### 4-[(4E)-3-(4-Carboxybenzyl)-5-{2-[4-(4-fluorphenoxy)butoxy]phenyl}pent-4-en-1-yl]benzoesäure

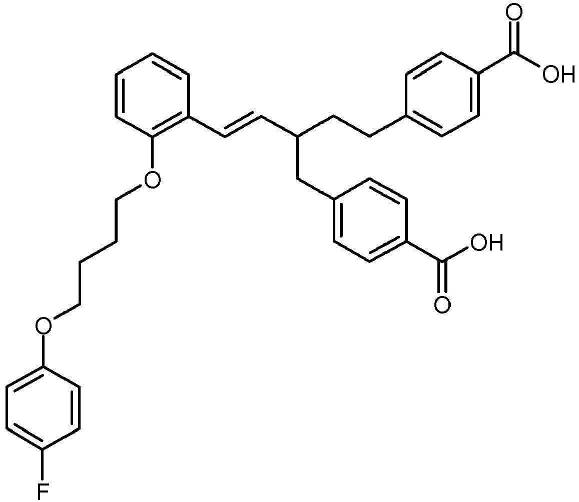

Eine Lösung von 460 mg (0.80 mmol) 4-[(4*E*)-3-(4-Cyanobenzyl)-5-{2-[4-(4-fluorphenoxy)-butoxy]phenyl}pent-4-en-1-yl]benzoesäuremethylester in 15 ml 1-Propanol und 2 ml Wasser wird mit 1340 mg (23.89 mmol) Kaliumhydroxid versetzt und 12 h bei 110°C gerührt. Nach dem Abkühlen wird der Ansatz mit 1 M Salzsäure sauer gestellt und die ausgefallenen Kristalle abgesaugt, mehrfach mit Wasser gewaschen und getrocknet. Es werden 325 mg (0.52 mmol, 65% d. Th., Reinheit 93%) der Titelverbindung erhalten.

¹H-NMR (400 MHz, DMSO-d₆, δ/ppm): 12.78 (br. s, 2H), 7.89-7.77 (4H, m), 7.38 (1H, d), 7.32-7.21 (4H, m), 7.18 (1H, t), 7.08 (2H, t), 6.94 (1H, d), 6.92-6.83 (3H, m), 6.44 (1H, d), 6.17-6.04 (1H, m), 4.07-3.90 (4H, m), 2.91-2.81 (1H, m), 2.79-2.66 (2H, m), 2.66-2.56 (1H, m), 2.54-2.41 (1H, m), 1.90-1.72 (5H, m), 1.71-1.56 (1H, m).

LC-MS (Methode 4): Rₜ = 3.04 min; MS (ESIpos): m/z = 583 (M+H)⁺.

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro:

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Repräsentative Ergebnisse zu den erfindungsgemäßen Verbindungen sind in Tabelle 1 aufgeführt:

**Tabelle 1: Gefäßrelaxierende Wirkung in vitro (Beispiele 7, 10, 44, 48 und 52 sind Vergleichsbeispiele)**

| **Beispiel Nr.** | **IC₅₀ [nM]** |
|---|---|
| 2 | 0.54 |
| 3 | 4.7 |
| 5 | 4.9 |
| 6 | 121.5 |
| 7 | 21 |
| 10 | 48 |
| 11 | 129 |
| 13 | 52.2 |
| 17 | 1.1 |
| 20 | 0.7 |
| 42 | 19 |
| 44 | 22 |
| 45 | 6 |
| 48 | 4 |
| 50 | 370 |
| 54 | 90 |
| 55 | 287 |
| 56 | 77 |

### B-2. Stimulation der rekombinanten löslichen Guanylatcyclase (sGC) in vitro:

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) werden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als n-fache Stimulation der Basalaktivität angegeben. Das Ergebnis für Beispiel 5 ist in Tabelle 2 gezeigt:

**Tabelle 2: Stimulation (n-fach) der rekombinanten löslichen Guanylatcyclase (sGC) in vitro durch Beispiel 5**

| **Konzentration Beispiel 5 [µM]** | **Häm-haltige sGC** | | | **Häm-freie sGC** | |
|---|---|---|---|---|---|
| | **Basal** | **+ 0.1 µM DEA/NO** | **+ 10 µM ODQ** | **Basal** | **+ 10 µM ODQ** |
| 0.0 | 1.0 | 78.7 | 38.8 | 1.0 | 2.0 |
| 0.001 | 3.5 | 88.3 | 40.3 | 19.2 | 18.4 |
| 0.01 | 17.2 | 102.9 | 83.0 | 105.8 | 100.5 |
| 0.1 | 35.1 | 123.1 | 149.5 | 185.3 | 170.4 |
| 1 | 44.2 | 124.1 | 162.1 | 201.2 | 189.2 |
| 10 | 59.5 | 152.0 | 171.2 | 203.1 | 180.7 |

[DEA/NO = 2-(*N,N*-Diethylamino)diazenolat-2-oxid; ODQ = 1*H*-1,2,4-Oxadiazol-(4,3a)-chinoxalin-1-on].

Aus Tabelle 2 ist ersichtlich, dass eine Stimulation sowohl des Häm-haltigen als auch des Hämfreien Enzyms erreicht wird. Weiterhin zeigt die Kombination aus Beispiel 5 und 2-(*N,N*-Diethylamino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch den Häm-abhängigen Inhibitor der löslichen Guanylatcyclase ODQ nicht blockiert, sondern sogar gesteigert. Die Ergebnisse aus Tabelle 2 belegen somit den Wirkmechanismus der erfindungsgemäßen Verbindungen als Aktivatoren der löslichen Guanylatcyclase.

### B-3. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (2) Empfänger, die über einen Multiplexer mit einem (3) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (*1*) systolischer Blutdruck (SBP), (2) diastolischer Blutdruck (DBP), (3) arterieller Mitteldruck (MAP) und (4) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

Die Verbindung aus Beispiel 20 zeigt in diesem Test nach oraler Gabe von 0.3 mg/kg eine deutliche Blutdrucksenkung über einen Zeitraum von 11 Stunden.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I-A) 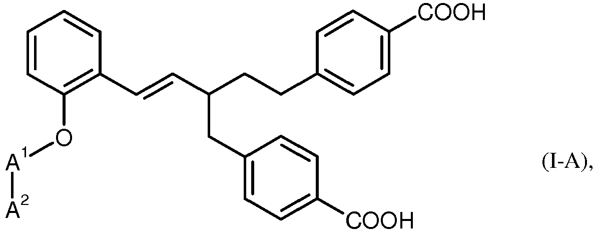 in welcher
A¹ für (C₁-C₇)-Alkandiyl
und
A² für Wasserstoff oder eine Gruppe der Formel 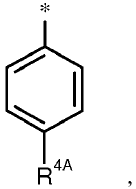 worin * die Verknüpfungsstelle mit der Gruppe A¹ und
R^{4A} Wasserstoff, Fluor, Chlor, Methyl, *tert*.-Butyl, Trifluormethyl, Methoxy oder Trifluormethoxy bedeuten,
stehen,
sowie ihre Salze, Solvate und Solvate der Salze.

2. Verbindung der Formel (I-B) 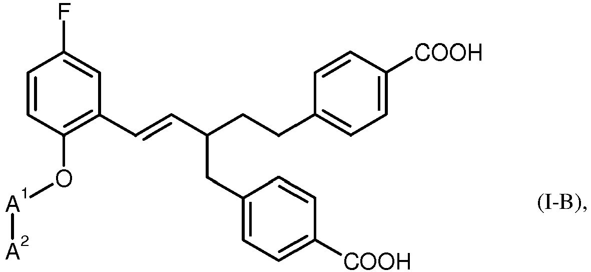 in welcher
A¹ für (C₁-C₇)-Alkandiyl
und
A² für Wasserstoff oder eine Gruppe der Formel worin * die Verknüpfungsstelle mit der Gruppe A¹ und
R^{4A} Wasserstoff, Fluor, Chlor, Methyl, *tert*.-Butyl, Trifluormethyl, Methoxy oder Trifluormethoxy bedeuten, 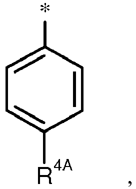
stehen,
sowie ihre Salze, Solvate und Solvate der Salze.

3. 4-[(4*E*)-5-{2-[(4-*tert*.-Butylbenzyl)oxy]phenyl}-3-(4-carboxybenzyl)pent-4-en-1-yl]benzoesäure gemäß Anspruch 1 der Formel 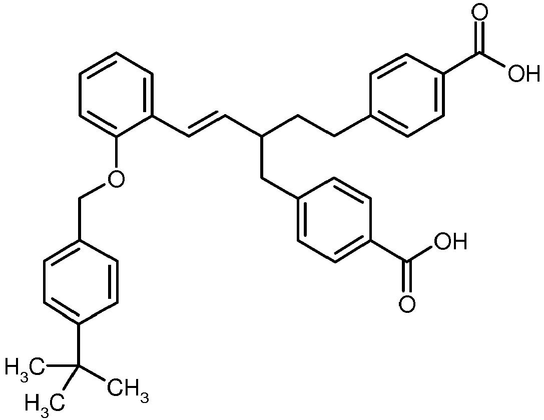 sowie deren Enantiomere.

4. 4-((4*E*)-3-(4-Carboxybenzyl)-5-{2-[(5-phenylpentyl)oxy]phenyl}pent-4-en-1-yl)benzoesäure gemäß Anspruch 1 der Formel 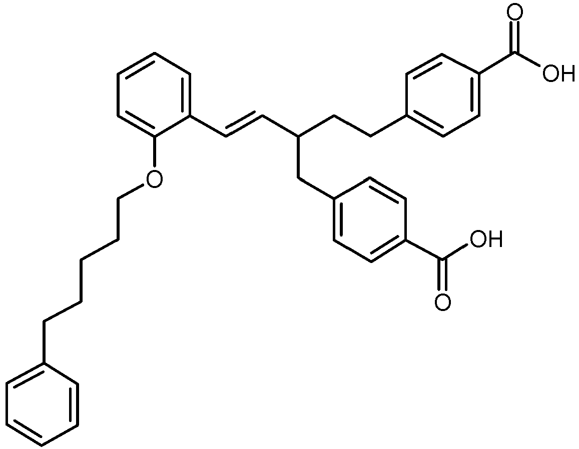 sowie deren Enatiomere.

5. Verfahren zur Herstellung einer Verbindung der Formel (I-A) bzw. (I-B), wie in den Ansprüchen 1 bis 2 definiert, **dadurch gekennzeichnet, dass** man Verbindungen der Formel (II) 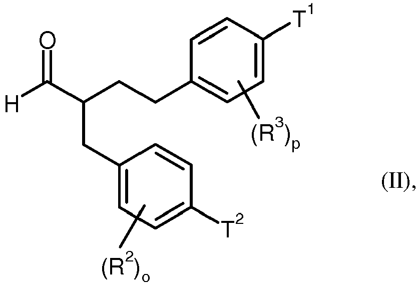 in welcher R², R³ für Wasserstoff stehen, o und p jeweils gleich 4 sind und
T¹ und T² gleich oder verschieden sind und für Cyano oder (C₁-C₄)-Alkoxycarbonyl
stehen,
entweder
[A] in einem inerten Lösungsmittel in Gegenwart einer Base mit einer Verbindung der Formel (III-A) 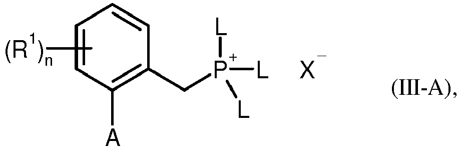 in welcher A für -O-A¹-A²-, R¹ für Wasserstoff steht und n gleich 4 ist und
L für Phenyl oder o-, m- oder p-Tolyl
und
X für Halogenid oder Tosylat steht,
zu Verbindungen der Formel (IV-A) 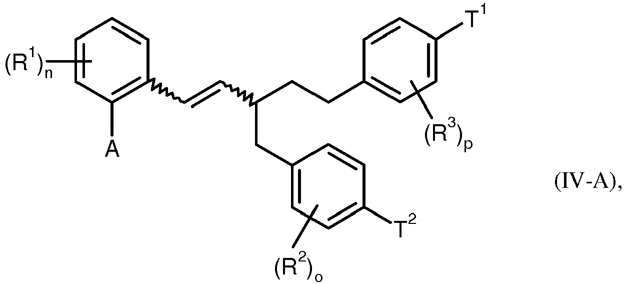 in welcher A, R¹, R², R³, n, o, p, T¹ und T² jeweils die oben angegebenen Bedeutungen haben,
umsetzt
oder
[B] in einem inerten Lösungsmittel in Gegenwart einer Base mit einer Verbindung der
Formel (III-B) 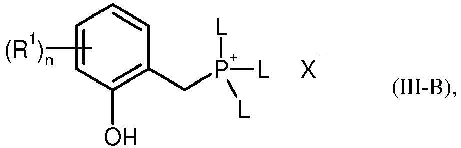 in welcher R¹, n, L und X jeweils die oben angegebenen Bedeutungen haben, zunächst zu Verbindungen der Formel (IV-B) 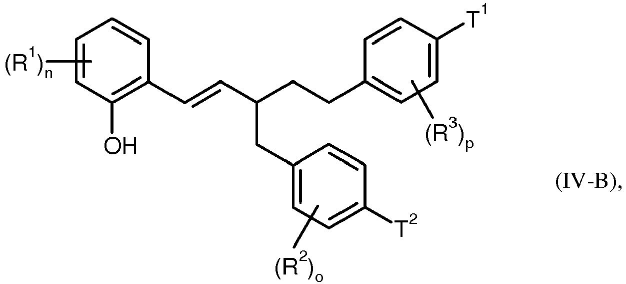 in welcher R¹, R², R³, n, o, p, T¹ und T² jeweils die oben angegebenen Bedeutungen haben,
umsetzt und diese anschließend in einem inerten Lösungsmittel in Gegenwart einer Base mit einer Verbindung der Formel (V)
A²-A^{1A}-Q (V),
in welcher A² die in den Ansprüchen 1 bis 2 angegebene Bedeutung hat,
A^{1A} die in den Ansprüchen 1 bis 2 angegebene Bedeutung von A¹ hat, jedoch nicht für eine Bindung steht,
und
Q für eine Abgangsgruppe, wie beispielsweise Halogen, Tosylat oder Mesylat, steht,
zu Verbindungen der Formel (IV-C) 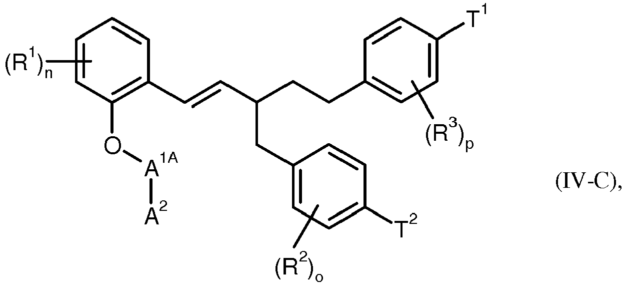 in welcher A^{1A}, A², R¹, R², R³, n, o, p, T¹ und T² jeweils die oben angegebenen Bedeutungen haben,
alkyliert
und die resultierenden Verbindungen der Formel (IV-A) bzw. (IV-C) dann durch Hydrolyse der Ester- bzw. Nitril-Gruppen T¹ und T² in die Dicarbonsäuren der Formel (I-A) bzw. (I-B) überführt
und die Verbindungen der Formel (I-A) bzw. (I-B) gegebenenfalls mit den entsprechenden (i) Lösungsmitteln und/oder (ii) Basen oder Säuren zu ihren Solvaten, Salzen und/oder Solvaten der Salze umsetzt.

6. Verbindung, wie in einem der Ansprüche 1 bis 4 definiert, zur Behandlung und/oder Prävention von Krankheiten.

7. Verwendung einer Verbindung, wie in einem der Ansprüche 1 bis 4 definiert, zur Herstellung eines Arzneimittels zur Behandlung und/oder Prävention von Herzinsuffizienz, Angina pectoris, Hypertonie, pulmonaler Hypertonie, Ischämien, Gefäßerhrankungen, thromboembolischen Erkrankungen und Arteriosklerose.

8. Arzneimittel enthaltend eine Verbindung, wie in einem der Ansprüche 1 bis 4 definiert, in Kombination mit einem inerten, nicht-toxischen, pharmazeutisch geeigneten Hilfsstoff.

9. Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

10. Arzneimittel nach Anspruch 8 oder 9 zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose in Menschen und Tieren.

11. Verbindung, wie in einem der Ansprüche 1 bis 4 definiert, zur Behandlung und/oder Prävention von Herzinsuffizienz, Angina pectoris, Hypertonie, pulmonaler Hypertonie, Ischämien, Gefäßerhrankungen, thromboembolischen Erkrankungen und Arteriosklerose in Menschen und Tieren.

## Claims

1. Compound of the formula (I-A) 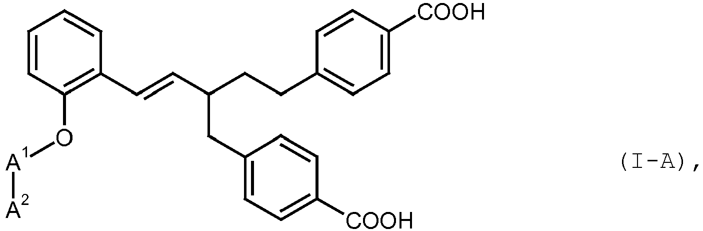 in which
A¹ is (C₁-C₇) -alkanediyl
and
A² is hydrogen or a group of the formula 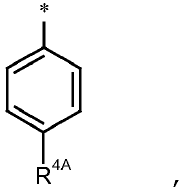 in which * is the point of linkage to the group A¹ and
R^{4A} is hydrogen, fluorine, chlorine, methyl, *tert*-butyl, trifluoromethyl, methoxy or trifluoromethoxy,
and the salts, solvates and solvates of the salts thereof.

2. Compound of the formula (I-B) 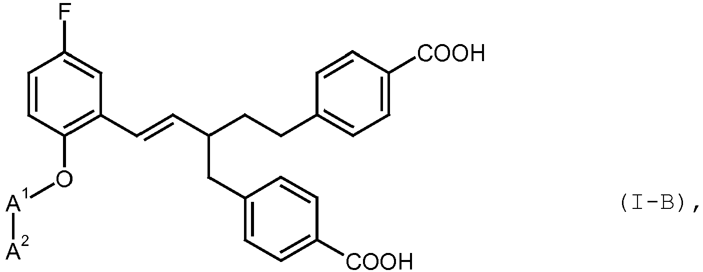 in which
A¹ is (C₁-C₇) -alkanediyl
and
A² is hydrogen or a group of the formula 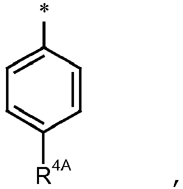 in which * is the point of linkage to the group A¹ and
R^{4A} is hydrogen, fluorine, chlorine, methyl, *tert*-butyl, trifluoromethyl, methoxy or trifluoromethoxy,
and the salts, solvates and solvates of the salts thereof.

3. 4-[(4*E*)-5-{2-[(4-tert-Butylbenzyl)oxy]phenyl}-3-(4-carboxybenzyl)pent-4-en-1-yl]benzoic acid according to Claim 1 of the formula 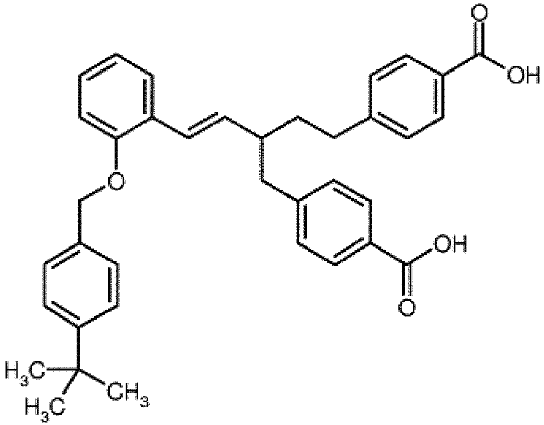 and its enantiomers.

4. 4-((4*E*)-3-(4-Carboxybenzyl)-5-{2-[(5-phenylpentyl)oxy]phenyl}pent-4-en-1-yl)benzoic acid according to Claim 1 of the formula 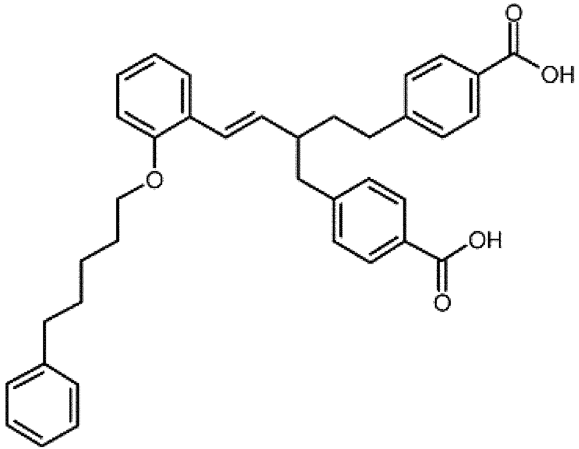 and its enantiomers.

5. Process for preparing a compound of the formula (I-A) or (I-B) as defined in Claims 1 and 2, **characterized in that** compounds of the formula (II) 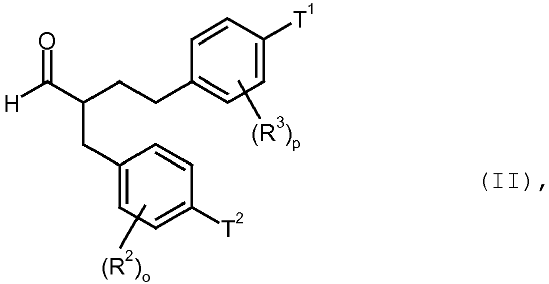 in which R², R³ are hydrogen, o and p are each equal to 4 and
T¹ and T² are identical or different and are cyano or (C₁-C₄)-alkoxycarbonyl,
either
[A] are reacted in an inert solvent in the presence of a base with a compound of the formula (III-A) 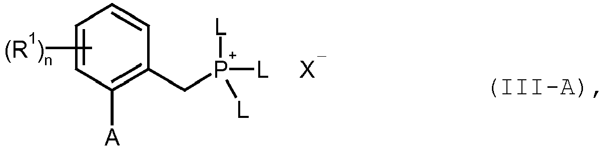 in which A is -O-A¹-A², R¹ is hydrogen and n is equal to 4, and
L is phenyl or o-, m- or p-tolyl
and
X is halide or tosylate,
to give compounds of the formula (IV-A) 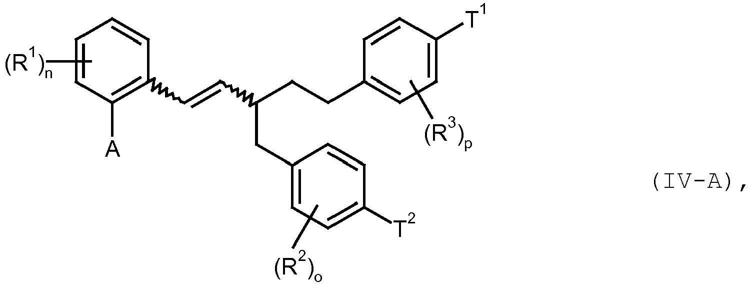 in which A, R¹, R², R³, n, o, p, T¹ and T² each have the meanings indicated above,
or
[B] are reacted in an inert solvent in the presence of a base with a compound of the formula (III-B) 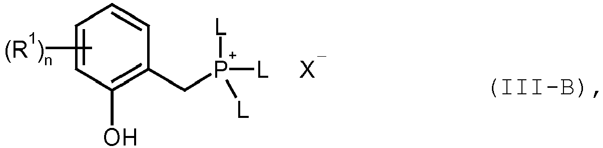 in which R¹, n, L and X each have the meanings indicated above,
initially to give compounds of the formula (IV-B) 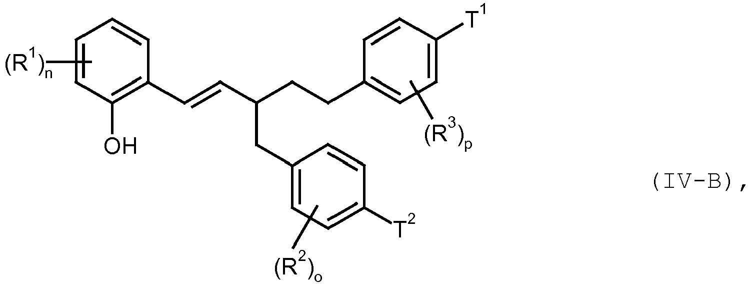 in which R¹, R², R³, n, o, p, T¹ and T² each have the meanings indicated above,
and the latter are then alkylated in an inert solvent in the presence of a base with a compound of the formula (V)
A²-A^{1A}-Q (V),
in which A² has the meaning indicated in Claims 1 and 2,
A^{1A} has the meaning indicated in Claims 1 and 2 for A¹ but is not a bond,
and
Q is a leaving group such as, for example, halogen, tosylate or mesylate,
to give compounds of the formula (IV-C) 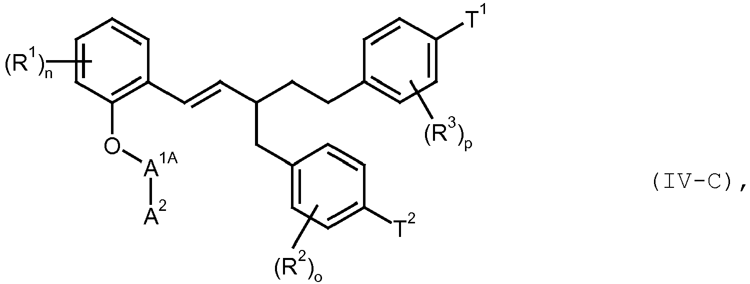
in which A^{1A}, A², R¹, R², R³, n, o, p, T¹ and T² each have the meanings indicated above,
and the resulting compounds of the formula (IV-A) or (IV-C) are then converted by hydrolysis of the ester or nitrile groups T¹ and T² into the dicarboxylic acids of the formula (I-A) or (I-B),
and the compounds of the formula (I-A) or (I-B) are reacted where appropriate with the appropriate (i) solvents and/or (ii) bases or acids to give the solvates, salts and/or solvates of the salts thereof.

6. Compound as defined in any of Claims 1 to 4 for the treatment and/or prevention of diseases.

7. Use of a compound as defined in any of Claims 1 to 4 for producing a medicament for the treatment and/or prevention of heart failure, angina pectoris, hypertension, pulmonary hypertension, ischaemias, vascular disorders, thromboembolic disorders and arteriosclerosis.

8. Medicament comprising a compound as defined in any of Claims 1 to 4 in combination with an inert, non-toxic, pharmaceutically suitable excipient.

9. Medicament comprising a compound as defined in any of Claims 1 to 4 in combination with one or more further active ingredients selected from the group consisting of organic nitrates, NO donors, cGMP-PDE inhibitors, stimulators of guanylate cyclase, agents having antithrombotic activity, agents lowering blood pressure, and agents altering lipid metabolism.

10. Medicament according to Claim 8 or 9 for the treatment and/or prevention of heart failure, angina pectoris, hypertension, pulmonary hypertension, ischaemias, vascular disorders, thromboembolic disorders and arteriosclerosis in humans and animals.

11. Compound as defined in any of Claims 1 to 4 for the treatment and/or prevention of heart failure, angina pectoris, hypertension, pulmonary hypertension, ischaemias, vascular disorders, thromboembolic disorders and arteriosclerosis in humans and animals.

## Revendications

1. Composé de formule (I-A) 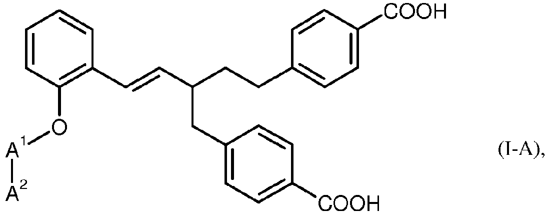 dans laquelle
A¹ représente (C₁-C₇)-alcanediyle et
A² représente hydrogène ou un groupe de formule 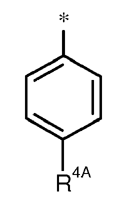 dans laquelle
* représente le site de liaison avec le groupe A¹ et R^{4A} représente hydrogène, fluor, chlore, méthyle, tert-butyle, trifluorométhyle, méthoxy ou trifluorométhoxy,
ainsi que ses sels, solvates et solvates des sels.

2. Composé de formule (I-B) 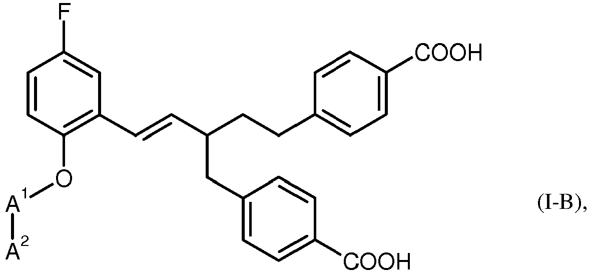 dans laquelle
A¹ représente (C₁-C₇)-alcanediyle et
A² représente hydrogène ou un groupe de formule 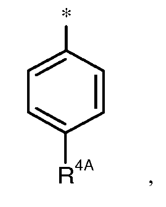 dans laquelle
* représente le site de liaison avec le groupe A¹ et R^{4A} représente hydrogène, fluor, chlore, méthyle, tert-butyle, trifluorométhyle, méthoxy ou trifluorométhoxy,
ainsi que ses sels, solvates et solvates des sels.

3. Acide 4-[(4E)-5-{2-[(4-tert-butylbenzyl)oxy]phényl}-3-(4-carboxybenzyl)pent-4-én-1-yl]benzoïque selon la revendication 1 de formule 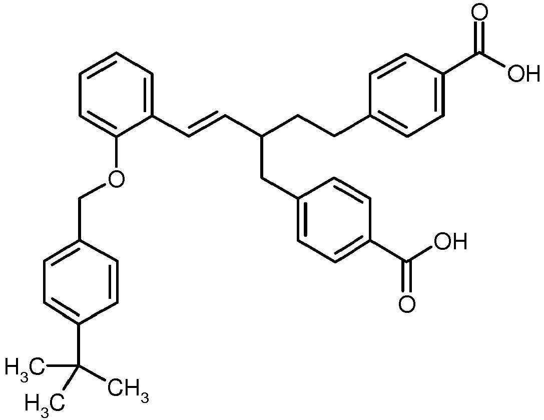 ainsi que ses énantiomères.

4. Acide 4-((4E)-3-(4-carboxybenzyl)-5-{2-[(5-phénylpentyl)oxy]phényl}pent-4-én-1-yl)benzoïque selon la revendication 1 de formule 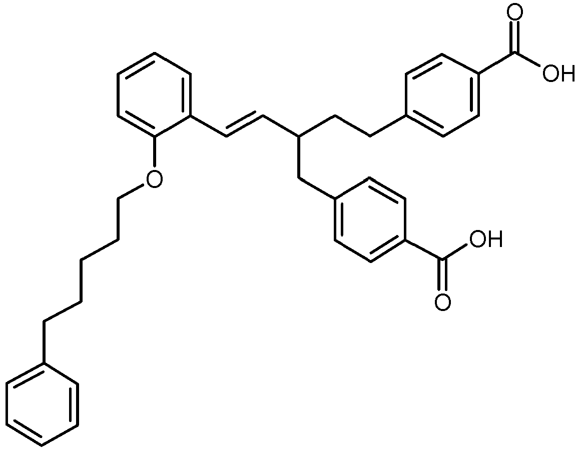 ainsi que ses énantiomères.

5. Procédé pour la préparation d'un composé de formule (I-A) ou (I-B) tel que défini dans les revendications 1 à 2, **caractérisé en ce qu'**on transforme des composés de formule (II) 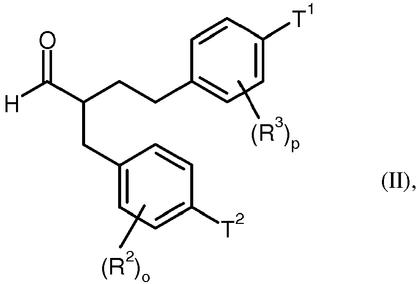 dans laquelle
R², R³ représentent hydrogène, o et p valent à chaque fois 4 et
T¹ et T² sont identiques ou différents et représentent cyano ou (C₁-C₄) -alcoxycarbonyle,
soit
[A] dans un solvant inerte en présence d'une base avec un composé de formule (III-A) 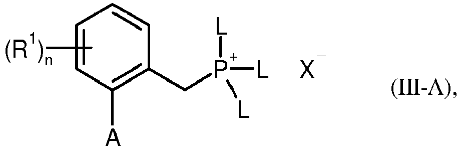
dans laquelle A représente -O-A¹-A², R¹ représente hydrogène et n vaut 4 et
L représente phényle ou o-toluyle, m-toluyle ou p-toluyle et
X représente halogénure ou tosylate,
en composés de formule (IV-A) 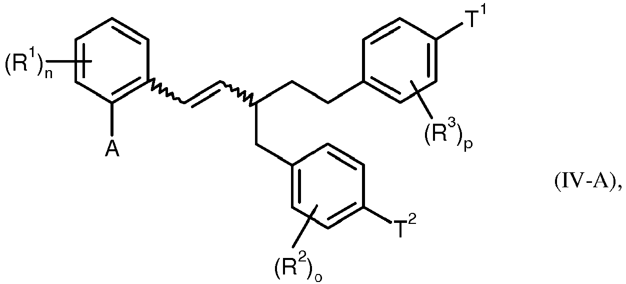 dans laquelle A, R¹, R², R³, n, o, p, T¹ et T² présentent à chaque fois les significations indiquées ci-dessus,
soit
[B] dans un solvant inerte en présence d'une base avec un composé de formule (III-B) 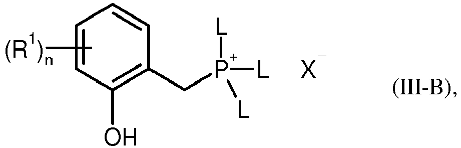 dans laquelle R¹, n, L et X présentent à chaque fois les significations indiquées ci-dessus, d'abord en composés de formule (IV-B) 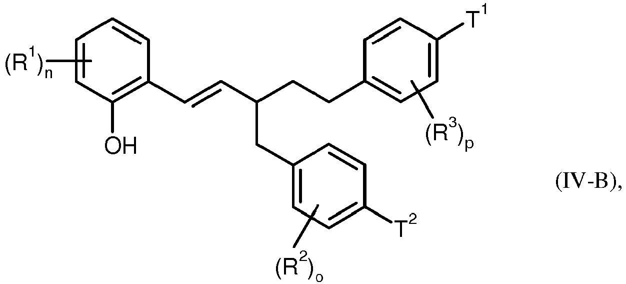 dans laquelle R¹, R², R³, n, o, p, T¹ et T² présentent à chaque fois les significations indiquées ci-dessus, et on alkyle ensuite ceux-ci dans un solvant inerte en présence d'une base avec un composé de formule (V)
A²-A^{1A}-Q (V),
dans laquelle
A² présente la signification indiquée dans les revendications 1 à 2 et
A^{1A} présente la signification de A¹ indiquée dans les revendications 1 à 2, mais ne représente pas une liaison, et
Q représente un groupe partant tel que par exemple halogène, mésylate ou tosylate,
en composés de formule (IV-C) 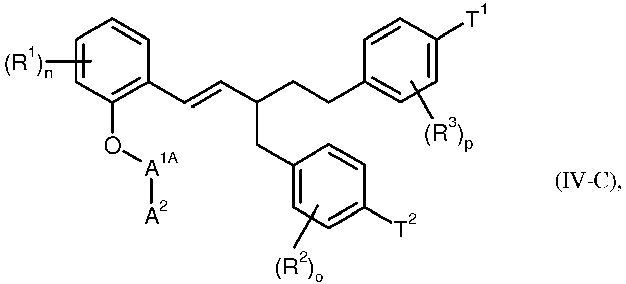 dans laquelle
A^{1A}, A², R¹, R², R³, n, o, p, T¹ et T² présentent à chaque fois les significations indiquées ci-dessus,
et on transforme ensuite les composés résultants respectivement de formule (IV-A) ou (IV-C) par hydrolyse des groupes ester ou nitrile T¹ et T² en acides dicarboxyliques de formule (I-A) ou (I-B) et on transforme les composés de formule (I-A) ou (I-B) le cas échéant avec (i) les solvants et/ou (ii) les bases ou acides correspondants en leurs solvates, sels et/ou les solvates des sels.

6. Composé, tel que défini dans l'une quelconque des revendications 1 à 4, pour le traitement et/ou la prévention de maladies.

7. Utilisation d'un composé tel que défini dans l'une quelconque des revendications 1 à 4 pour la préparation d'un médicament pour le traitement et/ou la prévention de l'insuffisance cardiaque, de l'angine de poitrine, de l'hypertonie, de l'hypertonie pulmonaire, des ischémies, des maladies vasculaires, des maladies thromboemboliques et de l'artériosclérose.

8. Médicament contenant un composé, tel que défini dans l'une quelconque des revendications 1 à 4, en combinaison avec un adjuvant inerte, non toxique, pharmaceutiquement approprié.

9. Médicament contenant un composé tel que défini dans l'une quelconque des revendications 1 à 4, en combinaison avec une ou plusieurs autres substances actives choisies dans le groupe constitué par les nitrates organiques, les donneurs de NO, les inhibiteurs de cGMP-PDE, les stimulateurs de la guanylate-cyclase, les agents à effet antithrombotique, les agents hypotenseurs ainsi que les agents modifiant le métabolisme des lipides.

10. Médicament selon la revendication 8 ou 9, pour le traitement et/ou la prévention de l'insuffisance cardiaque, de l'angine de poitrine, de l'hypertonie, de l'hypertonie pulmonaire, des ischémies, des maladies vasculaires, des maladies thromboemboliques et de l'artériosclérose chez les êtres humains et les animaux.

11. Composé tel que défini dans l'une quelconque des revendications 1 à 4 pour le traitement et/ou la prévention de l'insuffisance cardiaque, de l'angine de poitrine, de l'hypertonie, de l'hypertonie pulmonaire, des ischémies, des maladies vasculaires, des maladies thromboemboliques et de l'artériosclérose chez les êtres humains et les animaux.
